# EUROPÄISCHE PATENTSCHRIFT

(11) **EP 2 046 915 B1**
(45) Veröffentlichungstag und Bekanntmachung des Hinweises auf die Patenterteilung: **02.12.2015**
(21) Anmeldenummer: 07726183.2
(22) Anmeldetag: 02.07.2007
(51) Int. Cl.: C09K 11/06, H05B 33/14, C07C 15/38, C07D 311/82

(54) **NEUE MATERIALIEN FÜR ORGANISCHE ELEKTROLUMINESZENZVORRICHTUNGEN**
NEW MATERIALS FOR ORGANIC ELECTROLUMINESCENT DEVICES
NOUVEAUX MATÉRIAUX POUR DISPOSITIFS ORGANIQUES ÉLECTROLUMINESCENTS

(30) Priorität: 28.07.2006 DE 102006035035
(43) Veröffentlichungstag der Anmeldung: 15.04.2009
(73) Patentinhaber: Merck Patent GmbH, 64293 Darmstadt (DE)
(72) Erfinder: STOESSEL, Philipp, 60487 Frankfurt am Main (DE); BUESING, Arne, 65959 Frankfurt am Main (DE); HEIL, Holger, 64295 Darmstadt (DE)
(86) Internationale Anmeldenummer: PCT/EP2007/005848
(87) Internationale Veröffentlichungsnummer: WO 2008/011964

(56) Entgegenhaltungen:
- EP-A- 0 866 110
- EP-A- 1 289 343
- EP-A- 1 359 790
- EP-A- 1 452 574
- JP-A- 2000 150 167
- US-A1- 2005 048 313
- US-A1- 2005 212 409
- US-A1- 2005 214 566
- DATABASE CAPLUS [Online] CHEMICAL ABSTRACTS SERVICE, COLUMBUS, OHIO, US; Februar 2006 (2006-02), SONG, JI-GUO ET AL: "Quick synthesis and properties of peri-xanthenoxanthene" XP002453221 gefunden im STN Database accession no. 2006:334795 -& FAGUANG XUEBAO , 27(1), 95-99 CODEN: FAXUEW; ISSN: 1000-7032, Februar 2006 (2006-02), XP008084199
- DATABASE CA [Online] CHEMICAL ABSTRACTS SERVICE, COLUMBUS, OHIO, US; 1985, BLOKHIN, A. P. ET AL: "Polarized luminescence of complex molecules in the vapor phase" XP002453222 gefunden im STN Database accession no. 1985:228762 -& SPECTROSCOPY LETTERS , 18(4), 301-16 CODEN: SPLEBX; ISSN: 0038-7010, 1985, XP008084201

## Beschreibung

Die vorliegende Erfindung betrifft organische Halbleiter und deren Verwendung in organischen elektronischen Vorrichtungen.

Organische Halbleiter werden für eine Reihe verschiedenartiger Anwendungen, die im weitesten Sinne der Elektronikindustrie zugerechnet werden können, entwickelt. Der Aufbau organischer Elektrolumineszenzvorrichtungen (OLEDs), in denen diese organischen Halbleiter als funktionelle Materialien eingesetzt werden, ist beispielsweise in US 4539507, US 5151629, EP 0676461 und WO 98/27136 beschrieben. Allerdings zeigen diese Vorrichtungen noch erhebliche Probleme, die einer dringenden Verbesserung bedürfen:
1. Viele Dotanden gemäß dem Stand der Technik sind nur in aufwändigen mehrstufigen Syntheseverfahren zugänglich.
2. Die Lebensdauer der organischen Elektrolumineszenzvorrichtungen gemäß dem Stand der Technik ist noch nicht ausreichend für hochwertige Anwendungen.
3. Die thermische Stabilität insbesondere vieler blauer Dotanden ist nicht ausreichend.
4. Viele Verbindungen, insbesondere blaue Dotanden, gemäß dem Stand der Technik weisen eine hohe Empfindlichkeit gegenüber Sauerstoff und Licht auf, was ihre Synthese und Handhabung erheblich erschwert.

Als Stand der Technik bei blau emittierenden Verbindungen kann die Verwendung von Arylvinylaminen genannt werden (z. B. WO 04/013073, WO 04/016575, WO 04/018587). Diese Verbindungen sind jedoch thermisch instabil und lassen sich nicht unzersetzt verdampfen, was einen hohen technischen Aufwand für die OLED-Herstellung erfordert und somit einen technischen Nachteil darstellt. Einen weiteren Nachteil stellt die Emissionsfarbe dieser Verbindungen dar: Während im Stand der Technik mit diesen Verbindungen tiefblaue Emission (CIE-y-Koordinaten im Bereich von 0.15-0.18) beschrieben wird, konnten diese Farbkoordinaten nicht in einfachen Vorrichtungen gemäß dem Stand der Technik reproduziert werden. Hier erhält man grünblaue Emission. Es ist nicht offensichtlich, wie mit diesen Verbindungen blaue Emission erzeugt werden kann.
Weiterhin sind als blaue und grüne Emitter gemäß dem Stand der Technik aromatische Diamine mit kondensierten Aromaten, beispielsweise mit Anthracen, Pyren oder Chrysen (WO 04/078872, EP 1437395, US 2005/0214566, WO 04/044088) bekannt. Jedoch weisen auch diese im Device noch keine zufriedenstellenden Eigenschaften auf, insbesondere in Bezug auf die Lebensdauer, aber auch in Bezug auf die Betriebsspannung. Weiterhin sind diese Verbindungen sehr empfindlich gegenüber Sauerstoff und Licht, was ihre Handhabung insbesondere im technischen Maßstab erschwert.

Es besteht daher weiterhin Bedarf an verbesserten Materialien, insbesondere blau emittierenden Verbindungen, die thermisch stabil sind, die in organischen elektronischen Vorrichtungen zu guten Effizienzen und gleichzeitig zu hohen Lebensdauern führen, die bei der Herstellung und beim Betrieb der Vorrichtung zu reproduzierbaren Ergebnissen führen und die synthetisch einfach zugänglich sind.

Überraschend wurde gefunden, dass substituierte Derivate von peri-Xanthenoxanthen, insbesondere solche, welche mit aromatischen Substituenten oder weiteren im Folgenden beschriebenen Substituenten substituiert sind, sich sehr gut für die Verwendung in organischen Elektrolumineszenzvorrichtungen eignen. Diese Verbindungen weisen eine hohe thermische Stabilität auf. Mit diesen Materialien ist weiterhin eine Steigerung der Effizienz und der Lebensdauer der organischen elektronischen Vorrichtung im Vergleich zu Materialien gemäß dem Stand der Technik möglich. Diese Materialien und deren Verwendung in organischen elektronischen Vorrichtungen sind daher der Gegenstand der vorliegenden Erfindung.

Gegenstand der Erfindung sind Verbindungen gemäß den Formeln (1) bis (4), 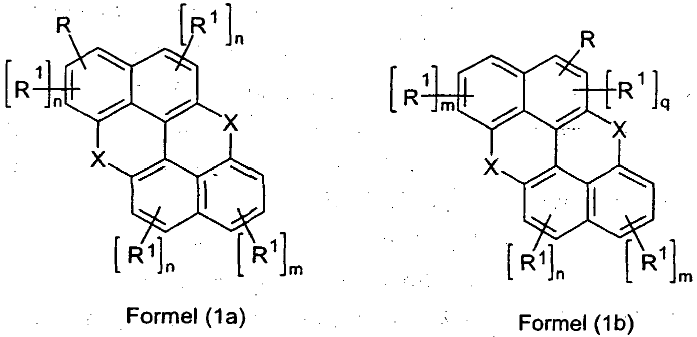 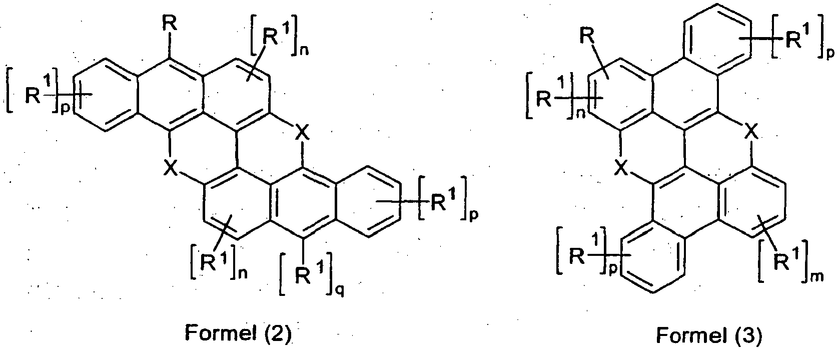 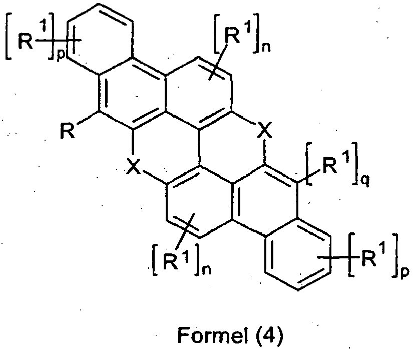 wobei für die Symbole und Indizes gilt:
- X: ist bei jedem Auftreten gleich oder verschieden O, S, NR¹, C(R¹)₂, BR¹, PR¹, POR¹, SO oder SO₂;
- R: ist bei jedem Auftreten gleich oder verschieden eine Gruppe N(Ar)₂, C(=O)Ar, P(=O)Ar₂, S(=O)Ar, S(=O)₂Ar, CR²=CR²Ar, OAr, SAr, Si(R¹)₃, eine geradkettige Alkylgruppe mit 1 bis 40 C-Atomen, eine verzweigte oder cyclische Alkylgruppe mit 3 bis 40 C-Atomen, die jeweils durch einen oder mehrere Reste R² substituiert sein kann und wobei ein oder mehrere H-Atome durch F, Cl, Br, I, CN oder NO₂ ersetzt sein können, eine geradkettige Alkoxygruppe mit 2 bis 40 C-Atomen oder eine verzweigte oder cyclische Alkoxygruppe mit 3 bis 40 C-Atomen, wobei die Alkoxygruppe jeweils durch einen oder mehrere Reste R² substituiert sein kann, wobei eine oder mehrere nicht benachbarte CH₂-Gruppen der Alkoxygruppen durch R²C=CR², C≡C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere nicht-aromatische Reste R¹ substituiert sein kann; dabei kann R auch mit benachbarten Substituenten R¹ ein mono- oder polycyclisches Ringsystem bilden;
- R¹: ist bei jedem Auftreten gleich oder verschieden H, F, Cl, Br, I, CHO, N(Ar)₂, C(=O)Ar, P(=O)Ar₂, S(=O)Ar, S(=O)₂Ar, CR²=CR²Ar, OAr, SAr, CN, NO₂, Si(R²)₃, B(OR²)₂, OSO₂R², eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C , Si(R²)₂, Ge(R²)₂, Sn(R²)₂, C=0, C=S, C=Se, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere nicht-aromatische Reste R¹ substituiert sein kann, oder eine Kombination dieser Systeme; dabei können zwei oder mehrere benachbarte Substituenten R¹ auch miteinander oder R¹ mit R ein mono- oder polycyclisches Ringsystem bilden;
- Ar: ist bei jedem Auftreten gleich oder verschieden ein aromatisches oder heteroaromatisches Ringsystem mit 5 bis 40 aromatischen Ringatomen, das mit einem oder mehreren nicht-aromatischen Resten R¹ substituiert sein kann; dabei können auch zwei Reste Ar, welche an dasselbe Stickstoff- oder Phosphoratom binden, durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verknüpft sein;
- R²: ist bei jedem Auftreten gleich oder verschieden H oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten R² auch miteinander ein mono- oder polycyclisches aliphatisches oder aromatisches Ringsystem bilden;
- n: ist bei jedem Auftreten gleich oder verschieden 0, 1 oder 2;
- m: ist bei jedem Auftreten gleich oder verschieden 0, 1, 2 oder 3;
- p: ist bei jedem Auftreten gleich oder verschieden 0, 1, 2, 3 oder 4;
- q: ist bei jedem Auftreten gleich oder verschieden 0 oder 1;
dabei sind die folgenden Verbindungen von der Erfindung ausgenommen:

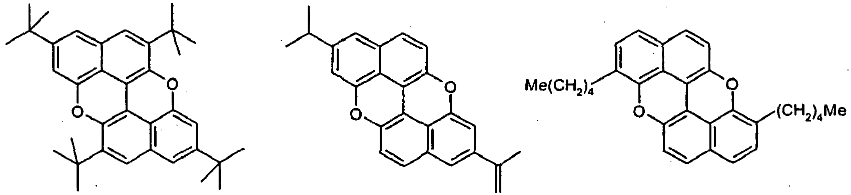

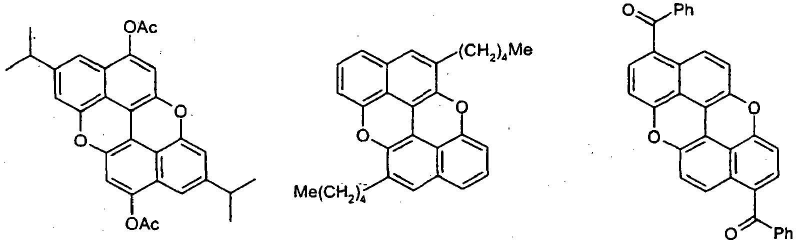

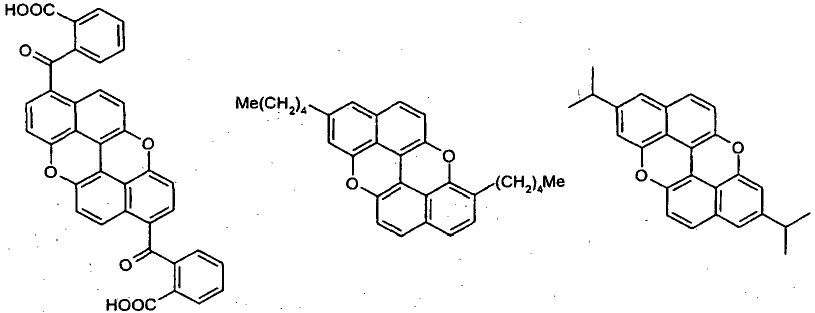

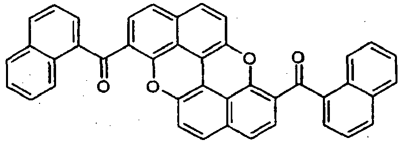

Ein weiterer Gegenstand der Erfindung sind die Verbindungen der folgenden Formeln (5) bis (8), 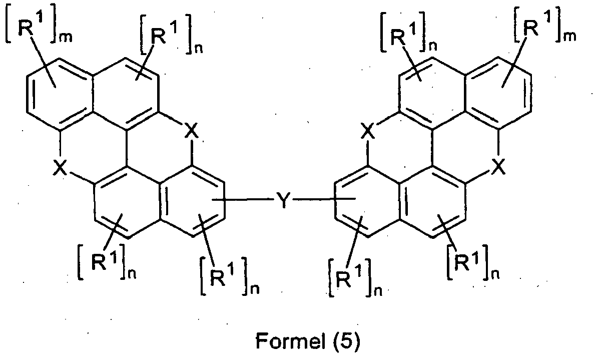 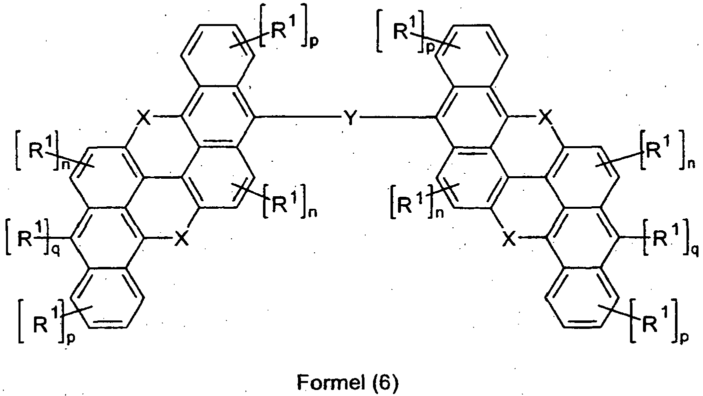 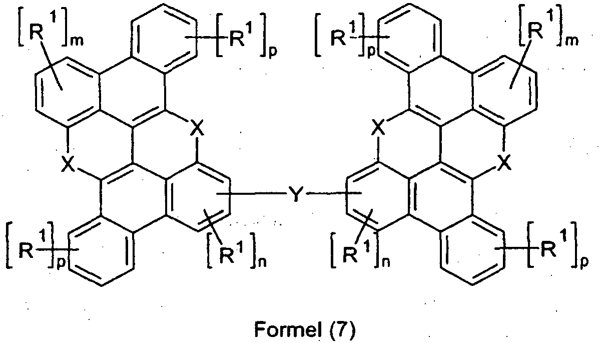 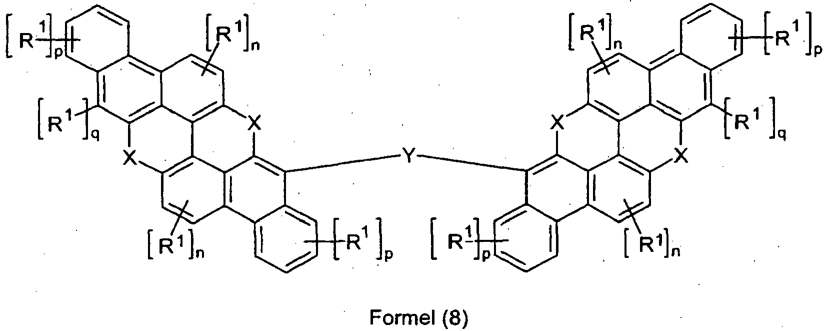 wobei R¹, R², X, m, n, p und q dieselbe Bedeutung haben, wie oben beschrieben, und weiterhin gilt:
- Y: ist eine Einfachbindung, eine Gruppe C(=O) P(=O)Ar, S(=O), S(=O)₂, N(Ar), O, S, eine Alkylen- oder Alkylidengruppe mit 1 bis 20 C-Atomen oder ein bivalentes aromatisches Ringsystem mit 5 bis 40 aromatischen Ringatomen, welches mit einem oder mehreren nicht-aromatischen Resten R¹ substituiert sein kann.

Dabei kann, wenn Y eine Gruppe der Formel P(=O)Ar oder N(Ar) darstellt, die Gruppe Ar auch eine weitere Gruppe mit dem Grundgerüst der Formel (1) bis (4) darstellen, so dass C₃-symmetrische Verbindungen entstehen.

Der Übersichtlichkeit halber ist die Nummerierung am Beispiel des peri-Xanthenoxanthens in der folgenden Formel dargestellt:

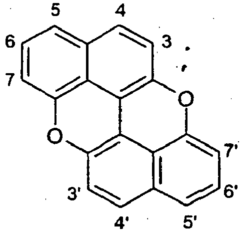

Bevorzugt weisen die Verbindungen gemäß Formel (1) bis (8) eine Glasübergangstemperatur T_{g} von größer als 70 °C auf, besonders bevorzugt größer als 100 °C, ganz besonders bevorzugt größer als 130 °C.

Unter benachbarten Resten R bzw. R¹ im Sinne dieser Erfindung werden Reste verstanden, die entweder am selben Kohlenstoffatom gebunden sind oder die an benachbarten Kohlenstoffatomen gebunden sind.

Eine Arylgruppe im Sinne dieser Erfindung enthält 6 bis 40 C-Atome; eine Heteroarylgruppe im Sinne dieser Erfindung enthält 2 bis 40 C-Atome und mindestens 1 Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Pyren, Chinolin, Isochinolin, etc., verstanden.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (bevorzugt weniger als 10 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, Stilben, Benzophenon, etc. als aromatische Ringsysteme im Sinne dieser Erfindung verstanden werden. Ebenso werden unter einem aromatischen bzw. heteroaromatischen Ringsystem Systeme verstanden, in denen mehrere Aryl- bzw. Heteroarylgruppen durch Einfachbindungen miteinander verknüpft sind, beispielsweise Biphenyl, Terphenyl oder Bipyridin. Auch die Grundgerüste der Formeln (1) bis (4), also peri-Xanthenoxanthen und die weiteren oben aufgeführten Grundgerüste, werden als aromatische Ringsysteme im Sinne der vorliegenden Erfindung verstanden.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Trifluormethoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5 - 60 aromatischen Ringatomen, welches noch jeweils mit den oben genannten Resten R substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, peri-Xanthenoxanthen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol. Weiterhin werden als unter aromatischen Ringsystemen auch beliebige Kombination der oben genannten Arylgruppen verstanden, beispielsweise Binaphthyl, Napthylanthryl, etc.

Bevorzugt sind Strukturen gemäß den Formeln (9) bis (24), 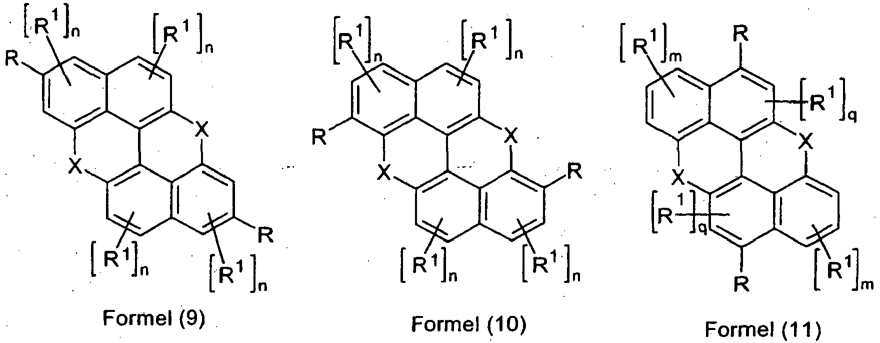 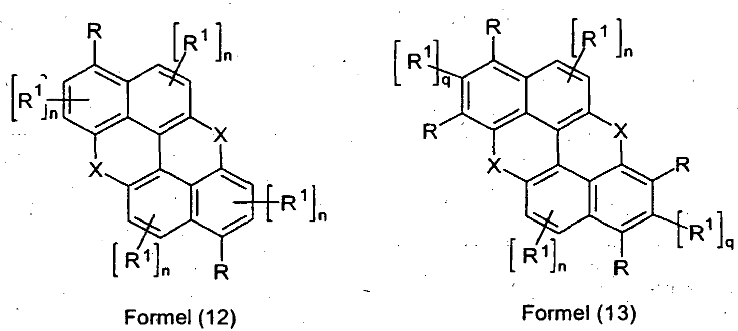 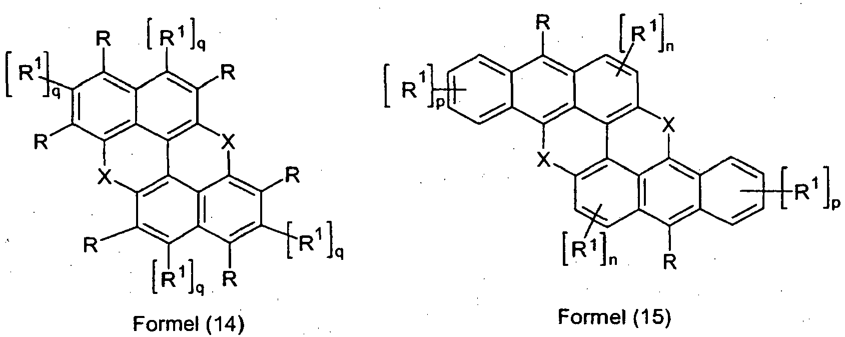 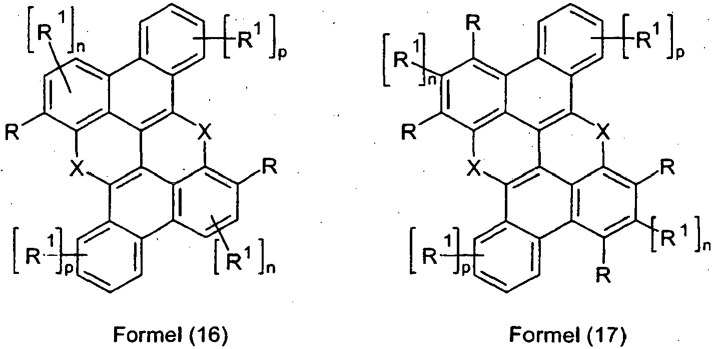 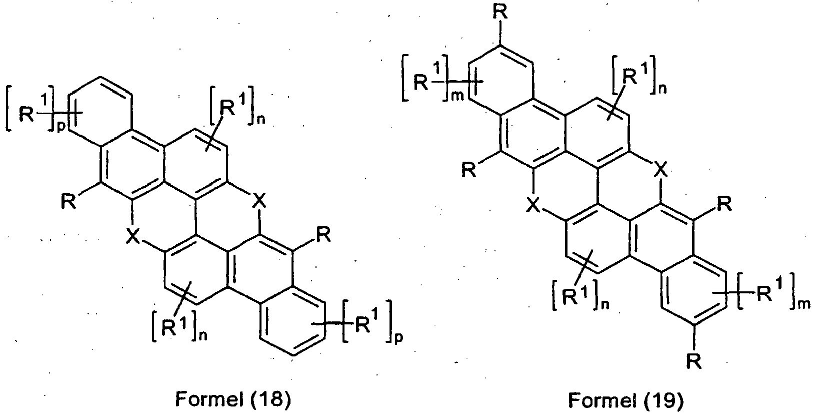 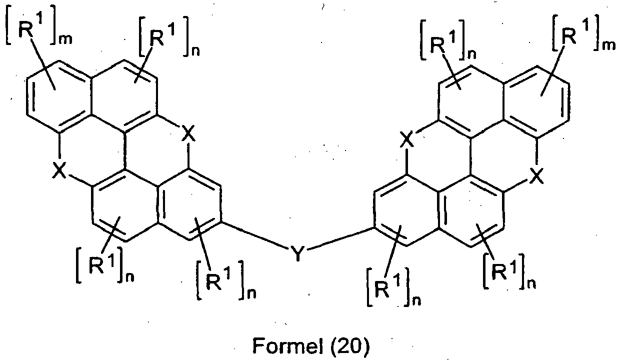 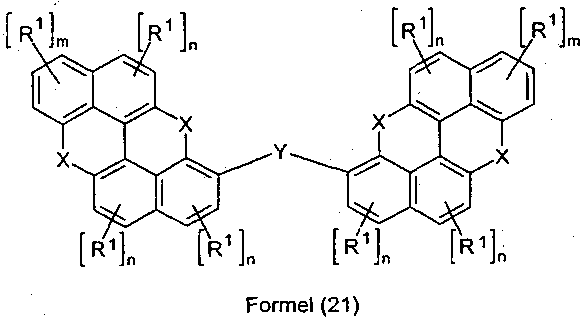 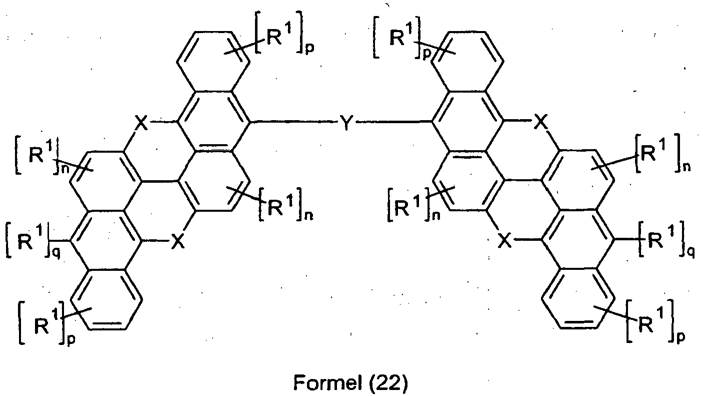 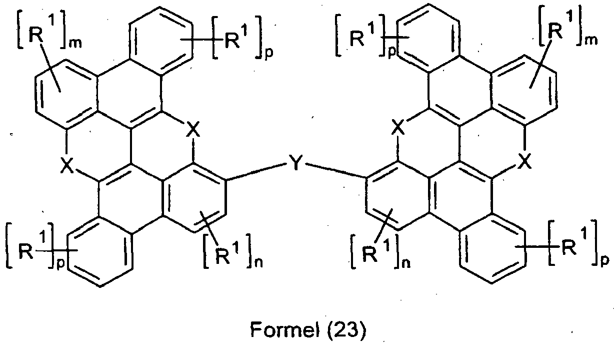 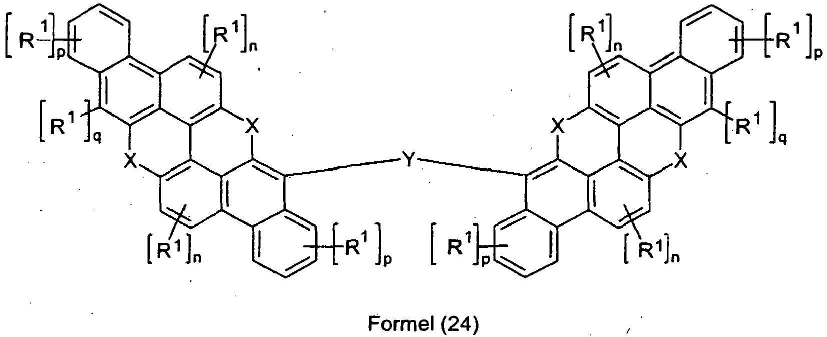 wobei R, R¹, R², Ar X, Y, m, n, p und q dieselbe Bedeutung haben, wie oben beschrieben.

Nochmals weiterhin bevorzugt sind Verbindungen gemäß Formel (1) bis (8), in denen die Indizes m, n, p und q für 0, 1 oder 2 stehen, besonders bevorzugt für 0 oder 1.

In einer besonders bevorzugten Ausführungsform sind die Strukturen gemäß Formel (1) bis (8) gewählt aus den Formeln (9a) bis (24a), 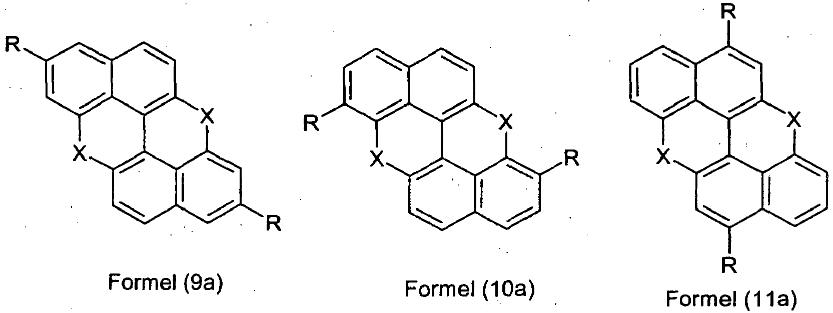 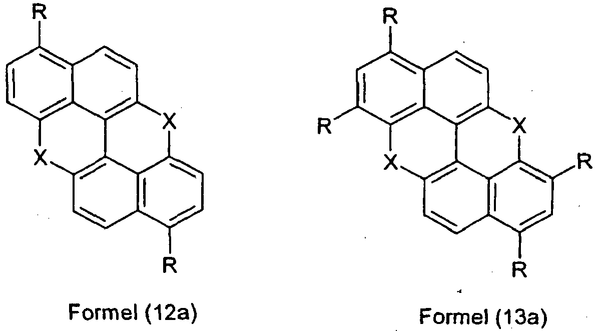 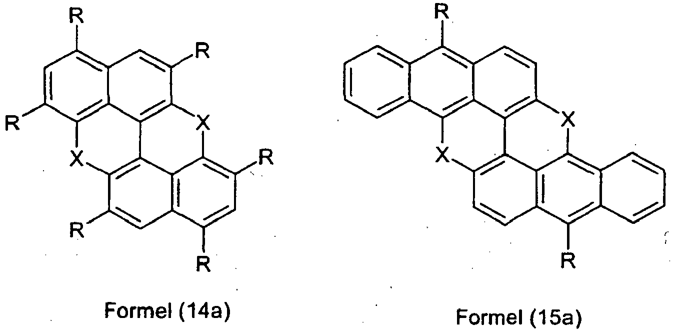 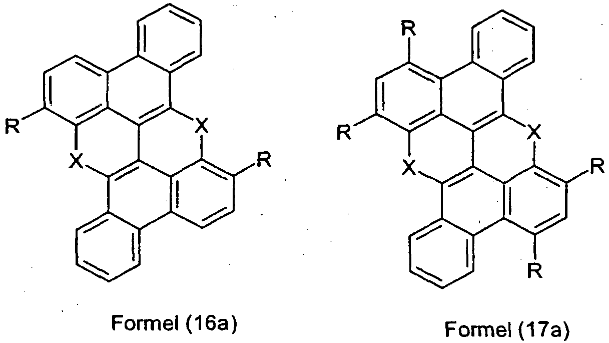 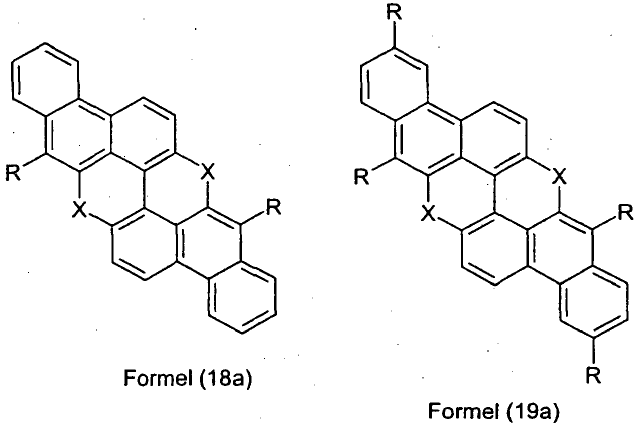 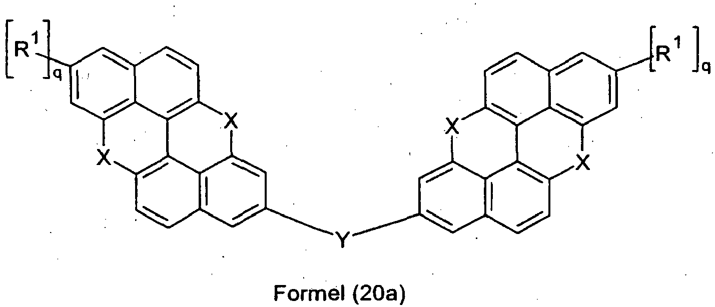 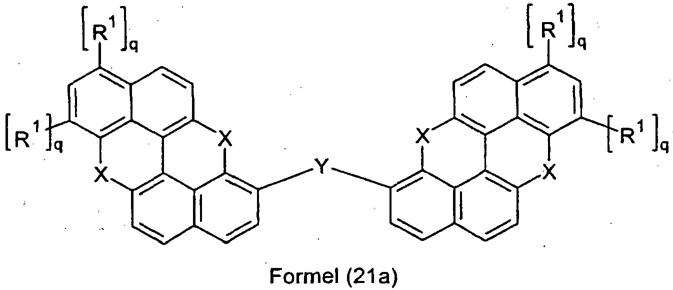 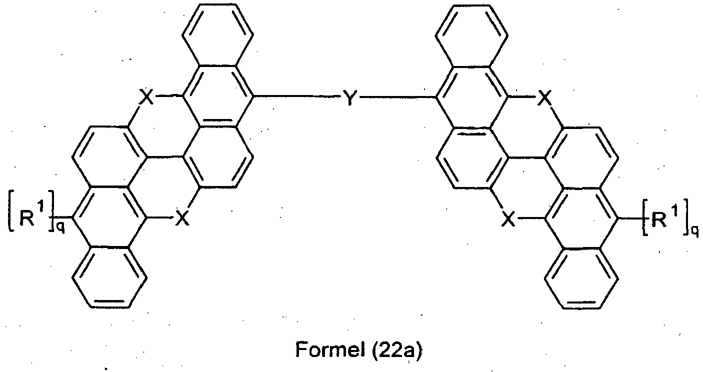 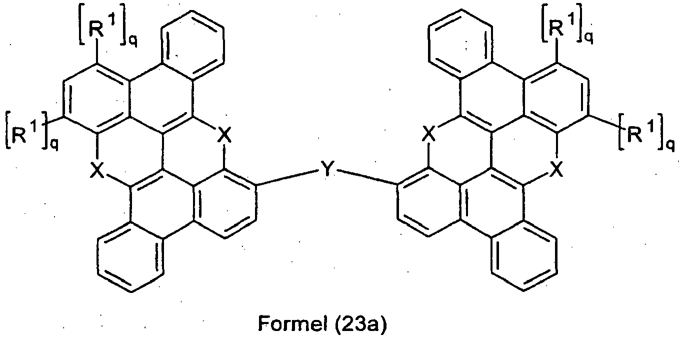 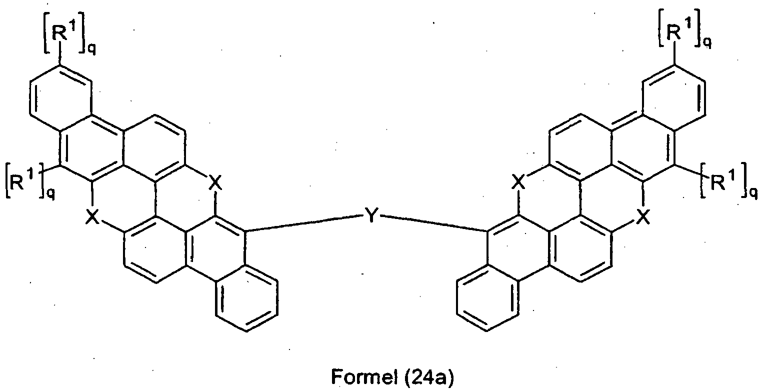 wobei R, R¹, R¹, Ar, X, Y und q dieselbe Bedeutung haben, wie oben beschrieben.

Bevorzugt sind weiterhin Verbindungen gemäß Formel (1) bis (8), in denen das Symbol X für O, S oder C(R¹)₂ steht, besonders bevorzugt für O oder C(R¹)₂. Wenn das Symbol X für eine Gruppe C(R¹)₂ steht, so können auch die beiden Reste R¹ an dieser Gruppe miteinander ein Ringsystem bilden. Bevorzugte Reste R¹ an der Gruppe C(R¹)₂ sind Methyl, Phenyl, ortho-Tolyl, para-Tolyl, para-tert-Butylphenyl oder zwei Phenylgruppen, welche miteinander ein Ringsystem bilden und so ein Spirosystem aufbauen.

Bevorzugt sind weiterhin Verbindungen gemäß Formel (1) bis (4) und (9) bis (19) bzw. (9a) bis (19a), in denen das Symbol R, gleich oder verschieden bei jedem Auftreten, für eine Gruppe N(Ar)₂, C(=O)Ar, P(=O)Ar₂, eine geradkettige Alkylgruppe mit 1 bis 4 C-Atomen oder eine verzweigte Alkylgruppe mit 3 bis 5 C-Atomen oder ein aromatisches oder heteroaromatisches Ringsystem mit 5 bis 25 aromatischen Ringatomen, welches durch einen oder mehrere nicht-aromatische Reste R¹ substutiert sein kann, steht. Besonders bevorzugt sind Verbindungen gemäß Formel (1) bis (4) und (9) bis (19) bzw. (9a) bis (19a), in denen das Symbol R, gleich oder verschieden bei jedem Auftreten, für eine Gruppe N(Ar)₂, C(=O)Ar oder ein aromatisches Ringsystem mit 6 bis 20 aromatischen Ringatomen, welches durch einen oder mehrere nicht-aromatische Reste R¹ substituiert sein kann, steht.

Wenn das Symbol R für eine Gruppe N(Ar)₂ steht, dann steht es bevorzugt für eine Gruppe der folgenden Formel (25) oder (26), 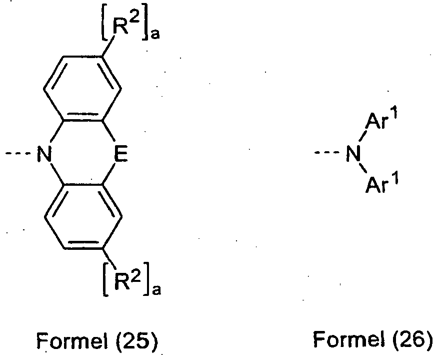 wobei R² die oben aufgeführte Bedeutung hat und weiterhin gilt:
- E: steht für eine Einfachbindung, O, S, N(R²) oder C(R²)₂;
- Ar¹: ist gleich oder verschieden bei jedem Auftreten eine Aryl- oder Heteroarylgruppe mit 5 bis 20 aromatischen Ringatomen oder eine Triarylamingruppe mit 15 bis 30 aromatischen Ringatomen, welche jeweils mit einem oder mehreren Resten R² substituiert sein kann, bevorzugt eine Aryl- oder Heteroarylgruppe mit 6 bis 14 aromatischen Ringatomen oder eine Triarylamingruppe mit 18 bis 22 aromatischen Ringatomen, welche jeweils mit einem oder mehreren Resten R² substituiert sein kann;
- a: ist bei jedem Auftreten gleich oder verschieden 0 oder 1.

Besonders bevorzugt steht Ar¹ gleich oder verschieden für Phenyl, 1-Naphthyl, 2-Naphthyl, Triphenylamin, Naphthyldiphenylamin oder Dinaphthylphenylamin, welches jeweils durch eine oder mehrere Alkylgruppen mit 1 bis 4 C-Atomen oder durch Fluor substituiert sein kann.

Bevorzugt sind weiterhin Verbindungen der Formel (5) bis (8) und (20) bis (24) bzw. (20a) bis (24a), in denen das Symbol Y für eine Gruppe C(=O), N(Ar) oder für eine bivalente Arylgruppe mit 6 bis 16 C-Atomen, welche mit einem oder mehreren nicht-aromatische Resten R¹ substituiert sein kann, steht.

Bevorzugt sind weiterhin Verbindungen der Formel (5) bis (8) und (20) bis (24) bzw. (10a) bis (24a), in denen das Symbol R¹ dieselbe Bedeutung hat wie R, und in denen insbesondere auch dieselbe Bevorzugung gilt, wie für R oben beschrieben.

Bevorzugt sind weiterhin Verbindungen gemäß den Formeln (1) bis (24) bzw. (9a) bis (24a), welche symmetrisch bezüglich den vorhandenen Substituenten aufgebaut sind, insbesondere Strukturen, welche mindestens eine zweizählige Drehachse aufweisen. Besonders bevorzugt sind in den Strukturen gemäß Formel (9a) bis (19a) alle Reste R innerhalb einer Verbindung gleich gewählt. Besonders bevorzugt sind weiterhin in den Strukturen gemäß (20a) bis (24a) alle Reste R¹ innerhalb einer Verbindung gleich gewählt.

Beispiele für bevorzugte erfindungsgemäße Verbindungen sind die im Folgenden abgebildeten Strukturen (1) bis (182).

| | |
|---|---|
| 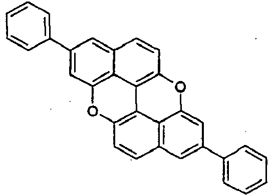 | 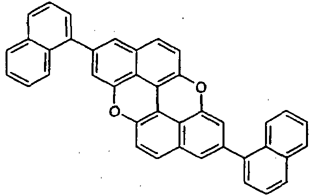 |
| (1) | (2) |
| 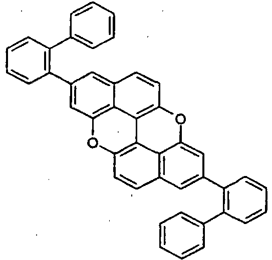 | 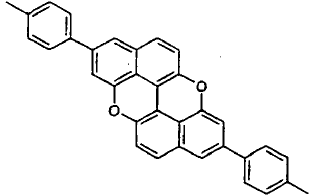 |
| (3) | (4) |
| 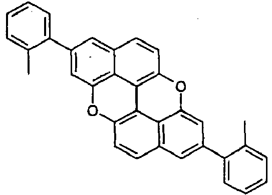 | 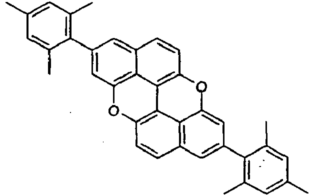 |
| (5) | (6) |
| 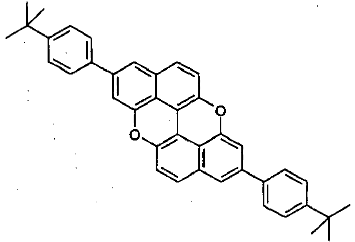 | 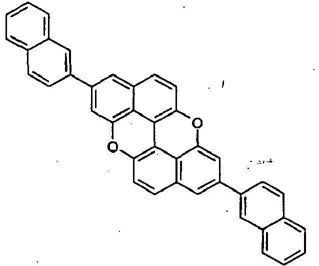 |
| (7) | (8) |
| 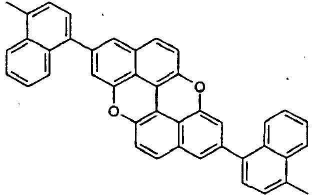 | 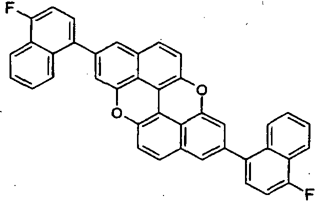 |
| (9) | (10) |
| 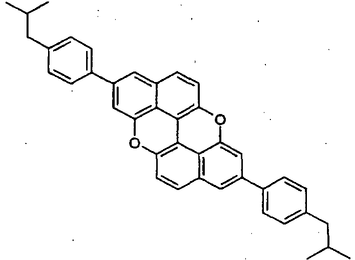 | 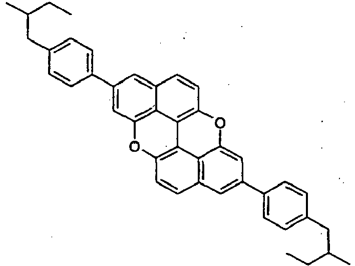 |
| (11) | (12) |
| 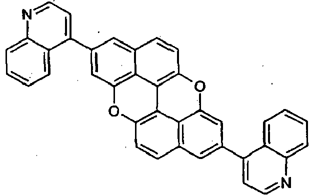 | 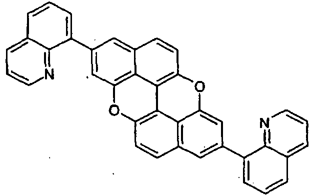 |
| (13) | (14) |
| 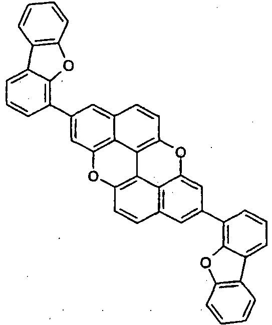 | 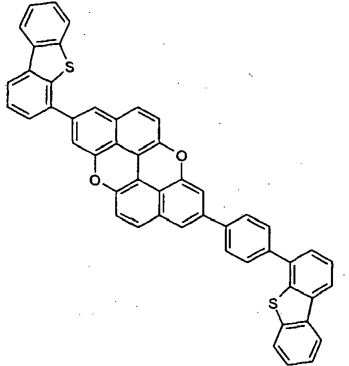 |
| (15) | (16) |
| 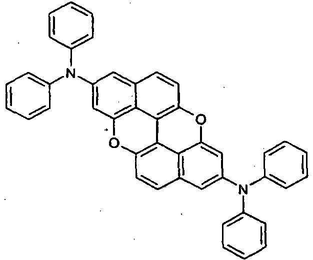 | 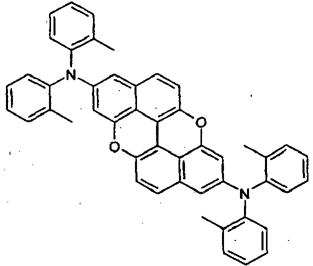 |
| (17) | (18) |
| 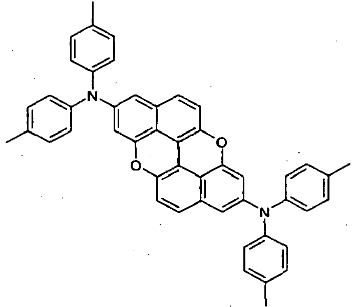 | 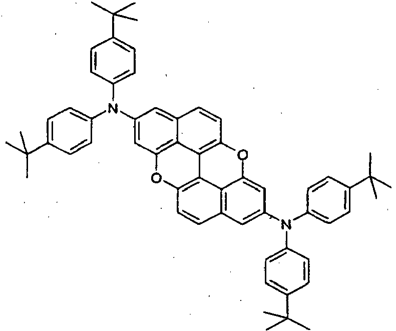 |
| (19) | (20) |
| 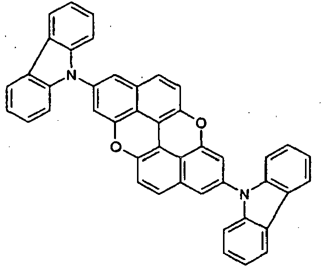 | 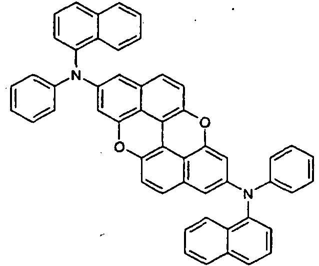 |
| (21) | (22) |
| 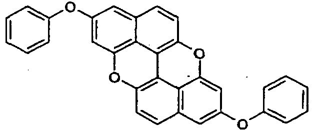 | 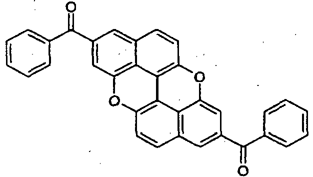 |
| (23) | (24) |
| 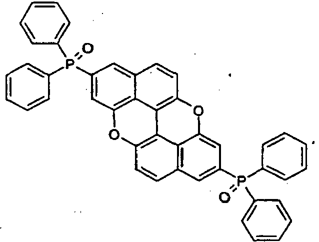 | 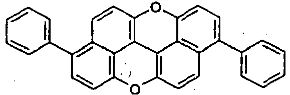 |
| (25) | (26) |
| 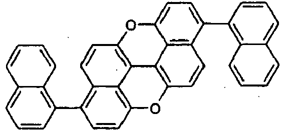 | 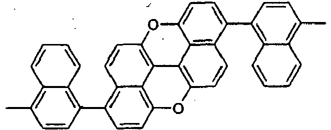 |
| (27) | (28) |
| 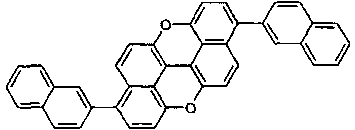 | 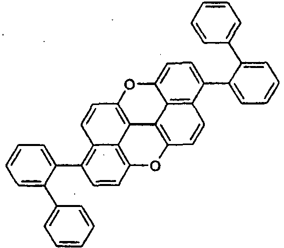 |
| (29) | (30) |
| 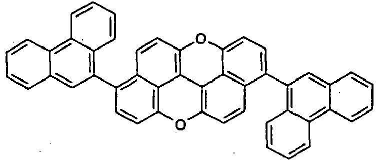 | 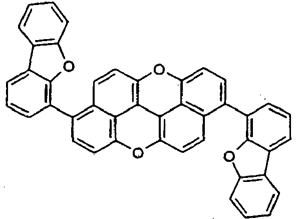 |
| (31) | (32) |
| 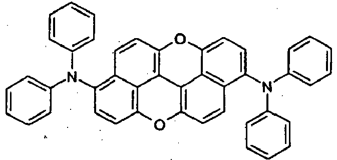 | 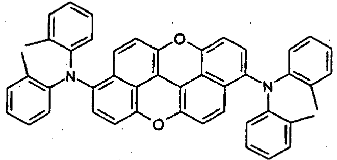 |
| (33) | (34) |
| 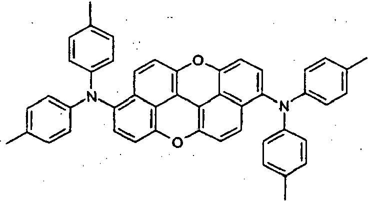 | 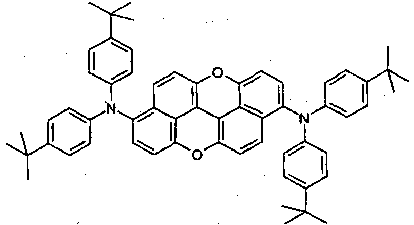 |
| (35) | (36) |
| 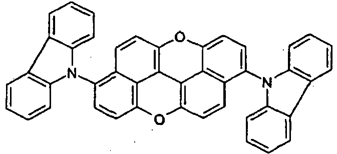 | 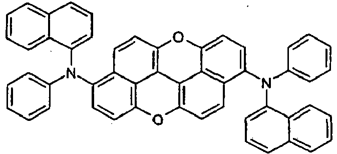 |
| (37) | (38) |
| 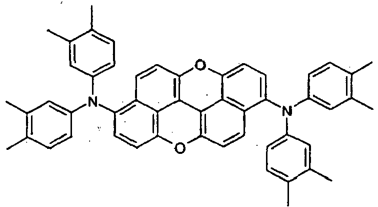 | 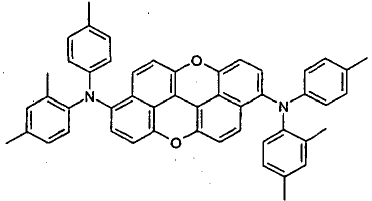 |
| (39) | (40) |
| 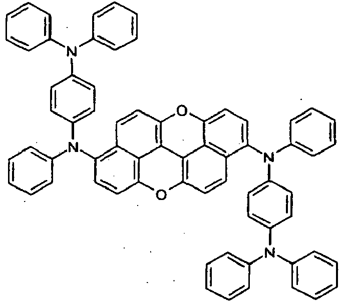 | 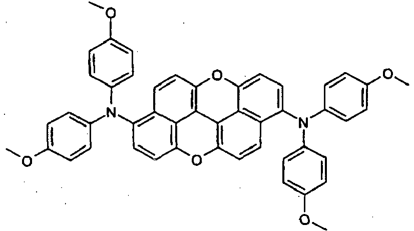 |
| (41) | (42) |
| 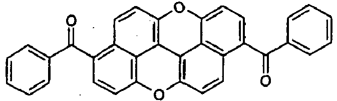 | 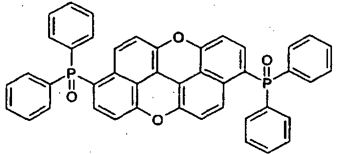 |
| (43) | (44) |
| 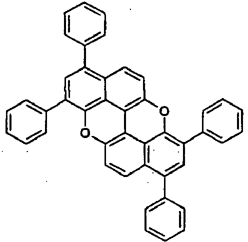 | 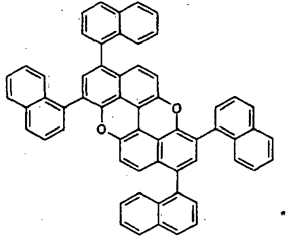 |
| (45) | (46) |
| 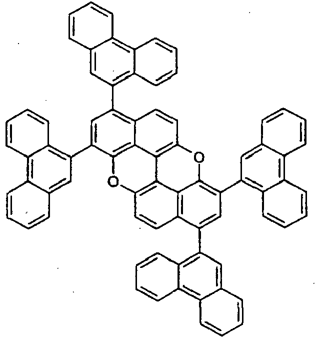 | 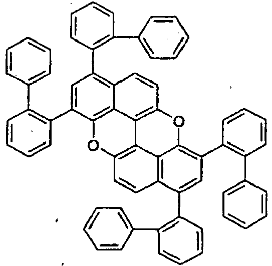 |
| (47) | (48) |
| 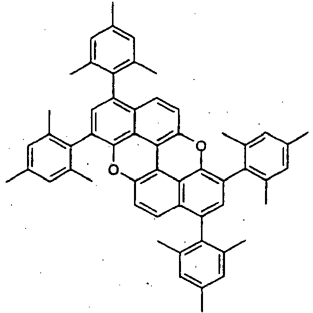 | 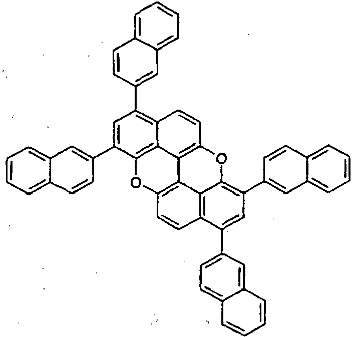 |
| (49) | (50) |
| 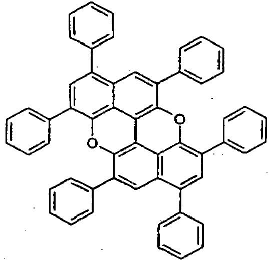 | 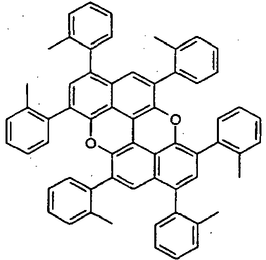 |
| (51) | (52) |
| 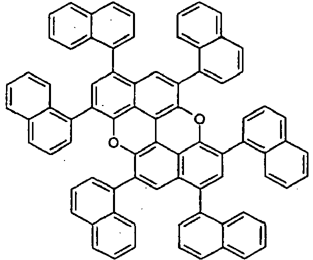 | 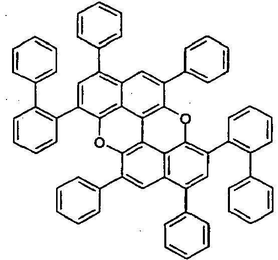 |
| (53) | (54) |
| 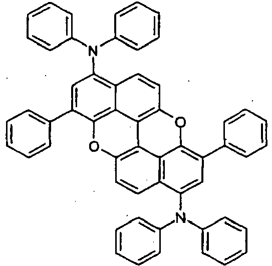 | 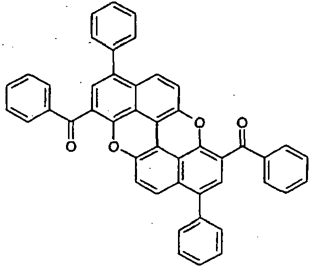 |
| (55) | (56) |
| 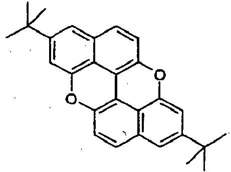 | 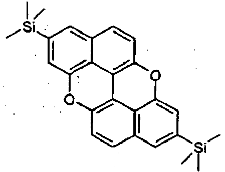 |
| (57) | (58) |
| 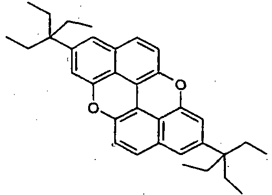 | 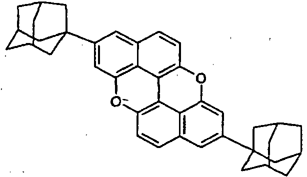 |
| (59) | (60) |
| 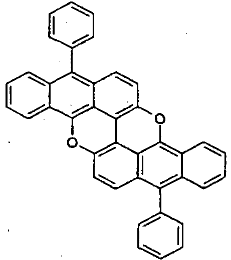 | 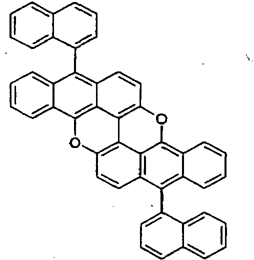 |
| (61) | (62) |
| 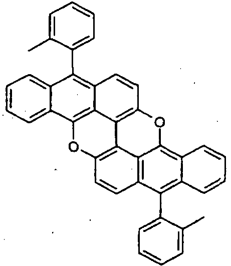 | 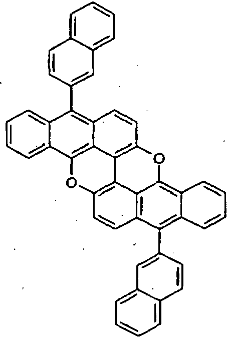 |
| (63) | (64) |
| 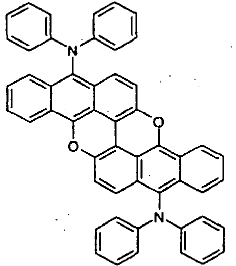 | 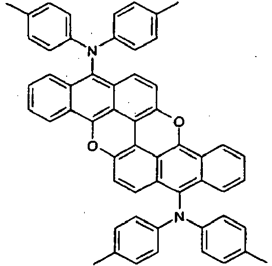 |
| (65) | (66) |
| 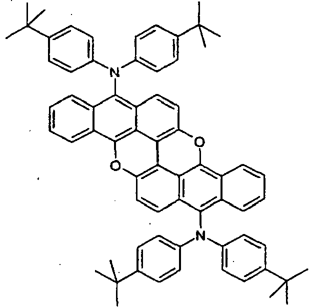 | 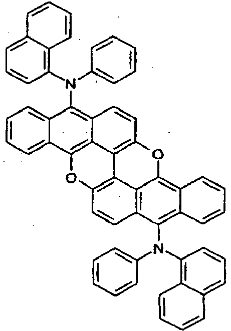 |
| (67) | (68) |
| 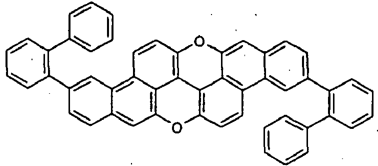 | 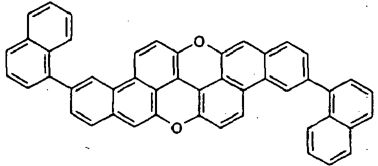 |
| (69) | (70) |
| 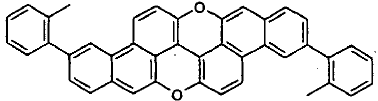 | 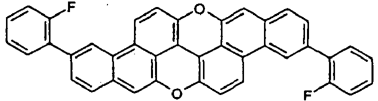 |
| (71) | (72) |
| 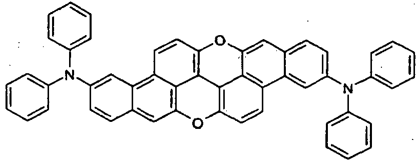 | 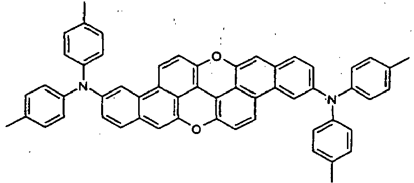 |
| (73) | (74) |
| 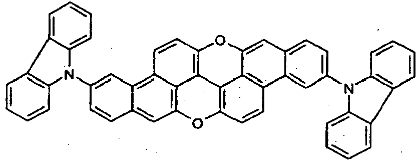 | 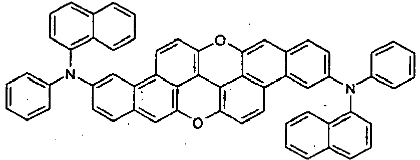 |
| (75) | (76) |
| 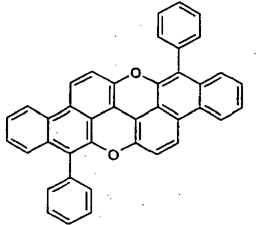 | 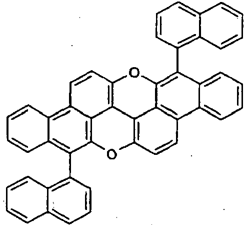 |
| (77) | (78) |
| 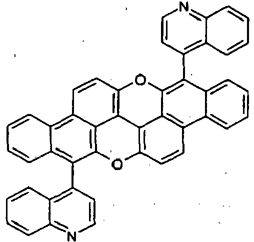 | 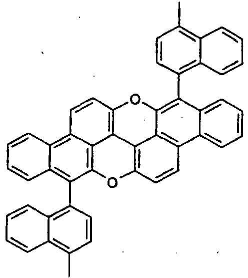 |
| (79) | (80) |
| 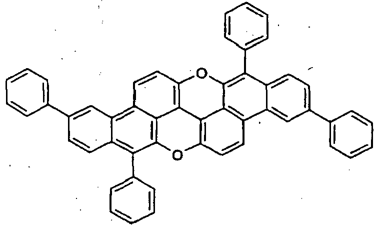 | 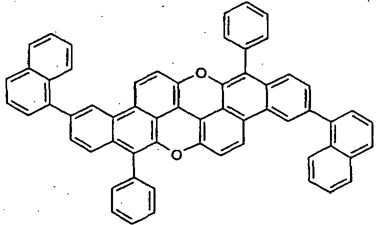 |
| (81) | (82) |
| 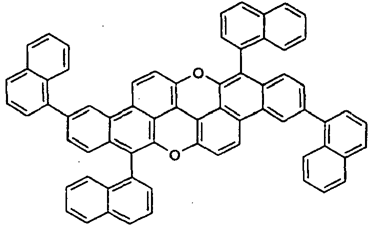 | 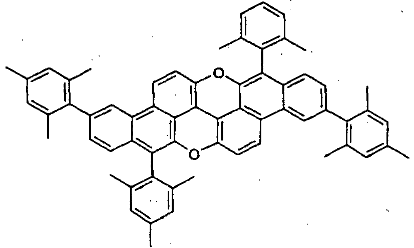 |
| (83) | (84) |
| 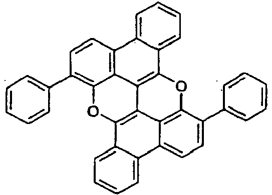 | 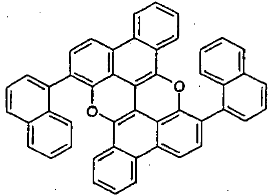 |
| (85) | (86) |
| 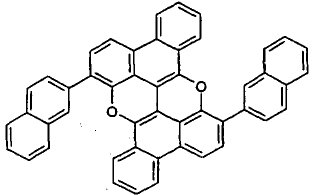 | 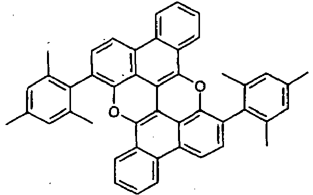 |
| (87) | (88) |
| 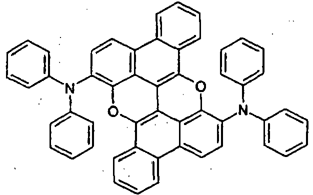 | 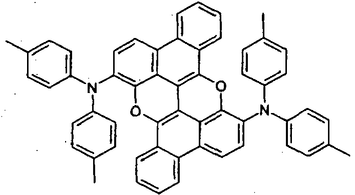 |
| (89) | (90) |
| 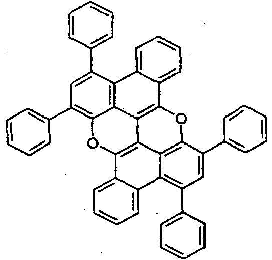 | 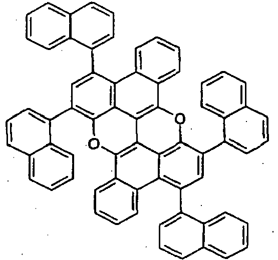 |
| (91) | (92) |
| 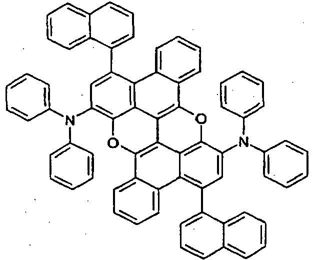 | 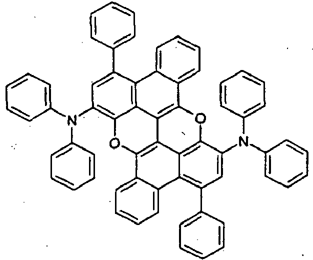 |
| (93) | (94) |
| 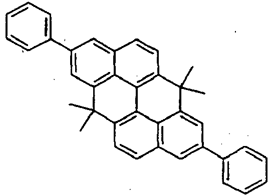 | 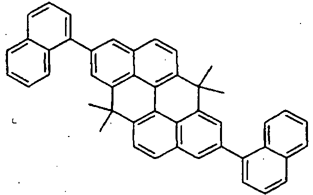 |
| (95) | (96) |
| 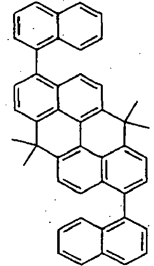 | 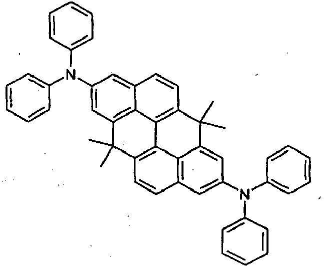 |
| (97) | (98) |
| 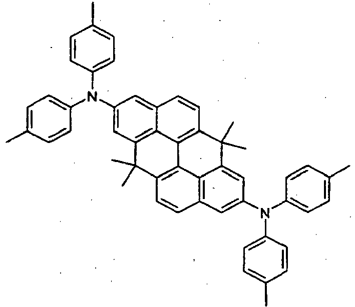 | 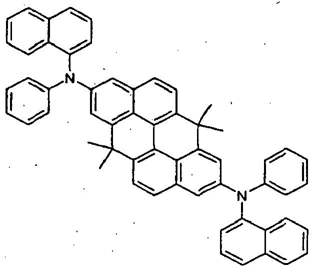 |
| (99) | (100) |
| 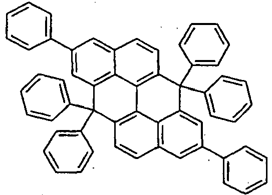 | 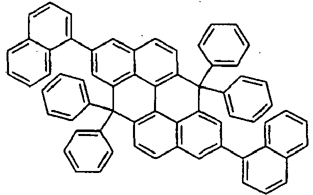 |
| (101) | (102) |
| 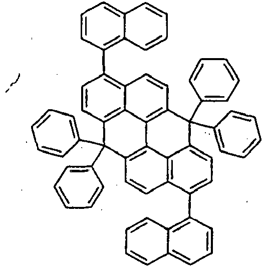 | 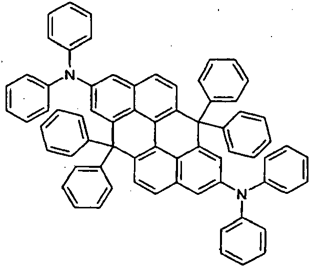 |
| (103) | (104) |
| 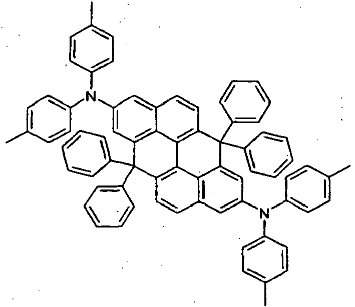 | 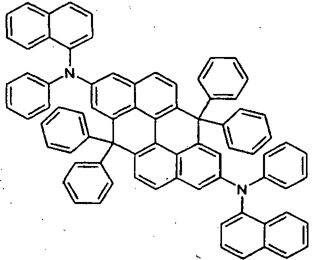 |
| (105) | (106) |
| 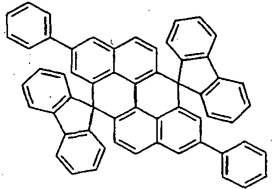 | 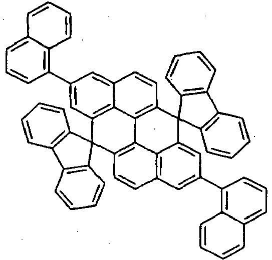 |
| (107) | (108) |
| 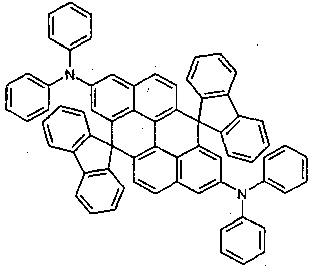 | 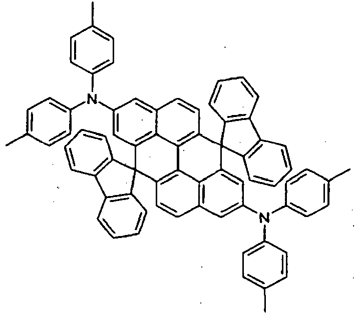 |
| (109) | (110) |
| 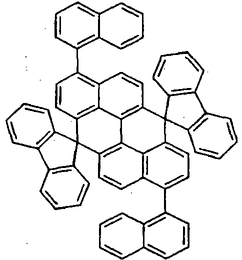 | 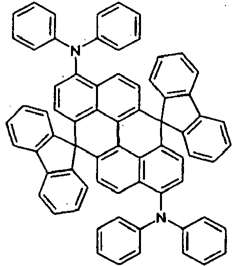 |
| (111) | (112) |
| 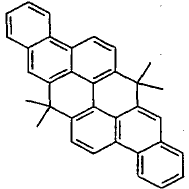 | 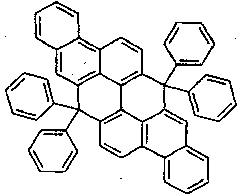 |
| (113) | (114) |
| 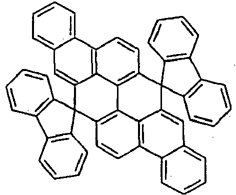 | 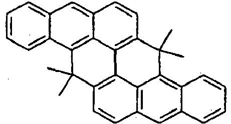 |
| (115) | (116) |
| 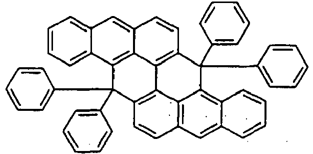 | 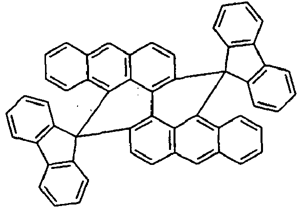 |
| (117) | (118) |
| 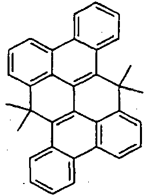 | 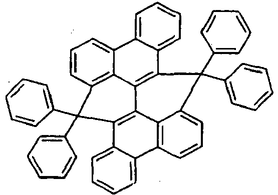 |
| (119) | (120) |
| 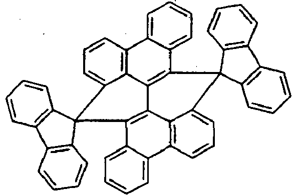 | 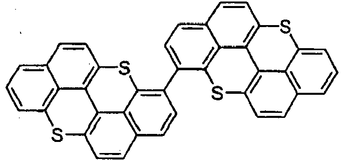 |
| (121) | (122) |
| 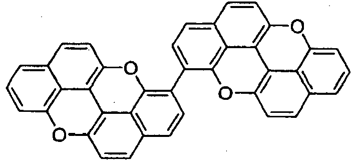 | |
| (123) | |
| 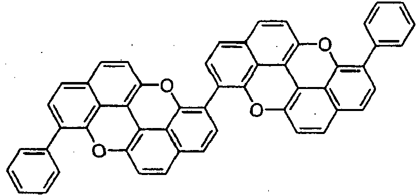 | |
| (124) | |
| 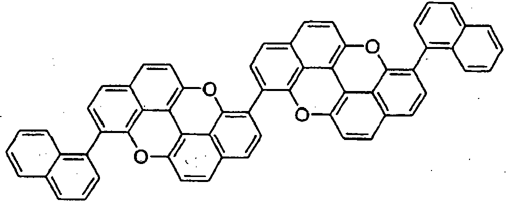 | |
| (125) | |
| 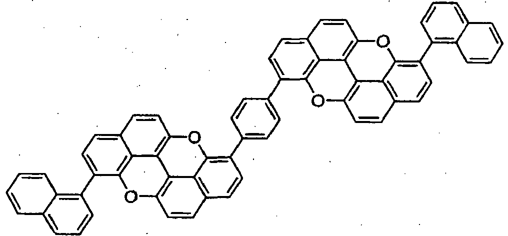 | |
| (126) | |
| 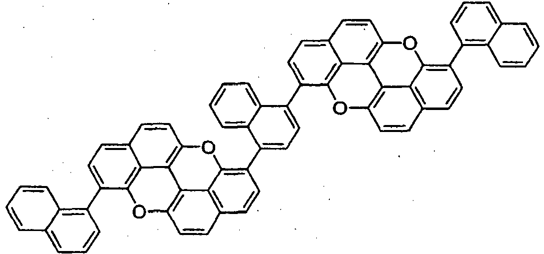 | |
| (127) | |
| 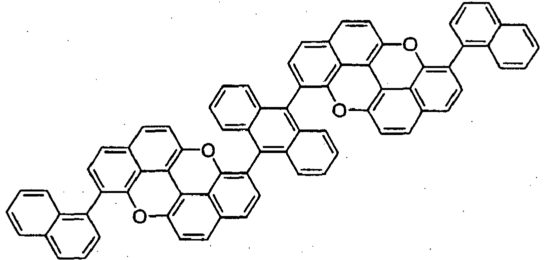 | |
| (128) | |
| 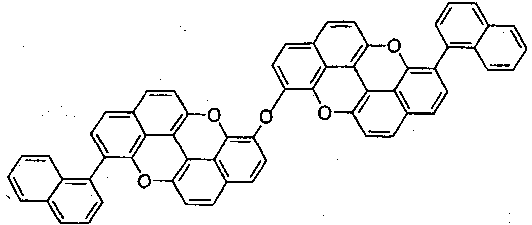 | |
| (129) | |
| 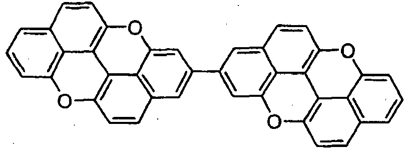 | |
| (130) | |
| 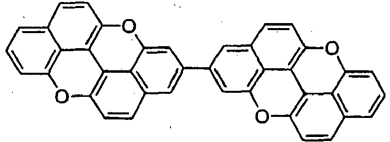 | |
| (131) | |
| 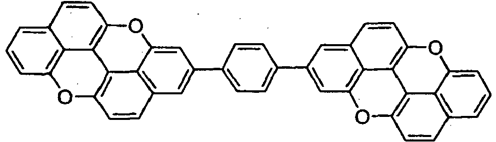 | |
| (132) | |
| 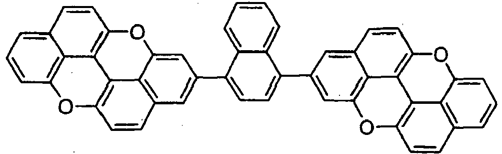 | |
| (133) | |
| 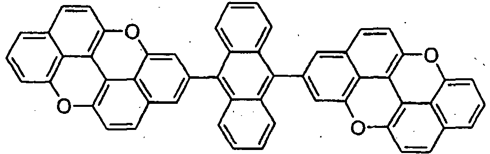 | |
| (134) | |
| 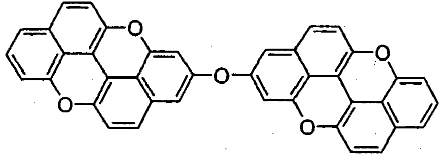 | |
| (135) | |
| 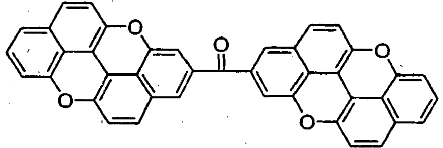 | |
| (136) | |
| 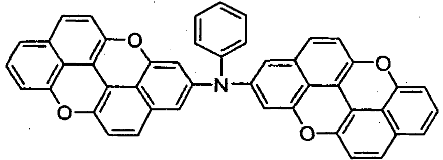 | |
| (137) | |
| 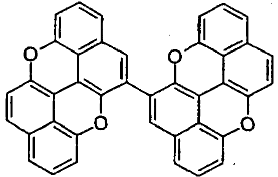 | |
| (138) | |
| 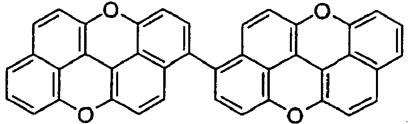 | |
| (139) | |
| 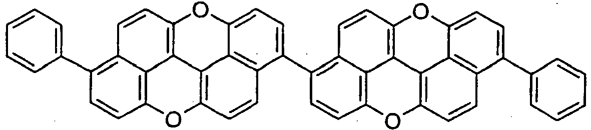 | |
| (140) | |
| 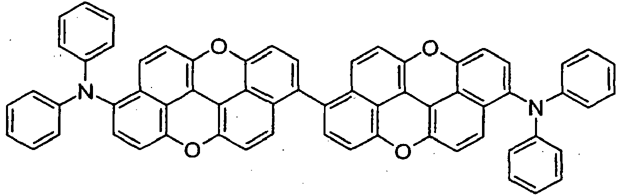 | |
| (141) | |
| 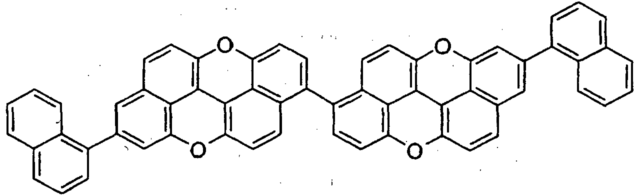 | |
| (142) | |
| 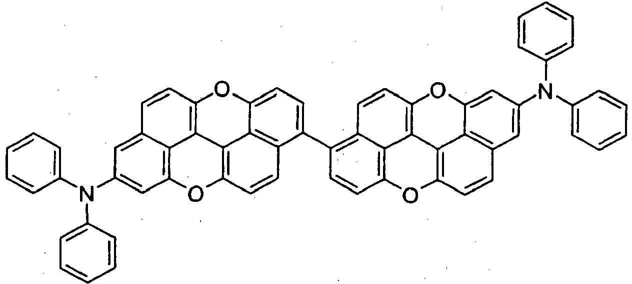 | |
| (143) | |
| 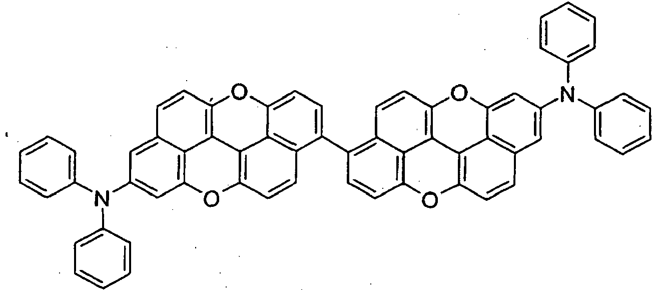 | |
| (144) | |
| 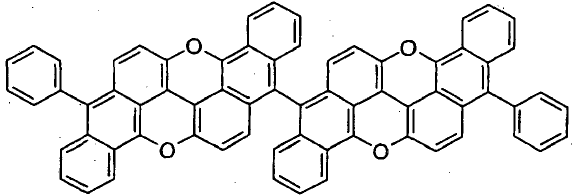 | |
| (145) | |
| 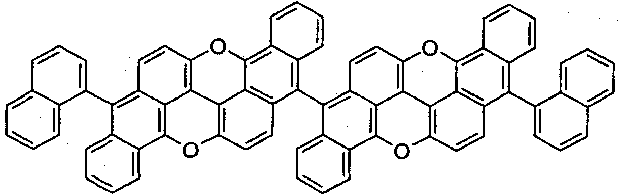 | |
| (146) | |
| 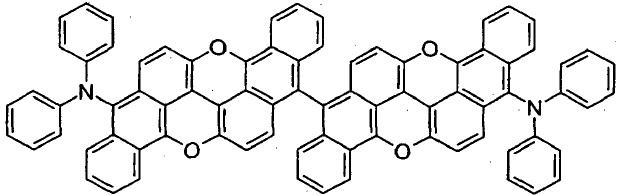 | |
| (147) | |
| 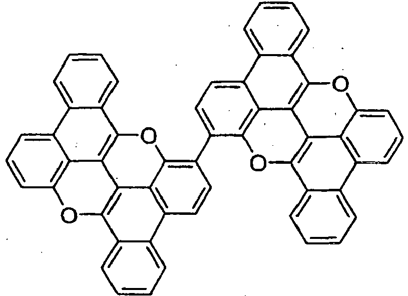 | |
| (148) | |
| 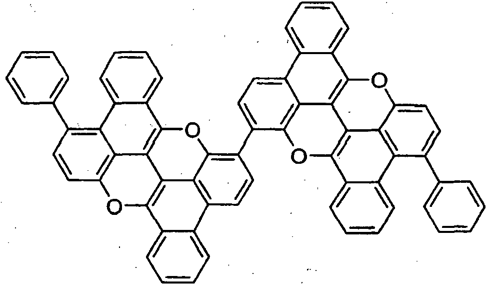 | |
| (149) | |
| 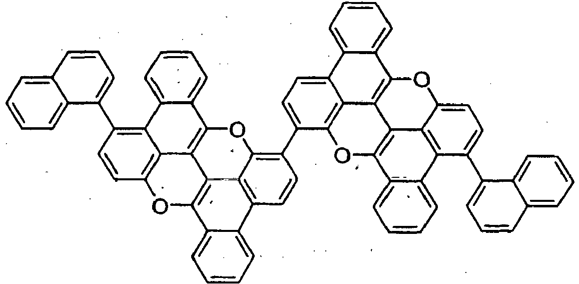 | |
| (150) | |
| 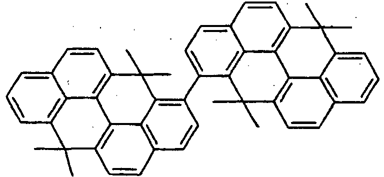 | |
| (151) | |
| 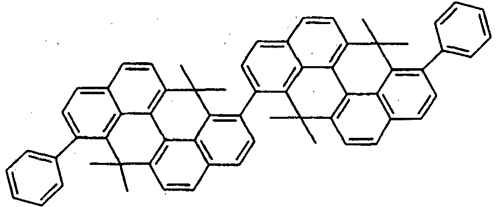 | |
| (152) | |
| 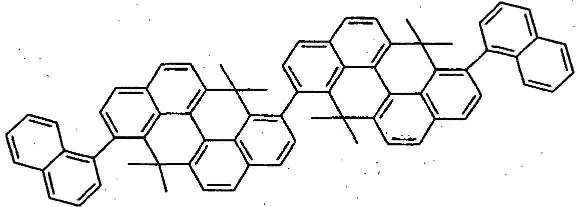 | |
| (153) | |
| 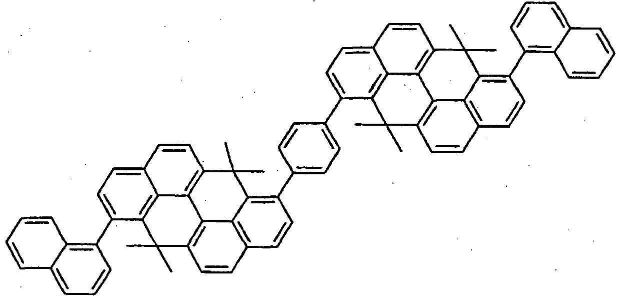 | |
| (154) | |
| 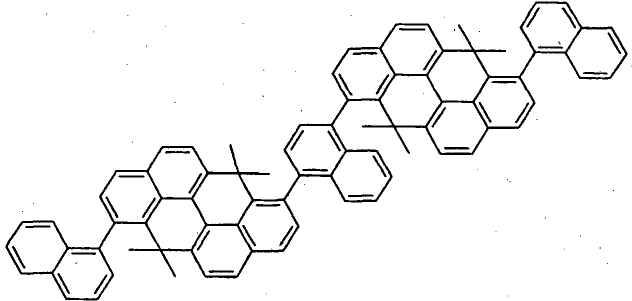 | |
| (155) | |
| 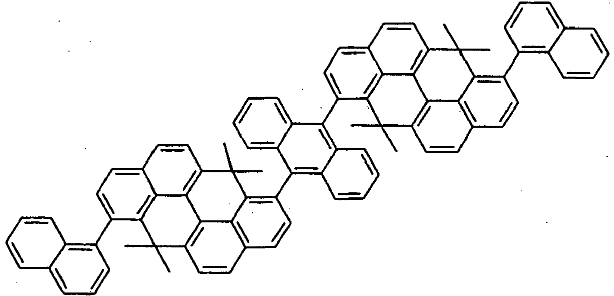 | |
| (156) | |
| 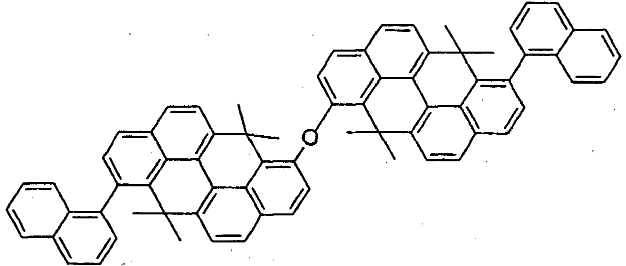 | |
| (157) | |
| 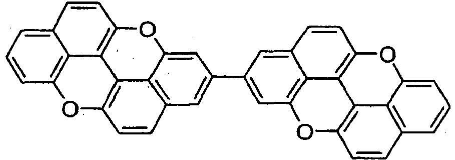 | |
| (158) | |
| 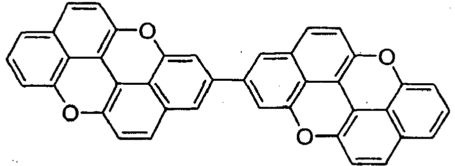 | |
| (159) | |
| 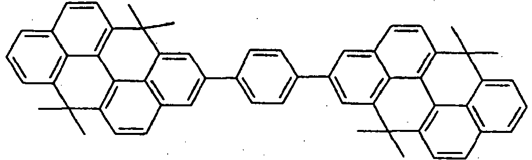 | |
| (160) | |
| 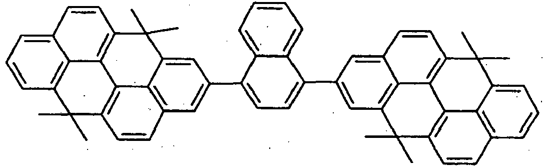 | |
| (161) | |
| 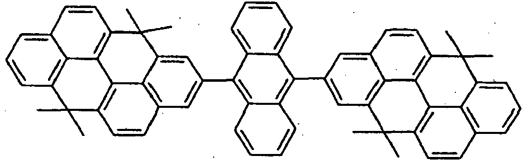 | |
| (162) | |
| 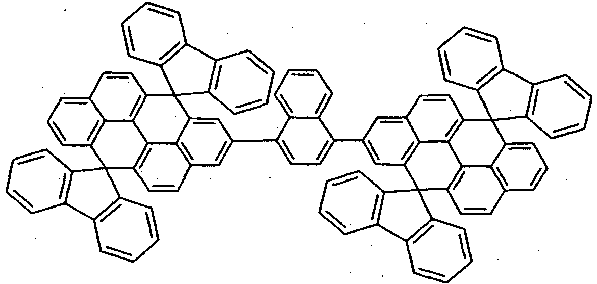 | |
| (163) | |
| 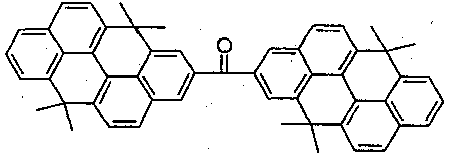 | |
| (164) | |
| 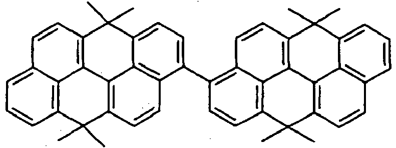 | |
| (165) | |
| 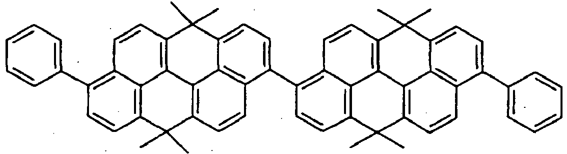 | |
| (166) | |
| 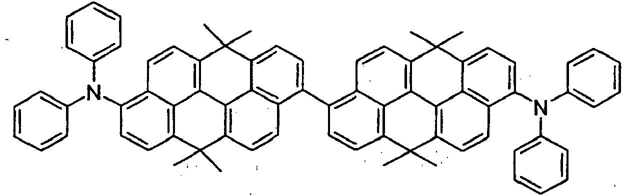 | |
| (167) | |
| 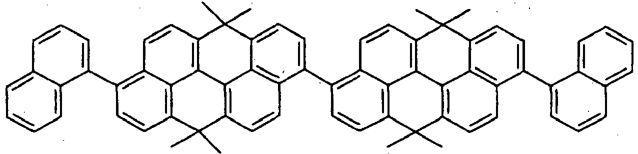 | |
| (168) | |
| 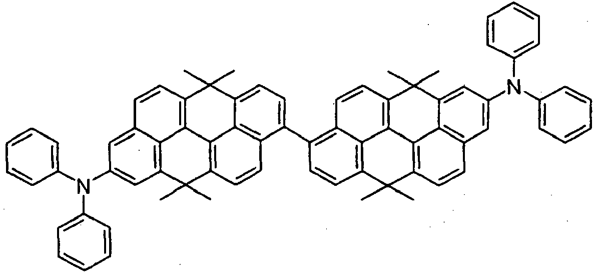 | |
| (169) | |
| 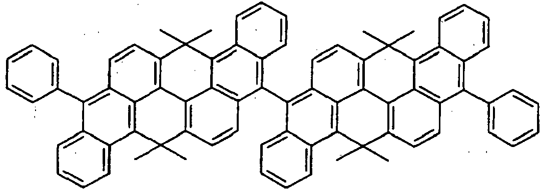 | |
| (170) | |
| 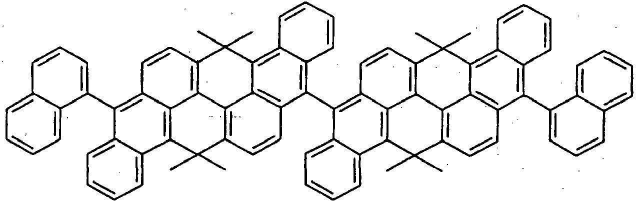 | |
| (171) | |
| 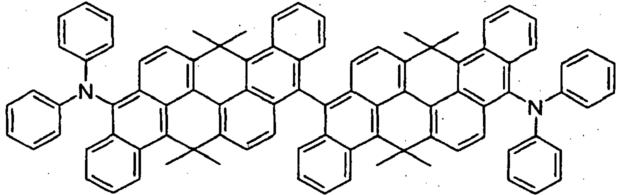 | |
| (172) | |
| 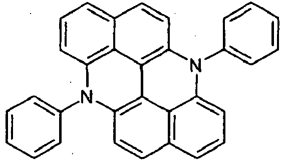 | 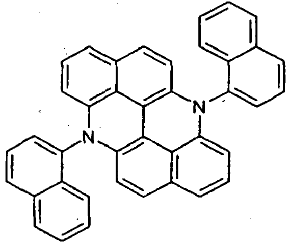 |
| (173) | (174) |
| 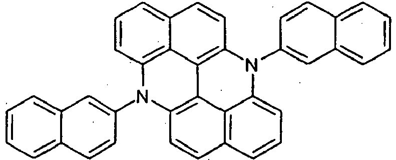 | 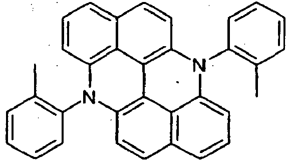 |
| (175) | (176) |
| 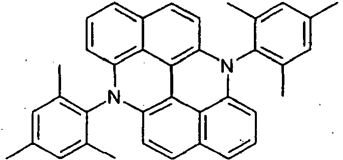 | 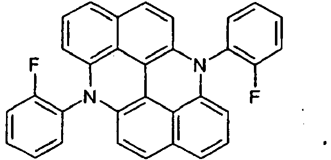 |
| (177) | (178) |
| 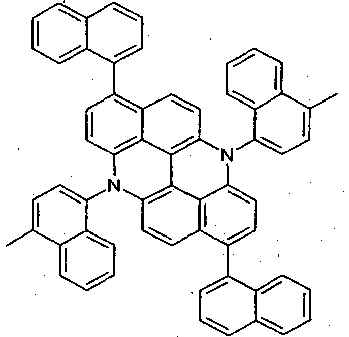 | 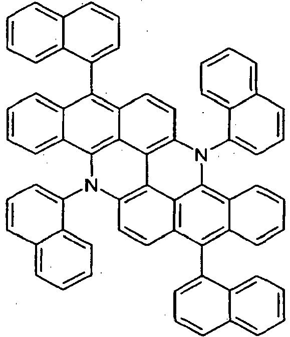 |
| (179) | (180) |
| 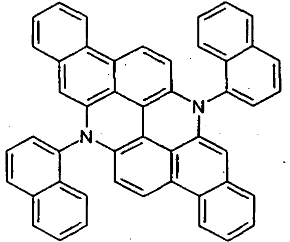 | 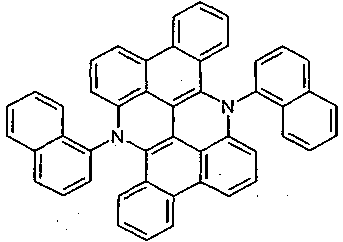 |
| (181) | (182) |
| 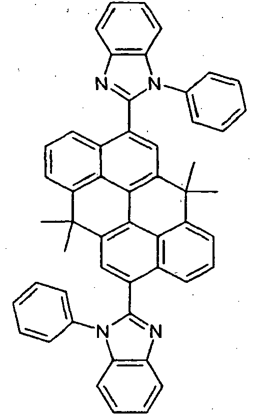 | 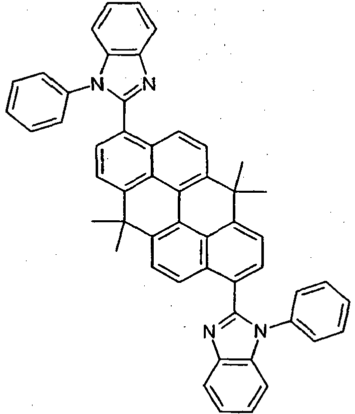 |
| (183) | (184) |

Die oben beschriebenen erfindungsgemäßen Verbindungen, insbesondere Verbindungen, welche mit reaktiven Abgangsgruppen, wie Brom, Iod, Boronsäure oder Boronsäureester, substituiert sind, können auch als Monomere zur Erzeugung entsprechender Polymere, Oligomere oder als Kern von Dendrimeren Verwendung finden. Die Polymerisation erfolgt dabei bevorzugt über die Halogenfunktionalität bzw. die Boronsäurefunktionalität.

Weiterer Gegenstand der Erfindung sind daher Polymere, Oligomere oder Dendrimere enthaltend eine oder mehrere Verbindungen gemäß Formel (1) bis (8), wobei ein oder mehrere Reste R, R¹ und/oder R² Bindungen der Verbindung gemäß Formel (1) bis (8) zum Polymer, Oligomer oder Dendrimer darstellen. Die Polymere, Oligomere oder Dendrimere können konjugiert, teilkonjugiert oder nicht-konjugiert sein.

Für die Wiederholeinheiten gemäß Formel (1) bis (8) gelten dieselben Bevorzugungen wie oben beschrieben.

Diese Monomere werden homopolymerisiert oder mit weiteren Monomeren copolymerisiert. Geeignete und bevorzugte Comonomere sind gewählt aus Fluorenen (z. B. gemäß EP 842208 oder WO 00/22026), Spirobifluorenen (z. B. gemäß EP 707020, EP 894107 oder WO 06/061181), Paraphenylenen (z. B. gemäß WO 92/18552), Carbazolen (z. B. gemäß WO 04/070772 oder WO 04/113468), Thiophenen (z. B. gemäß EP 1028136), Dihydrophenanthrenen (z. B. gemäß WO 05/014689), cis- und trans-Indenofluorenen (z. B. gemäß WO 04/041901 oder WO 04/113412), Ketonen (z. B. gemäß WO 05/040302), Phenanthrenen (z. B. gemäß WO 05/104264 oder WO 07/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß der nicht offen gelegten Anmeldung DE 102005060473.0) oder phosphoreszierende Metallkomplexe (z. B. gemäß WO 06/003000), und/oder Ladungstransporteinheiten, insbesondere solche basierend auf Triarylaminen.

Abhängig davon, ob die Einheit gemäß Formel (1) bis (8) ein, zwei oder mehr funktionelle Gruppen trägt, wird sie als Endgruppe, als lineare Wiederholeinheit oder als Verzweigungseinheit in das Polymer, Oligomer oder Dendrimer eingebaut.

Die erfindungsgemäßen Verbindungen gemäß Formel (1) bis (8) können nach dem Fachmann bekannten Syntheseschritten dargestellt werden. So lassen sich die verschiedenen substituierten Grundgerüste beispielsweise durch oxidative Kupplung von unsubstituiertem und substituierten 1,1'-Bi- 2-naphtholen darstellen, wie in Schema 1 am Beispiel des peri-Xanthenoxanthens und des 6,6'-Dibrom-peri-xanthenoxanthens gezeigt. Die oxidative Cyclisierung erfolgt entweder durch Umsetzung mit stöchiometrischen bzw. überstöchiometrischen Mengen an Übergangsmetalloxiden wie MnO₂, CuO, etc. (DE 545212) oder durch Luftsauerstoff unter Zusatz katalytischer Mengen an Übergangsmetallverbindungen und einer Base (DE 510443).

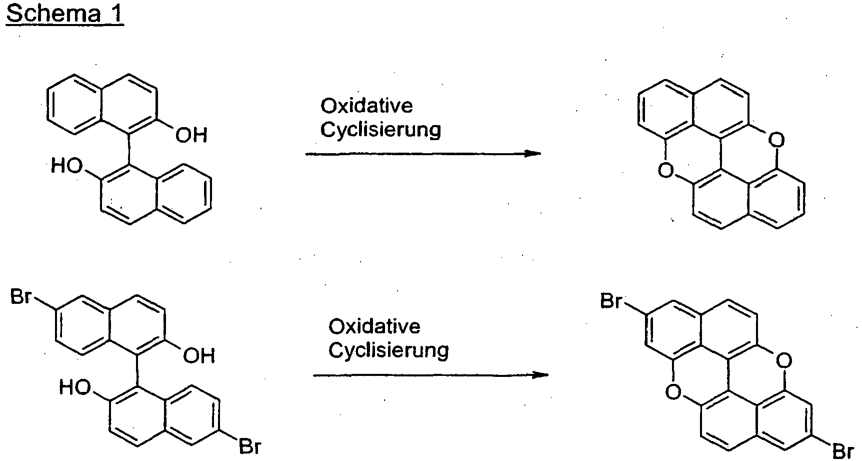

Überraschend wurde gefunden, dass dieser Kupplung auch Alkyl-, Vinyl-, Aryl- und Diarylamino-substituierte 1,1'-Bi-2-naphthole zugänglich sind (Schema 2 und 3), ohne dass es zur oxidativen Zersetzung der genannten Reste kommt. Die als Edukte dieser Kupplung verwendeten 6-Alkyl-, 6-Vinyl-, 6-Aryl- und 6-Diarylamino-substituierten 1,1'-Bi-2-naphthole sind leicht aus 6-Brom-2-naphthol durch Umsetzung mit Alkyl-, Vinyl- oder Aryl-Boronsäuren in einer Suzuki-Kupplung bzw. mit Arylaminen in einer Hartwig-Buchwald-Aminierung und anschließende oxidative Kupplung der 6-substituierten 2-Naphthole zu den 6,6'-substituierten 1,1'-Bi-2-naphtholen mit wässrigem Eisen(III)chlorid (z. B. Ding et al., Tetrahedron 1996, 52, 1005) zugänglich.

In analoger Weise lassen sich auch 1,1'-Bi-2-anthrole zu Verbindungen der Formel (2), 9,9'-Biphenanthren-10,10'-diole zu Verbindungen der Formel (3) und 1,1'-Biphenanthren-2,2'-diole zu Verbindungen der Formel (4) kuppleln.

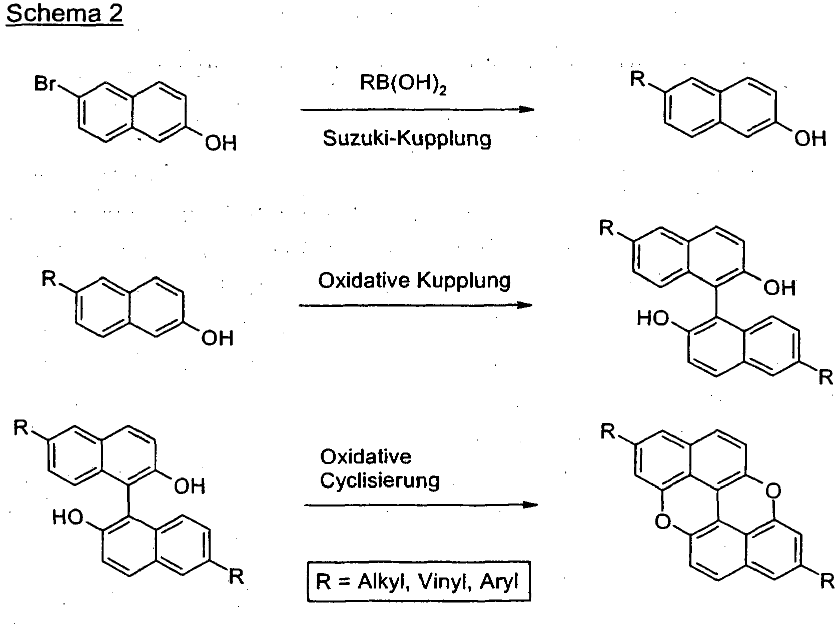

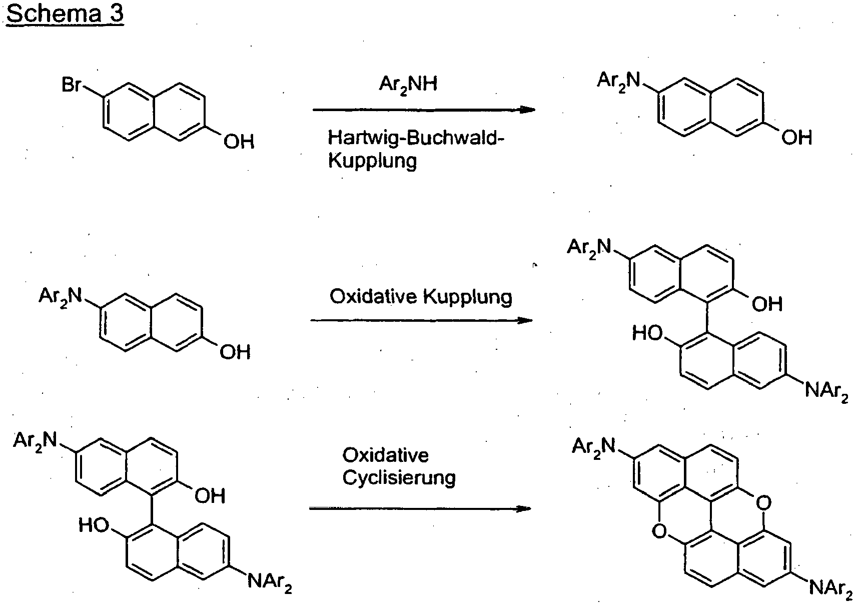

Eine direkte Funtionalisierung der Grundgerüste, z. B. durch Bromierung, ist ebenso möglich, wie in Schema 4 exemplarisch für die peri-Xanthenoxanthene gezeigt. Die Bromierung erfolgt bevorzugt mit N-Bromsuccinimid (NBS) in einem inerten hochsiedenden Lösemittel wie o-Dichlorbenzol oder Nitrobenzol, wobei ein geeignetes stöchiometrischem Verhältnis von NBS zu peri-Xanthenoxanthen zu 5-Mono, 5,5'-Di-, 5,5',7,7'-Tetra- bzw. 3,3',5,5',7,7'-Hexabromiden führt. Im Falle der Monobromierung entstehen neben ca. 15 % unverändertem peri-Xanthenoxanthen ca. 15 % 5,5'-Dibrom-peri-xanthenoxanthen, wobei ersteres durch Auskochen der Mischung mit Dichlormethan leicht entfernt werden kann, und letzteres aufgrund seine Schwerlöslichkeit durch fraktionierte Kristallisation aus Chlorbenzol abgetrennt werden kann.

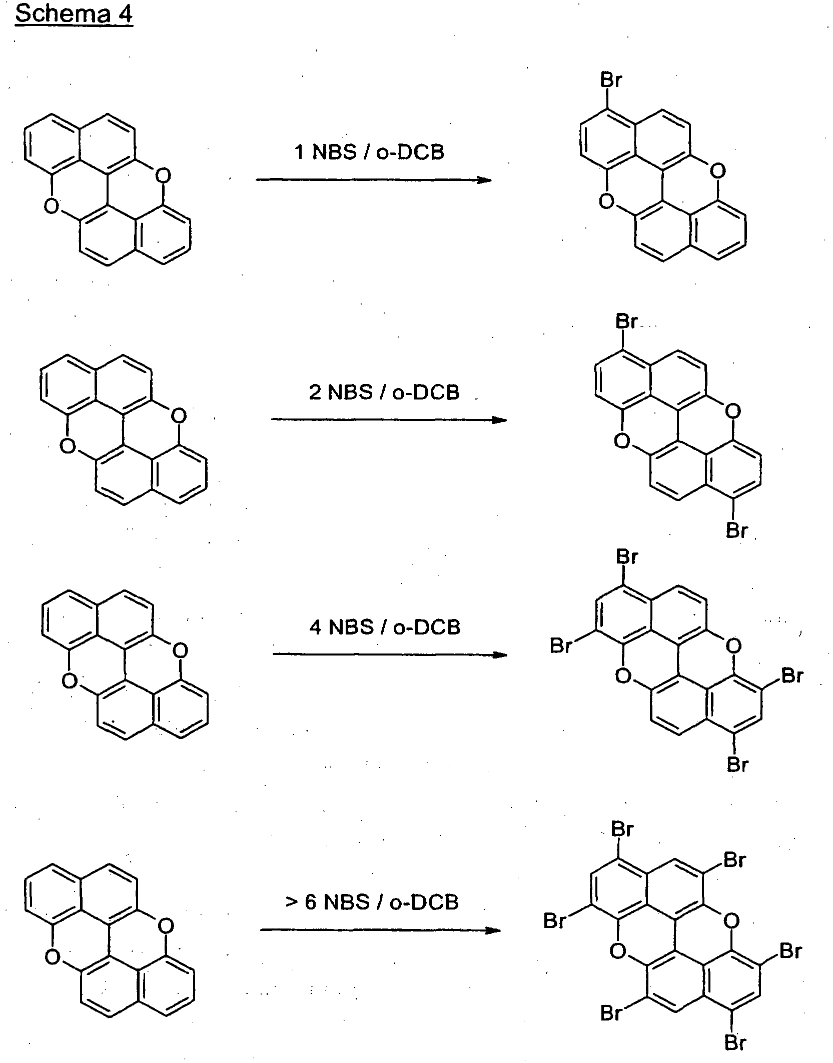

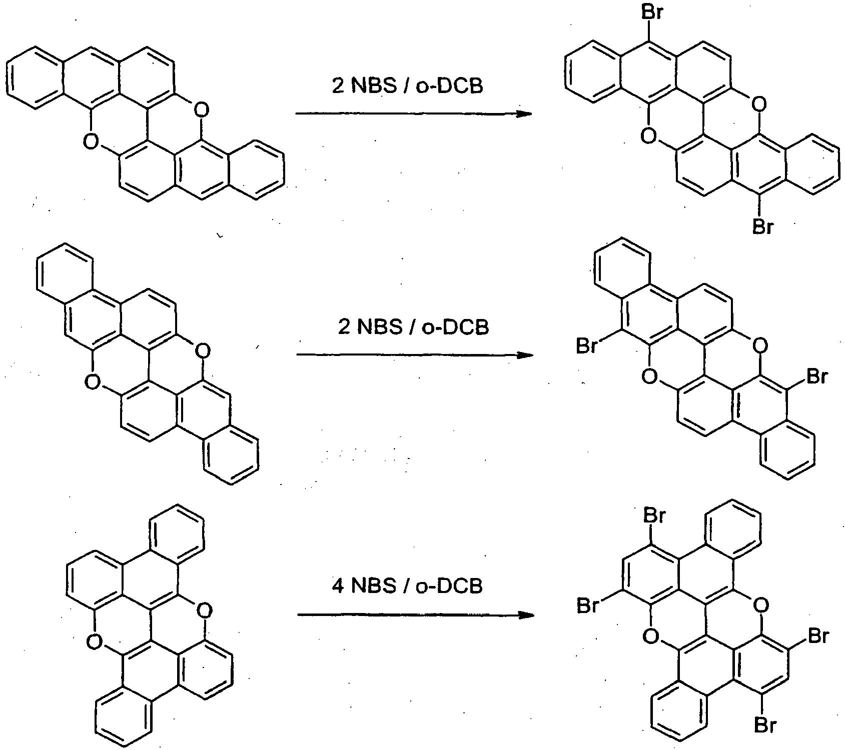

Die direkte Funktionalisierung (Bromierung) ist zur oben beschriebenen oxidativen Cyclisierung bezüglich der Regiochemie komplementär. Daher erlauben beide Methoden zusammen die gezielte Funktionalisierung in allen möglichen Positionen.

Selbstverständlich können die so erhaltenen 5-Mono, 5,5'-Di-, 6,6'-Di-, 5,5',7,7'-Tetra- bzw. 3,3',5,5',7,7'-Hexabromide durch gängige Methoden weiter umgewandelt werden, wie in Schema 5 exemplarisch am 6,6'-Dibrom-peri-xanthenoxanthen gezeigt.

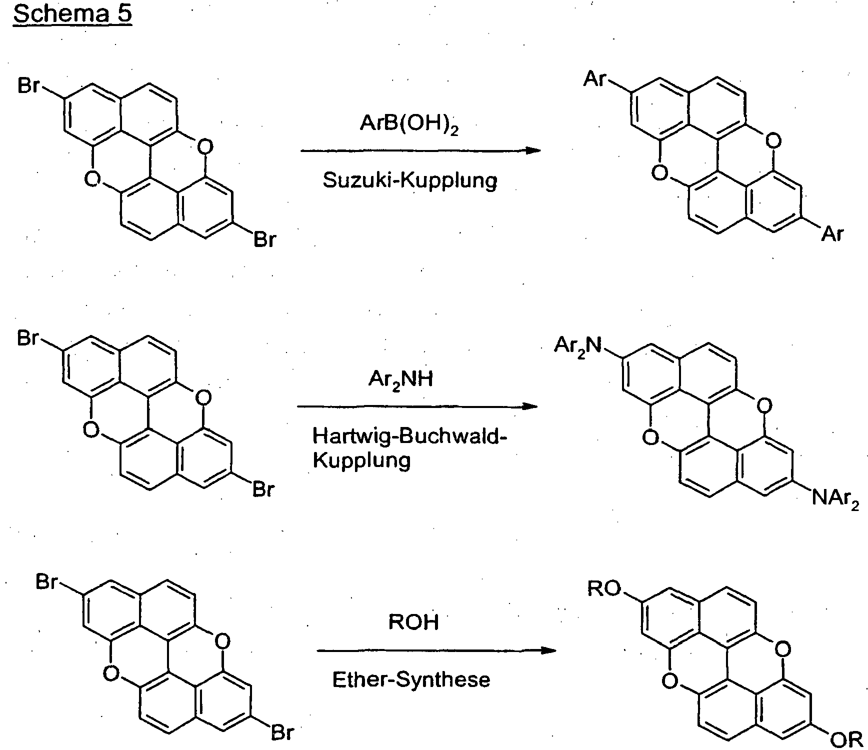

Neben der Bromierung gelingt auch die direkte Umwandlung der Grundkörper in Ketone durch Friedel-Crafts-Reaktion mit aromatischen Säurechloriden in Gegenwart von Lewis-Säuren wie Aluminiumchlorid (Schema 6) (Pummerer et al., Annalen der Chemie 1942, 553, 103).

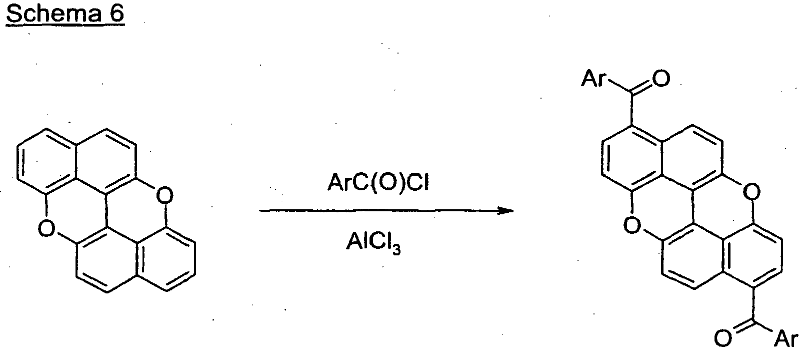

Durch direkte Lithiierung der unsubstituierten oder substituierten Grundköper mit Lithiierungsreagenzien wie n-BuLi, sec-BuLi oder tert-BuLi in Ethern wie THF, gegebenenfalls unter Zusatz von Additiven wie TMEDA, können gezielt die zu den Heteroatomen benachbarten Positionen funktionalisiert werden (Schema 7). Völlig analog lassen sich die aus den Bromiden durch Transmetallierung erhaltenen Organolithiumverbindungen umsetzen. Die Organolithium-Verbindungen können nach üblichen Methoden mit Elektrophilen wie Iod, Boronsäureestern, Organozinnverbindungen, Chlorphosphinen, Nitrilen, Carbamoylchloriden, etc. umgesetzt oder mit Oxidationsmitteln wie wasserfreiem Kupfer(II)chlorid dimerisiert (Schema 8) werden.

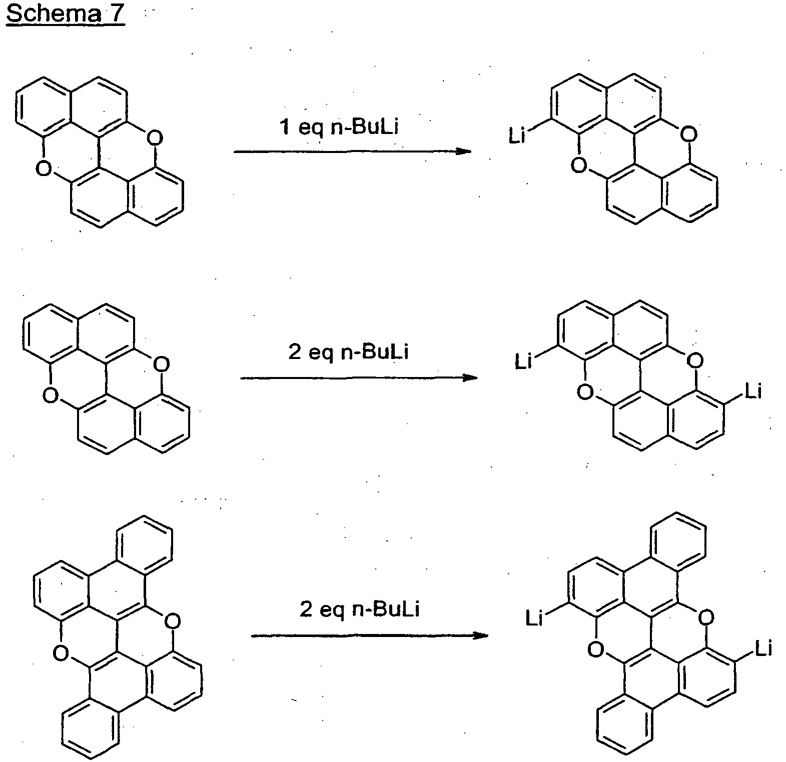

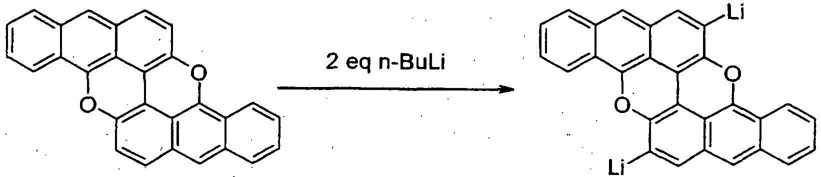

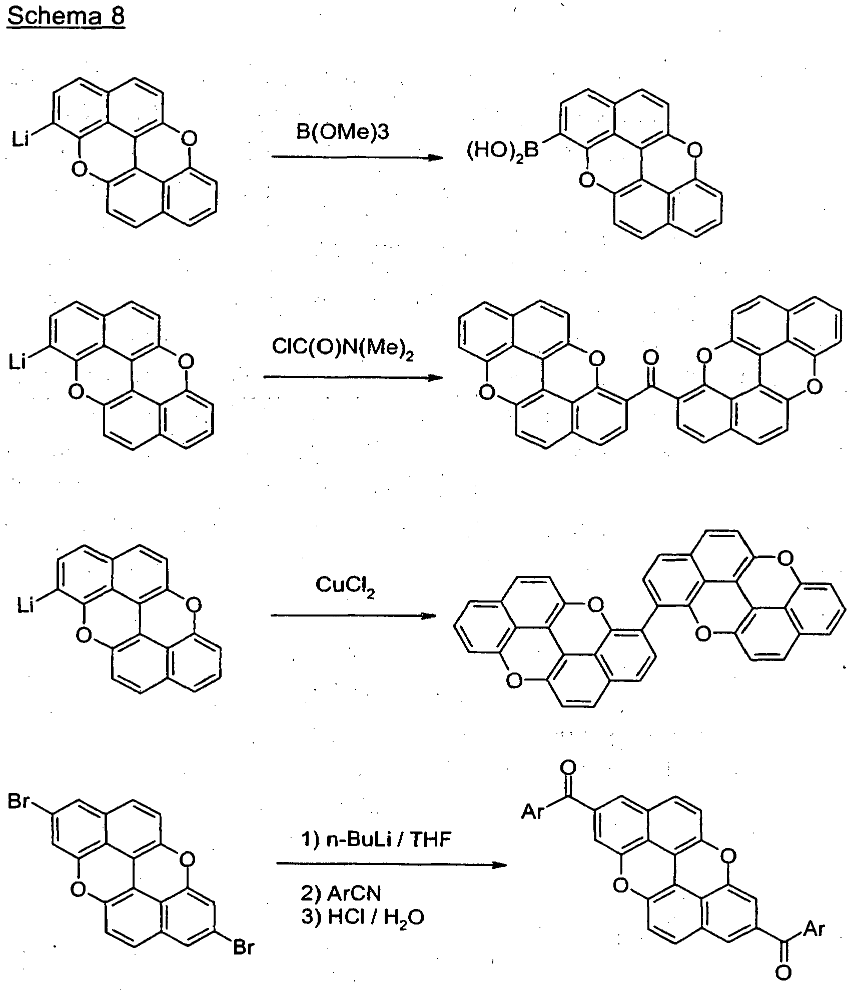

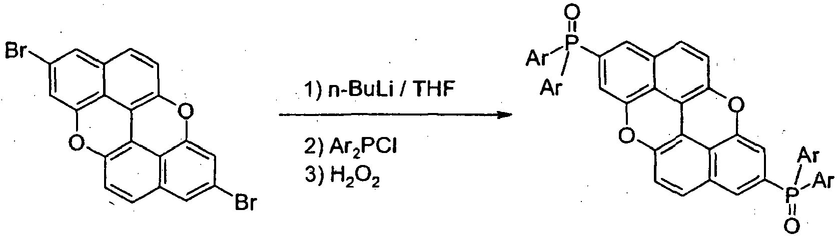

Die grundlegende Synthesestrategie zur Darstellung der reinen Kohlenwasserstoffe der Formel (1) bis (4) mit X = C(R¹)₂ ist in Schema 9 exemplarisch am Beispiel des Dihydro-anthanthrens aufgezeigt. Dabei können statt Methyllithium im ersten Schritt generell reaktive Organometallverbindungen, wie beispielsweise allgemein Organolithium- oder Grignardverbindungen verwendet werden. Zwischenstufen sind jeweils tertiäre Alkohole, die aus geeignet substituierten 1,1'-Bi-2-naphthylen durch gängige Methoden zugänglich sind und abschließend durch säurekatalysierte dehydrierende Cyclisierung geschlossen werden. Die Grundgerüste können anschließend in völliger Analogie zum oben beschriebenen durch Bromierung oder Friedel-Crafts-Reaktion weiter funktionalisiert werden. Selbstverständlich können auch hier schon funktionalisierte Bausteine in der säurekatalysierten dehydrierenden Cyclisierung eingesetzt werden.

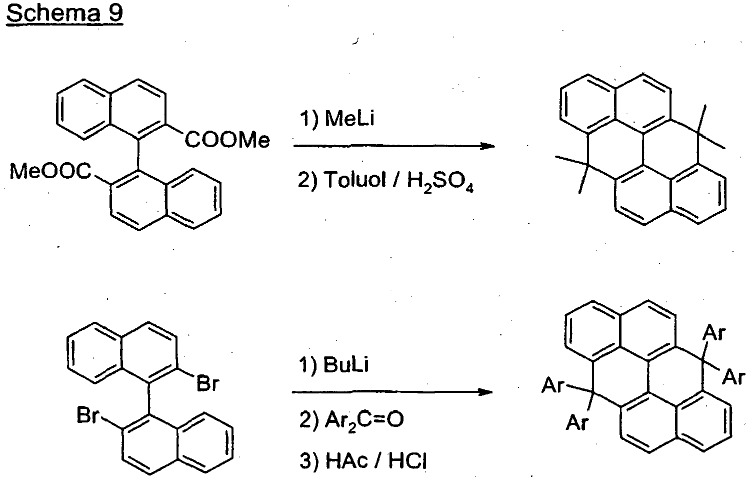

Ein weiterer Gegenstand der Erfindung ist ein Verfahren zur Herstellung der Verbindungen gemäß den Formeln (1) bis (4), in denen X = O gilt, gekennzeichnet durch oxidative Cyclisierung des zuvor entsprechend funktionalisierten Binaphthol- bzw. Bianthrol- bzw. Biphenanthrolderivats gemäß Schema 2.

Nochmals ein weiterer Gegenstand der Erfindung ist ein Verfahren zur Herstellung der Verbindungen gemäß den Formeln (1) bis (4), in denen X = C(R¹)₂ gilt, gekennzeichnet durch säurekatalysierte dehydratisierende Cyclisierung des gegebenenfalls zuvor entsprechend funktionalisierten Binaphthyl- bzw. Bianthryl- bzw. Biphenanthrylderivats, welches jeweils mit zwei Gruppen der Formel -C(R¹)₂(OH) in den ortho-Positionen zur Verknüpfung des Binapthyls bzw. Bianthryls bzw. Biphenanthryls substituiert ist, gemäß Schema 9.

Die funktionalisierten Verbindungen, beispielsweise die bromierten Verbindungen, können entweder direkt oder nach Überführung in ein Boronsäurederivat als Monomere zur Herstellung von Polymeren, Oligomeren oder Dendrimeren eingesetzt werden.

Die Verbindungen gemäß Formel (1) bis (8) eignen sich für den Einsatz in elektronischen Vorrichtungen, insbesondere in organischen Elektrolumineszenzvorrichtungen (OLEDs, PLEDs). Abhängig von der Substitution werden die Verbindungen in unterschiedlichen Funktionen und Schichten eingesetzt.

Ein weiterer Gegenstand der Erfindung ist daher die Verwendung von Verbindungen gemäß Formel (1) bis (8) in elektronischen Vorrichtungen, insbesondere in organischen Elektrolumineszenzvorrichtungen.

Nochmals ein weiterer Gegenstand der Erfindung sind elektronische Vorrichtungen, enthaltend mindestens eine Verbindung gemäß Formel (1) bis (8), insbesondere organische Elektrolumineszenzvorrichtungen, enthaltend Anode, Kathode und mindestens eine emittierende Schicht, dadurch gekennzeichnet, dass mindestens eine organische Schicht, die eine emittierende Schicht oder eine andere Schicht sein kann, mindestens eine Verbindung gemäß Formel (1) bis (8) enthält.

Außer Kathode, Anode und der emittierenden Schicht kann die organische Elektrolumineszenzvorrichtung noch weitere Schichten enthalten. Diese sind beispielsweise gewählt aus Lochinjektionsschicht, Lochtransportschicht, Elektronentransportschicht, Elektroneninjektionsschicht und/oder Charge-Generation Layer (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer)*.* Es sei aber darauf hingewiesen, dass nicht notwendigerweise jede dieser Schichten vorhanden sein muss.

In einer weiteren bevorzugten Ausführungsform der Erfindung enthält die organische Elektrolumineszenzvorrichtung mehrere emittierende Schichten, wobei mindestens eine organische Schicht mindestens eine Verbindung gemäß Formel (1) bis (8) enthält. Besonders bevorzugt weisen diese Emissionsschichten insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei mindestens eine dieser Schichten mindestens eine Verbindung gemäß Formel (1) bis (8) enthält und wobei die drei Schichten blaue, grüne und orange oder rote Emission zeigen (für den prinzipiellen Aufbau siehe z. B. WO 05/011013). Ebenso eignen sich für weiße Emission Emitter, welche breitbandige Emissionsbanden aufweisen und dadurch weiße Emission zeigen.

In einer bevorzugten Ausführungsform der Erfindung werden die Verbindungen gemäß Formel (1) bis (8) als emittierende Materialien eingesetzt. Die Verbindungen sind insbesondere dann als emittierende Verbindungen geeignet, wenn die Symbole X für O, S oder N(R¹) stehen, insbesondere für O. Abhängig von der Natur der Substituenten R und der Position, in der diese Substituenten gebunden sind, eignen sich die erfindungsgemäßen Verbindungen als emittierende Materialien für unterschiedliche Emissionsfarben. So zeigen beispielsweise die Verbindungen gemäß Formel (9), in denen die Substituenten R für aromatische oder heteroaromatische Gruppen stehen, blaue Emission, während Verbindungen der Formel (10) oder (11) mit den gleichen Substituenten R blaugrüne Emission zeigen. Verbindungen der Formel (12), welche vier aromatische oder heteroaromatische Substituenten R aufweisen, zeigen dagegen intensive grüne Emission.

Der Anteil der Verbindung gemäß Formel (1) bis (8) in der Mischung der emittierenden Schicht beträgt zwischen 0.1 und 50.0 Gew.-%, bevorzugt zwischen 0.5 und 20.0 Gew.-%, besonders bevorzugt zwischen 1.0 und 10.0 Gew.-%. Entsprechend beträgt der Anteil des Hostmaterials zwischen 50.0 und 99.9 Gew.-%, bevorzugt zwischen 80.0 und 99.5 Gew.-%, besonders bevorzugt zwischen 90.0 und 99.0 Gew.-%.

Als Hostmaterialien kommen Materialien verschiedener Stoffklassen in Frage. Bevorzugte Hostmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 04/081017), der lochleitenden Verbindungen (z. B. gemäß WO 04/058911), der elektronenleitenden Verbindungen, insbesondere Ketone, Phosphinoxide, Sulfoxide, etc. (z. B. gemäß WO 05/084081 und WO 05/084082), der Atropisomere (z. B. gemäß WO 06/048268) oder der Boronsäurederivate (z. B. gemäß WO 06/117052). Weiterhin kommen als Hostmaterialien auch die erfindungsgemäßen Verbindungen gemäß Formel (1) bis (8) in Frage, insbesondere die unten für diese Verwendung beschriebenen Derivate. Besonders bevorzugte Hostmaterialien sind außer den erfindungsgemäßen Verbindungen ausgewählt aus den Klassen der Oligoarylene, enthaltend Naphthalin, Anthracen und/oder Pyren oder Atropisomere dieser Verbindungen, der Oligoarylenvinylene, der Ketone, der Phosphinoxide und der Sulfoxide. Ganz besonders bevorzugte Hostmaterialien sind außer den erfindungsgemäßen Verbindungen ausgewählt aus den Klassen der Oligoarylene, enthaltend Anthracen und/oder Pyren oder Atropisomere dieser Verbindungen, der Phosphinoxide und der Sulfoxide. Unter einem Oligoarylen im Sinne dieser Erfindung soll eine Verbindung verstanden werden, in der mindestens drei Aryl- bzw. Arylengruppen aneinander gebunden sind.

In einer weiteren Ausführungsform der Erfindung werden die Verbindungen gemäß Formel (1) bis (8) als Hostmaterial eingesetzt. Als Hostmaterial eignen sich besonders Verbindungen, in denen die Symbole X für C(R¹)₂ stehen. Weiterhin bevorzugt sind in diesem Fall bevorzugt ein oder mehrere Substituenten R aus Aryl- oder Heteroarylgruppen gewählt, insbesondere Phenyl, o-, m- oder p-Biphenyl, 1- oder 2-Naphthyl, 2-Fluorenyl und 2-Spirobifluorenyl, welche jeweils durch einen oder mehrere Reste R² substituiert sein können. Die Verbindungen können als Hostmaterial für fluoreszierende oder phosphoreszierende Dotanden eingesetzt werden, insbesondere für fluoreszierende Dotanden.

Unter einem Hostmaterial wird in einem System aus Host und Dotand diejenige Komponente verstanden, die in dem System im höheren Anteil vorliegt. Bei einem System aus einem Host und mehreren Dotanden wird als Host diejenige Komponente verstanden, deren Anteil der höchste in der Mischung ist.

Der Anteil des Hostmaterials gemäß Formel (1) bis (8) in der emittierenden Schicht beträgt zwischen 50.0 und 99.9 Gew.-%, bevorzugt zwischen 80.0 und 99.5 Gew.-%, besonders bevorzugt zwischen 90.0 und 99.0 Gew.-%. Entsprechend beträgt der Anteil des Dotanden zwischen 0.1 und 50.0 Gew.-%, bevorzugt zwischen 0.5 und 20.0 Gew.-%, besonders bevorzugt zwischen 1.0 und 10.0 Gew.-%.

Bevorzugte Dotanden in fluoreszierenden Vorrichtungen sind gewählt aus der Klasse der Monostyrylamine, der Distyrylamine, der Tristyrylamine, der Tetrastyrylamine und der Arylamine. Unter einem Monostyrylamin wird eine Verbindung verstanden, die eine Styrylgruppe und mindestens ein Amin enthält, welches bevorzugt aromatisch ist. Unter einem Distyrylamin wird eine Verbindung verstanden, die zwei Styrylgruppen und mindestens ein Amin enthält, welches bevorzugt aromatisch ist. Unter einem Tristyrylamin wird eine Verbindung verstanden, die drei Styrylgruppen und mindestens ein Amin enthält, welches bevorzugt aromatisch ist. Unter einem Tetrastyrylamin wird eine Verbindung verstanden, die vier Styrylgruppen und mindestens ein Amin enthält, welches bevorzugt aromatisch ist. Unter einem Arylamin bzw. einem aromatischen Amin im Sinne dieser Erfindung wird eine Verbindung verstanden, die drei aromatische oder heteroaromatische Ringsysteme direkt an den Stickstoff gebunden enthält, davon bevorzugt mindestens ein kondensiertes Ringsystem mit mindestens 14 aromatischen Ringatomen. Die Styrylgruppen sind besonders bevorzugt Stilbene, die auch an der Doppelbindung oder an den Aromaten weiter substituiert sein können. Beispiele für derartige Dotanden sind substituierte oder unsubstituierte Tristilbenamine oder weitere Dotanden, die beispielsweise in WO 06/000388, WO 06/000389 und WO 06/058737 und in den nicht offen gelegten Patentanmeldungen DE 102005058543.4 und DE 102006015183.6 beschrieben sind. Weiterhin sind Verbindungen gemäß WO 06/122630 und gemäß der nicht offen gelegten Anmeldung DE 102006025846.0 als Dotanden bevorzugt. Ebenso kommen als Dotanden die hier beschriebenen erfindungsgemäßen Verbindungen in Frage, insbesondere die oben als Dotanden ausgeführten Verbindungen.

In nochmals einer weiteren bevorzugten Ausführungsform der Erfindung werden die Verbindungen gemäß Formel (1) bis (8) als Lochtransportmaterial bzw. als Lochinjektionsmaterial eingesetzt. Als Lochtransport- bzw. Lochinjektionsmaterial eignen sich besonders Verbindungen, in denen das Symbol X für O, S oder N(R¹) steht, insbesondere für O. Es kann bevorzugt sein, wenn die Verbindungen mit mindestens einer Gruppe N(Ar)₂ substituiert sind, bevorzugt mit mindestens zwei Gruppen N(Ar)₂ und/oder wenn die Gruppe Y eine Gruppe N(Ar) darstellt. Die Gruppen N(Ar)₂ sind bevorzugt ausgewählt aus den oben beschriebenen Formeln (24) oder (25). Aber auch, wenn keine Substituenten der Formel N(Ar)₂ vorhanden sind, sind die Verbindungen der Formel (1) bis (8) gute Lochtransport- bzw. Lochinjektionsmaterialien. Die Verbindung wird bevorzugt in einer Lochtransport- bzw. in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen einer Lochinjektionsschicht und einer Emissionsschicht liegt. Wenn die Verbindungen gemäß Formel (1) bis (8) als Lochtransport- bzw. als Lochinjektionsmaterial verwendet werden, kann es bevorzugt sein, wenn sie mit Elektronenakzeptor-Verbindungen dotiert werden, beispielsweise mit F₄-TCNQ oder mit Verbindungen, wie in EP 1476881 oder EP 1596445 beschrieben.

In nochmals einer weiteren Ausführungsform der Erfindung werden die Verbindungen gemäß Formel (1) bis (8) in einer Elektronentransportschicht und/oder in einer Lochblockierschicht eingesetzt. Dies gilt bevorzugt dann, wenn die Symbole X für C(R¹)₂ stehen, und insbesondere, wenn ein oder mehrere Substituenten R und/oder R¹ für eine Gruppe der Formel C(=O)Ar oder P(=O)Ar₂ stehen und/oder wenn die Gruppe Y für eine Gruppe der Formel C(=O) oder P(=O)Ar steht.

Verbindungen, wie sie oben bereits als Lochtransportmaterial beschrieben sind, können auch als n-Dotand, welcher mindestens ein Elektron an das Elektronentransportmaterial abgibt, in einer Elektronentransportschicht eingesetzt werden.

In nochmals einer weiteren Ausführungsform der Erfindung werden die Verbindungen gemäß Formel (1) bis (8) als Elektronentransportmaterial eingesetzt. Dies gilt bevorzugt dann, wenn die Symbole X für C(R¹)₂, BR¹, POR¹, SO oder SO₂ stehen und insbesondere, wenn ein oder mehrere Substituenten R und/oder R¹ mindestens eine Einheit C=O, P(=O) und/oder SO₂ enthalten oder wenn ein oder mehrere Substituenten R und/oder R¹ einen elektronenarmen Heterocyclus enthalten. Besonders geeignete elektronenarme Heterocyclen sind Imidazol, Pyrazol, Thiazol, Benzimidazol, Benzothiazol, Triazol, Oxadiazol, Benzothiadiazol, Phenanthrolin, etc. Besonders bevorzugt sind diese Gruppen direkt an die erfindungsgemäße zentrale Einheit gebunden. Weiterhin kann es bevorzugt sein, wenn die Verbindung mit Elektronendonorverbindungen dotiert ist.

Auch in Polymeren können Wiederholeinheiten gemäß Formel (1) bis (8) entweder als Polymergrundgerüst (Backbone), als emittierende Einheit oder als lochtransportierende Einheit eingesetzt werden. Dabei entsprechen die bevorzugten Substitutionsmuster den oben beschriebenen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig. Hohe Löslichkeit lässt sich durch geeignete Substitution der Verbindungen erreichen.

Die erfindungsgemäßen Verbindungen weisen bei Verwendung in organischen Elektrolumineszenzvorrichtungen folgende überraschende Vorteile gegenüber dem Stand der Technik auf:
1. Die erfindungsgemäßen Verbindungen sind in wenigen Schritten mit hoher Ausbeute synthetisch zugänglich. Dabei fallen die Verbindungen bereits als Rohprodukt in hoher Reinheit an. Da an organische Materialien für elektronische Anwendungen sehr hohe Reinheitsanforderungen gestellt werden, ist dies ein technischer Vorteil, da sich die nachfolgende Reinigung der Verbindungen erheblich vereinfacht.
2. Die erfindungsgemäßen Verbindungen weisen in organischen Elektrolumineszenzvorrichtungen sehr gute Effizienzen und Lebensdauern auf.
3. Die erfindungsgemäßen Verbindungen weisen eine hohe thermische Stabilität auf und lassen sich unzersetzt sublimieren. Dies vereinfacht ihre Reinigung durch Sublimation und die Vakuum-Herstellung der Elektrolumineszenzvorrichtung erheblich.
4. Die erfindungsgemäßen Verbindungen weisen eine geringere Sauerstoff- und Lichtempfindlichkeit auf als Verbindungen, die gemäß dem Stand der Technik als blaue und grüne Emitter verwendet werden, insbesondere als Bis(diarylamin)-Derivate kondensierter Aromaten, wie Anthracen, Pyren oder Chrysen.

Im vorliegenden Anmeldetext wird auf die Verwendung der erfindungsgemäßen Verbindungen in Bezug auf OLEDs und PLEDs und die entsprechenden Displays abgezielt. Trotz dieser Beschränkung der Beschreibung ist es für den Fachmann ohne weiteres erfinderisches Zutun möglich, die erfindungsgemäßen Verbindungen auch in anderen elektronischen Vorrichtungen einzusetzen, z. B. in organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Laserdioden (O-Laser) oder organischen Photorezeptoren.

Die Verwendung der erfindungsgemäßen Verbindungen in den entsprechenden Vorrichtungen ebenso wie diese Vorrichtungen selbst sind ebenfalls ein Gegenstand der vorliegenden Erfindung.

Die Erfindung wird durch die nachfolgenden Beispiele näher erläutert, ohne sie dadurch einschränken zu wollen.

### Beispiele

Die nachfolgenden Synthesen werden, sofern nicht anders angegeben, unter einer Schutzgasatmosphäre durchgeführt. Die Edukte können von den Firmen ALDRICH bzw. ABCR bezogen werden. Peri-Xanthenoxanthen und 6,6'-Dibrom-peri-xanthenoxanthen werden nach DE 545212 dargestellt.

### Beispiel 1: 5,5'-Dibrom-peri-xanthenoxanthen

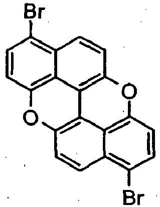

Eine Suspension von 28.2 g (100 mmol) peri-Xanthenoxanthen und 36.5 g (205 mmol) N-Bromsuccinimid in 200 ml o-Dichlorbenzol wird 6 h bei 180 °C gerührt. Nach Erkalten wird der Feststoff abgesaugt, fünfmal mit je 200 ml Ethanol gewaschen und im Vakuum getrocknet. Zweimalige Umkristallisation des Rohprodukts aus o-Dichlorbenzol ergibt 41.4 g (94 mmol) 5,5'-Dibrom-peri-xanthenoxanthen, entsprechend 94.0 % d. Th., mit einer Reinheit von 99.0 % nach HPLC.

### Beispiel 2: 5,5',7,7'-Tetra-brom-peri-xanthenoxanthen

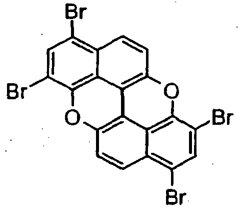

Eine Suspension von 28.2 g (100 mmol) peri-Xanthenoxanthen und 73.0 g (410 mmol) N-Bromsuccinimid in 300 ml o-Dichlorbenzol wird 16 h bei 180 °C gerührt. Nach Erkalten wird der Feststoff abgesaugt, fünfmal mit je 200 ml Ethanol gewaschen und im Vakuum getrocknet. Zweimalige Umkristallisation des Rohprodukts aus o-Dichlorbenzol ergibt 55.0 g (92 mmol) 5,5',7,7'-Tetrabrom-peri-xanthenoxanthen, entsprechend 92.0 % d. Th., mit einer Reinheit von 99.0 % nach HPLC.

### Beispiel 3: 3,3',5,5',7,7'-Hexabrom-peri-xanthenoxanthen

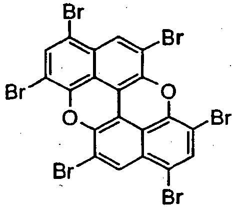

Eine Suspension von 28.2 g (100 mmol) peri-Xanthenoxanthen und 142.4 g (800 mmol) N-Bromsuccinimid in 500 ml o-Dichlorbenzol wird 28 h bei 180 °C gerührt. Nach Erkalten wird der Feststoff abgesaugt, fünfmal mit je 200 ml Ethanol gewaschen und im Vakuum getrocknet. Dreimaliges Auskochen des Rohprodukts aus o-Dichlorbenzol ergibt 72.5 g (96 mmol) 3,3',5,5',7,7'-Hexabrom-peri-xanthenoxanthen, entsprechend 96.0 % d. Th., mit einer Reinheit von 99.0 % nach HPLC.

### Beispiel 4: 6,6'-Bis(1-naphthyl)-peri-xanthenoxanthen

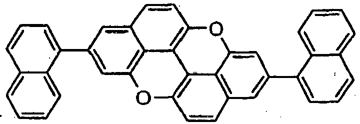

Eine Suspension von 22.0 g (50 mmol) 6,6'-Dibrom-peri-xantheno-xanthen, 22.4 g (130 mmol) Naphthylboronsäure und 44.6 g (210 mmol) Trikaliumphosphat in einem Gemisch aus 800 ml Toluol, 200 ml Dioxan und 800 ml Wasser wird mit 913 mg (3 mmol) Tri-o-tolylphosphin und dann mit 112 mg (0.5 mmol) Palladium(II)acetat versetzt und 18 h unter Rückfluss erhitzt. Nach Erkalten wird der Feststoff abgesaugt, dreimal mit je 300 ml Wasser, dreimal mit je 200 ml Ethanol und dreimal mit je 200 ml Toluol gewaschen und im Vakuum getrocknet. Viermalige Umkristallisation aus o-Dichlorbenzol und anschließende Sublimation bei T = 350 °C und p = 2 x 10⁻⁵ mbar ergibt 16.6 g (31 mmol) 6,6'-Bis(1-naphthyl)-peri-xanthenoxanthen, entsprechend 62.1 % d. Th., mit einer Reinheit von 99.9 % nach HPLC.

### Beispiele 5 bis 10:

Analog zu Beispiel 4 werden die in Tabelle 1 gezeigten Verbindungen durch Umsetzung der entsprechenden Brom-peri-xanthenoxanthene mit den entsprechenden Arylboronsäuren dargestellt, gereinigt und sublimiert.

In Beispiel 9 werden 25 mmol 5,5',7,7'-Teterabrom-peri-xanthenoxanthen eingesetzt. In Beispiel 10 werden 12.5 mmol 3,3',5,5',7,7'-Hexabrom-peri-xanthenoxanthen eingesetzt.

**Tabelle 1**

| **Bsp.** | **Produkt** | **Ausbeute** |
|---|---|---|
| 5 | 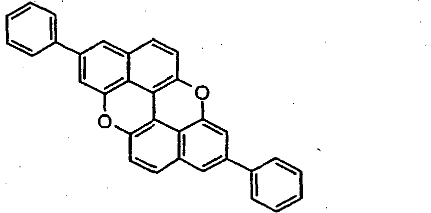 | 67.0 % |
| 6 | 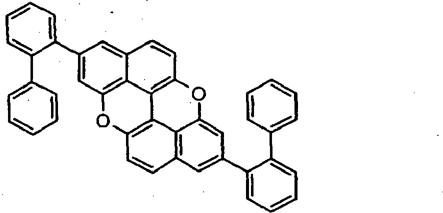 | 73.9 % |
| 7 | 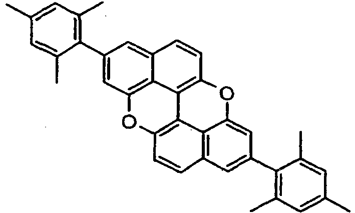 | 81.3 % |
| 8 | 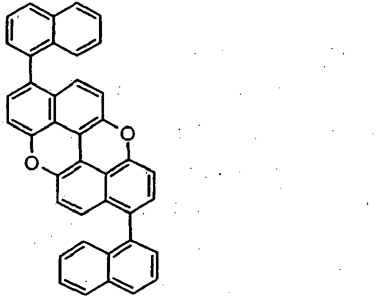 | 84.3 % |
| 9 | 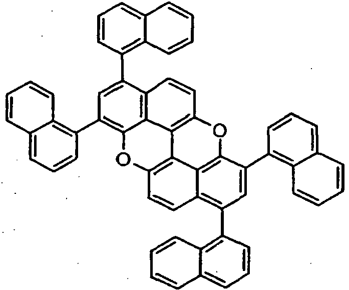 | 75.0 % |
| 10 | 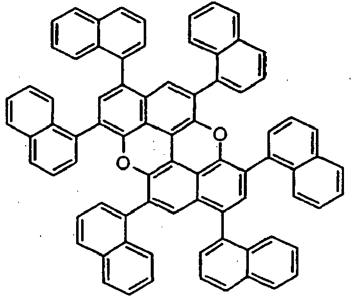 | 58.5 % |

### Beispiel 11: 6,6'-Bis(1-naphthyl)-peri-xanthenoxanthen

### a) 6-(1-Naphthyl)-2-naphthol

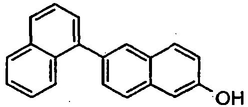

Eine Suspension von 111.5 g (500 mmol) 6-Brom-2-naphthol, 111.8 g (650 mmol) Naphthalinboronsäure und 224.1 g (1150 mmol) Trikaliumphosphat in einem Gemisch aus 1000 ml Toluol, 200 ml Dioxan und 1000 ml Wasser wird mit 913 mg (3 mmol) Tri-o-tolylphosphin und dann mit 112 mg (0.5 mmol) Palladium(II)acetat versetzt und 18 h unter Rückfluss erhitzt. Nach Erkalten wird mit 200 ml 2 N Salzsäure versetzt, die organische Phase wird abgetrennt, dreimal mit 500 ml 1 N Salzsäure gewaschen, einmal mit 500 ml gesättigter Kochsalzlösung gewaschen, über Kieselgel filtriert und im Vakuum komplett eingeengt. Der Rückstand wird aus 150 ml Toluol umkristallisiert. Ausbeute: 113.3 g (419 mmol), 83.8 % d. Th.; Reinheit: 98 % nach NMR.

### b) 6,6'-(1-Naphthyl)-1,1'-bi-2-naphthol

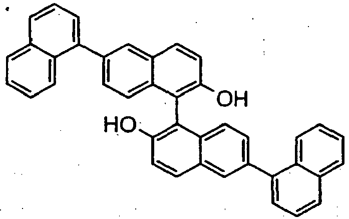

Eine Lösung von 58.9 g (218 mmol) 6-(1-Naphthyl)-2-naphthol in 600 ml Ethanol wird bei 70 °C unter Rühren mit einer Lösung von 53.0 g Eisen(III)chlorid, wasserfrei, gelöst in 300 ml Wasser, versetzt. Nach 8 h Rühren bei 70 °C wird der Feststoff abgesaugt und dreimal mit je 300 ml Wasser gewaschen. Der noch feuchte Feststoff wird in 1000 ml Toluol suspendiert. Unter gutem Rühren wird durch Abdestillieren von 800 ml Toluol azeotrop getrocknet. Nach Erkalten wird der Feststoff abgesaugt, mit Heptan gewaschen und im Vakuum getrocknet. Ausbeute: 90.4 g (168 mmol), 77.0 % d. Th.; Reinheit: 97 % nach NMR.

### c) 6,6'-Bis-(1-naphthyl)-peri-xanthenoxanthen

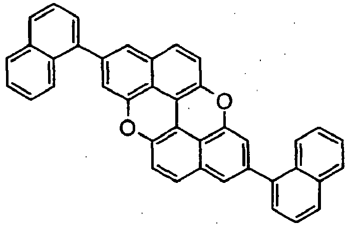

Eine Suspension von 32.3 g (60 mmol) 6,6'-(1-Naphthyl)-1,1'-bi-2-naphthol und 33.4 g (420 mmol) Kuper(II)oxid in 500 ml Nitrobenzol wird so lange am Wasserabscheider erhitzt, bis die Wasserabscheidung beendet ist (typischerweise 2-3 h). Dann wird das Nitrobenzol bis zur Trockene abdestilliert. Der Rückstand wird einer Soxhlett-Extraktion mit Chloroform unterzogen, bis das ablaufende Extrakt keine blaue Fluoreszenz mehr zeigt. Nach Erkalten der Suspension wird der Feststoff abgesaugt und mit etwas Chloroform gewaschen. Viermalige Umkristallisation des Rohprodukts aus o-Dichlorbenzol und anschließende Sublimation bei T = 350 °C und p = 2 x 10⁻⁵ mbar ergibt 24.7 g (46 mmol) 6,6'-Bis(1-naphthyl)-peri-xanthenoxanthen, entsprechend 77.0 % d. Th., mit einer Reinheit von 99.9 % nach HPLC.

### Beispiele 12 bis 14:

Analog zu Beispiel 11 werden die in Tabelle 2 gezeigten Verbindungen dargestellt, gereinigt und sublimiert. Die angegebenen Ausbeuten beziehen sich auf Schritt 11c). Die Ausbeuten in den Schritten a) und b) sind jeweils vergleichbar mit denen von Beispiel 11.

**Tabelle 2**

| **Bsp.** | **Produkt** | **Ausbeute** |
|---|---|---|
| 12 | 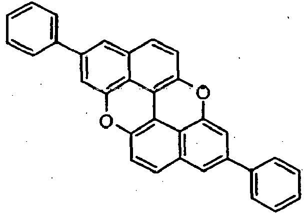 | 86.5 % |
| 13 | 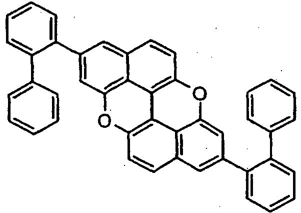 | 87.1 % |
| 14 | 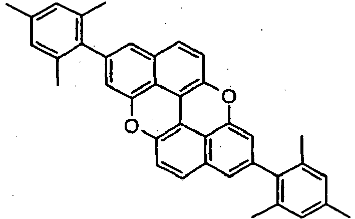 | 84.6 % |

### Beispiel 15: 5,5'-Bis(diphenylamino)-peri-xanthenoxanthen

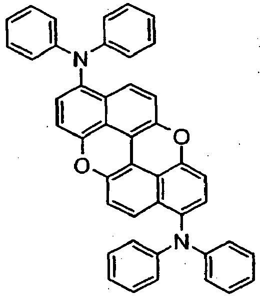

Eine Suspension von 22.0 g (50 mmol) 5,5'-Dibrom-peri-xanthenoxanthen und 20.3 g (120 mmol) Diphenylamin in 500 ml Toluol wird mit 14.4 g (150 mmol) Natrium-tert-butylat, dann mit 271 mg (1.5 mmol) Chlor-di-tert-butylphosphin und dann mit 225 mg (1 mmol) Palladium(II)acetat versetzt und 16 h unter Rückfluss erhitzt. Nach Erkalten gibt man 500 ml Wasser zu, saugt vom ausgefallenen Feststoff ab, wäscht diesen dreimal mit je 300 ml Wasser/Ethanol (1:1, v:v) und dreimal mit je 200 ml Ethanol. Fünfmalige Umkristallisation des Rohprodukts aus Chlorbenzol und anschließende Sublimation bei T = 365 °C und p = 2 x 10⁻⁵ mbar ergibt 17.9 g (29 mmol) 5,5'-Bis(diphenylamino)-peri-xanthenoxanthen, entsprechend 58.0 % d. Th., mit einer Reinheit von 99.9 % nach HPLC.

### Beispiel 16: 6,6,12,12-Tetramethyl-4,10-bis(naphth-1-yl)-2,8-bis(bis-3-methylphenyl)amino-6H,12H-dibenzo[def,mno]chrysen

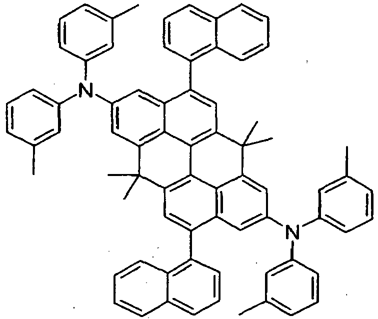

### a) 6,6,12,12-Tetramethyl-6H,12H-dibenzo[def,mno)chrysen

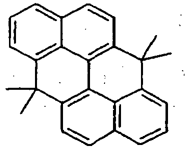

Eine auf -78 °C gekühlte Lösung von 37.0 g (100 mmol) 2,2'-Bismethoxycarbonyl-1,1'-binaphthyl in 1000 ml THF wird tropfenweise mit 375 ml (600 mmol) n-Methyllithium (1.6 molar in Diethylether) versetzt und anschließend 6 h nachgerührt. Man lässt die Mischung langsam auf Raumtemperatur erwärmen, gibt 200 ml gesättigte Ammoniumchloridlösung zu, rührt 15 min. gut nach, trennt die organische Phase ab und trocknet über Magnesiumsulfat. Nach Entfernen des THF wird der ölige Rückstand in 500 ml Eisessig aufgenommen, mit 5 ml konz. Schwefelsäure versetzt und 15 min unter Rückfluss erhitzt. Nach Erkalten werden die farblosen Kristalle abgesaugt, mit Eisessig (3 x 100 ml) und Ethanol (3 x 100 ml) gewaschen und im Vakuum getrocknet. Ausbeute 29.7 g, entsprechend 88.7 % d. Th.; Reinheit 97 %ig nach NMR.

### b) 4,10-Dibrom-6,6,12,12-tetramethyl-6H,12H-dibenzo[def,mno]-chrysen

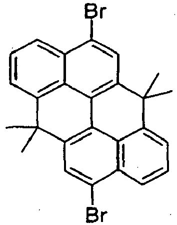

Eine auf 0 °C gekühlte Lösung von 26.8 g (80 mmol) 6,6,12,12-Tetra-methyl-6H,12H-dibenzo[def,mno]chrysen in 300 ml Dichlormethan wird unter Lichtausschluss tropfenweise mit einer Mischung von 8.2 ml (160 mmol) Brom in 200 ml Dichlormethan versetzt. Nach vollendeter Zugabe wird bis zur Entfärbung gerührt, dann wird mit 1000 ml Ethanol versetzt, 14 h bei Raumtemperatur nachgerührt, vom farblosen Feststoff abgesaugt und dieser anschließend mit Ethanol (3 x 100 ml) gewaschen und im Vakuum getrocknet. Ausbeute 37.3 g, entsprechend 94.7 % d. Th.; Reinheit 98 %ig nach NMR.

### c) 4,10-Bis(naphth-1-yl)-6,6,12,12-tetramethyl-6H,12H-dibenzo-[def,mno]-chrysen

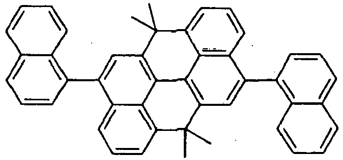

Durchführung analog Beispiel 4. Anstelle von 22.0 g (50 mmol) 6,6'-Dibrom-peri-xanthenoxanthen werden 24.6 g (50 mmol) 4,10-Dibrom-6,6,12,12-tetramethyl-6H,12H-dibenzo[def,mno]-chrysen eingesetzt. Ausbeute 25.0 g, entsprechend 85.2 % d. Th.; Reinheit 98 %ig nach NMR.

### d) 6,6,12,12-Tetramethyl-4,10-bis(naphth-1-yl)-2,8-dibrom-6H,12H-dibenzo[def,mno]chrysen

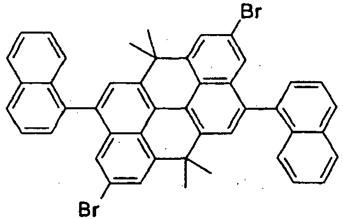

Durchführung analog Beispiel 16 b). Anstelle von 26.8 g (80 mmol) 6,6,12,12-Tetramethyl-6H,12H-dibenzo[def,mno]chrysen und 8.2 ml (160 mmol) Brom werden 20.5 g (35 mmol) 4,10-Bis(naphth-1-yl)-6,6,12,12-tetramethyl-6H,12H-dibenzo[def,mno]-chrysen und 3.6 ml (70 mmol) Brom eingesetzt. Zweimalige Umkristallisation des Rohproduktes aus DMF. Ausbeute 19.6 g, entsprechend 75.2 % d. Th.; Reinheit 99.5 %ig nach NMR.

### e) 6,6,12,12-Tetramethyl-4,10-bis(naphth-1-yl)-2,8-bis(bis-3-methylphenyl)amino-6H,12H-dibenzo[def,mno]chrysen

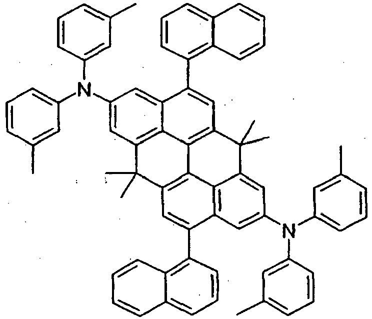

Durchführung analog Beispiel 15. Anstelle von 22.0 g (50 mmol) 5,5'-Dibrom-peri-xanthenoxanthen werden 14.9 g (20 mmol) 6,6,12,12-Tetra-methyl-4,10-bis(naphth-1-yl)-2,8-dibrom-6H,12H-dibenzo[def,mno]-chrysen eingesetzt. Die restlichen Reagenzien werden proportional angepasst. Dreimalige Umkristallisation des Rohprodukts aus Chlorbenzol und anschließende Sublimation bei T = 355 °C und p = 1 x 10⁻⁵ mbar ergibt 10.1 g, entsprechend 51.7 % d. Th., mit einer Reinheit von 99.9 % nach HPLC.

### Herstellung der OLEDs

Die Herstellung von OLEDs erfolgt nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wird.

In den folgenden Beispielen 17 bis 28 werden die Ergebnisse verschiedener OLEDs vorgestellt. Mit strukturiertem ITO (Indium-ZinnOxid) beschichtete Glasplatten bilden die Substrate der OLEDs. Zur verbesserten Prozessierung wird 20 nm PEDOT (aus Wasser aufgeschleudert; bezogen von H. C. Starck, Goslar, Deutschland; Poly(3,4-ethylendioxy-2,5-thiophen)) auf das Substrat aufgebracht. Die OLEDs bestehen aus folgender Schichtenfolge: Substrat / PEDOT / Lochinjektionsschicht (HIL1) 20 nm / Lochtransportschicht (HTM1) 60 nm / Emissionschicht (EML) 30 nm / Elektronentransportschicht (ETM1) 20 nm und abschließend eine Kathode. Die Materialien bis auf PEDOT werden in einer Vakuumkammer thermisch aufgedampft. Dabei besteht die Emissionsschicht immer aus einem Matrixmaterial (Host) und einem Dotierstoff (Dotand), der durch Coverdampfung dem Host beigemischt wird. Die Kathode wird durch eine 1 nm dünne LiF-Schicht und einer darauf abgeschiedenen 150 nm AI-Schicht gebildet. Die Tabelle 3 zeigt die Strukturen der zum Aufbau der OLEDs verwendeten Materialien.

Diese OLEDs werden standardmäßig charakterisiert; hierfür werden die Elektrolumineszenzspektren, die Effizienz (gemessen in cd/A), die Leistungseffizienz (gemessen in Im/W) in Abhängigkeit der Helligkeit, berechnet aus Strom-Spannungs-Helligkeit-Kennlinien (IUL-Kennlinien), und die Lebensdauer bestimmt. Als Lebensdauer wird die Zeit definiert, nach der die Anfangshelligkeit von 4000 cd/m² auf die Hälfte gesunken ist. In Tabelle 4 sind die Ergebnisse einiger OLEDs (Beispiele 17 bis 28) zusammengefasst. Als Vergleichsbeispiel werden der Dotand D1 und der Host H1 gemäß dem Stand der Technik verwendet.

**Tabelle 3**

| | | |
|---|---|---|
| 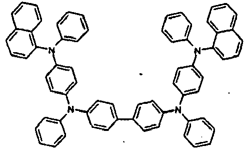 | 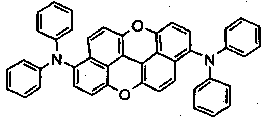 | 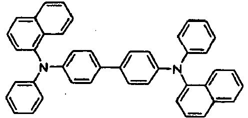 |
| HIL1 | HIL1 | HTM1 |
| 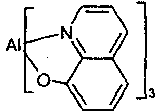 | | |
| ETM1 | | |
| 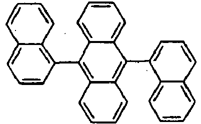 | 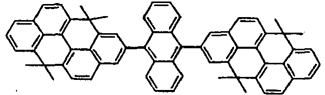 | 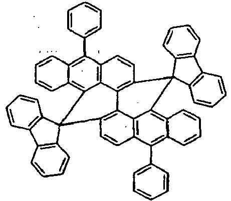 |
| H1 | H2 | H3 |
| 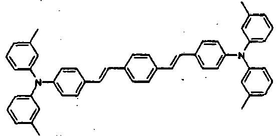 | 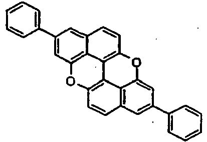 | 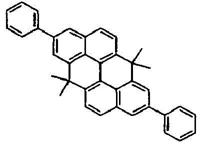 |
| D1 | D2, HIL3 | D3 |
| 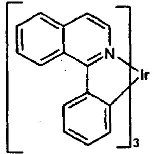 | 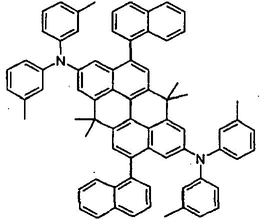 | 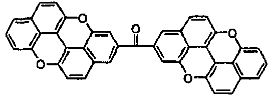 |
| D4 | D5 | TMM1 |

**Tabelle 4**

| **Beispiel** | **EML** | **HIL** | **Max Effizienz (cd/A)** | **Spannung(V) bei 1000 cd/m²** | **CIE** | **Lebensdauer (h) bei 4000 cd/m²** |
|---|---|---|---|---|---|---|
| 17 (Vergleich) | H1 + 5%D1 | HIL1 | 9.8 | 5.8 | x=0.17/ y=0.33 | 350 |
| 18 | H1 + 5% D1 | HIL3 | 10.1 | 5.9 | x=0.17/ y=0.33 | 500 |
| 19 | H1 + 5% D2 | HIL3 | 9.5 | 5.7 | x=0.16/ y=0.28 | 550 |
| 20 | H1 + 5% D3 | HIL1 | 4.3 | 5.9 | x=0.16/ y=0.09 | 380 |
| 21 | H2 + 5% D1 | HIL1 | 10.3 | 5.7 | x=0.17/ y=0.33 | 620 |
| 22 | H2 + 3% D1 | HIL1 | 9.6 | 5.7 | x=0.17/ y=0.30 | 590 |
| 23 | H1 + 5% D1 | HIL2 | 11.8 | 5.9 | x=0.17/ y=0.32 | 650 |
| 24 | H3 + 5% D1 | HIL1 | 9.5 | 5.8 | x=0.16/ y=0.28 | 480 |
| 25 | H3 + 7% D1 | HIL1 | 9.7 | 5.7 | x=0.16/ y=0.29 | 510 |
| 26 | TMM1 + 15% D4 | HIL1 | 12 | 5.3 | x=0.68/ y=0.32 | 950 |
| 27 | TMM1 + 10% D4 | HIL1 | 13 | 5.2 | x=0.68/ y=0.32 | 1100 |
| 28 | H1 + 5% D5 | HIL1 | 5.5 | 5.6 | x=0.15/ y=0.16 | 370 |

## Patentansprüche

1. Verbindungen gemäß den Formeln (1) bis (8), 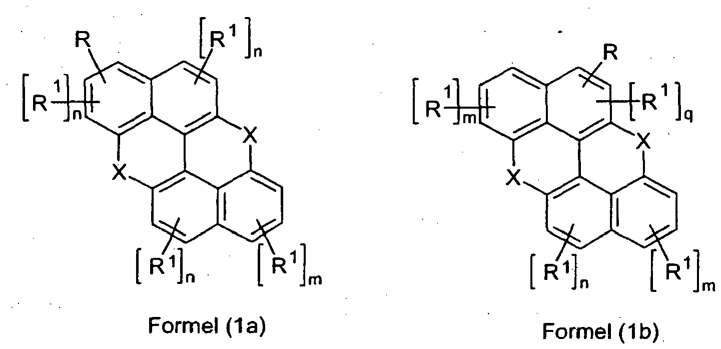 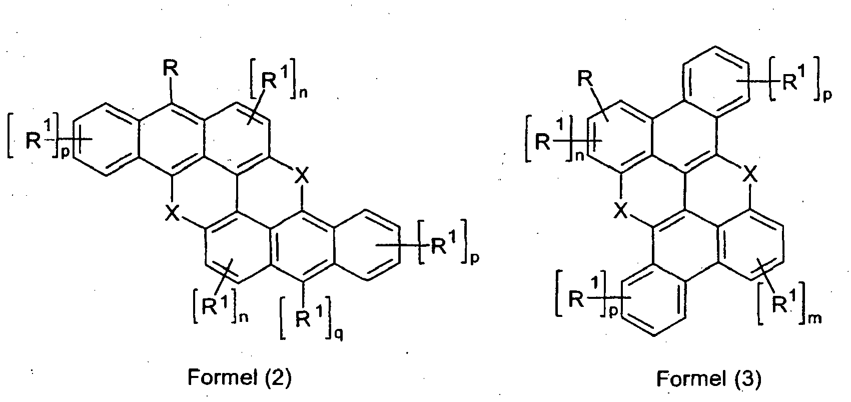 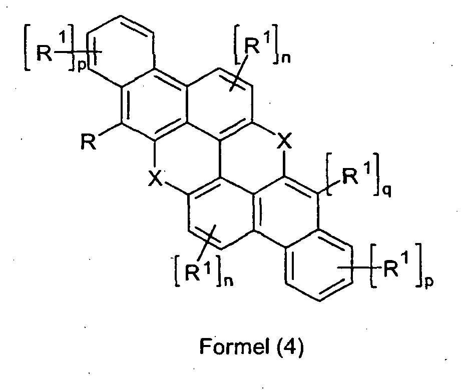 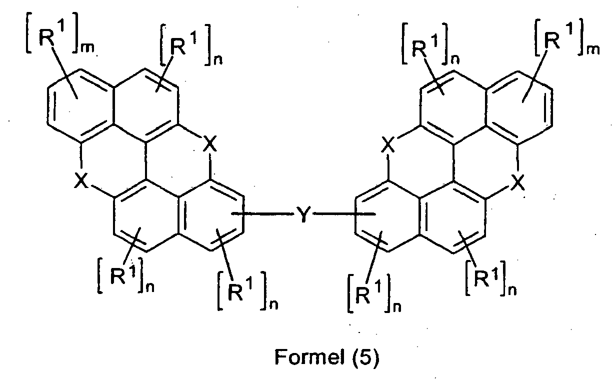 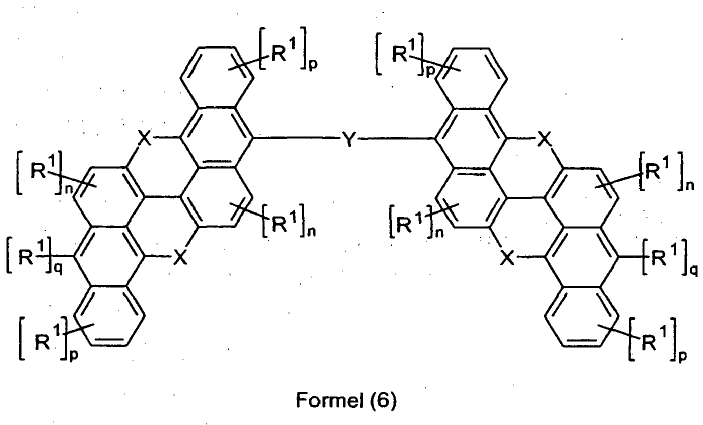 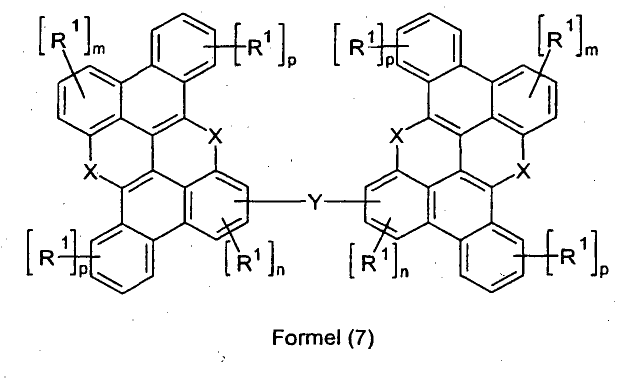 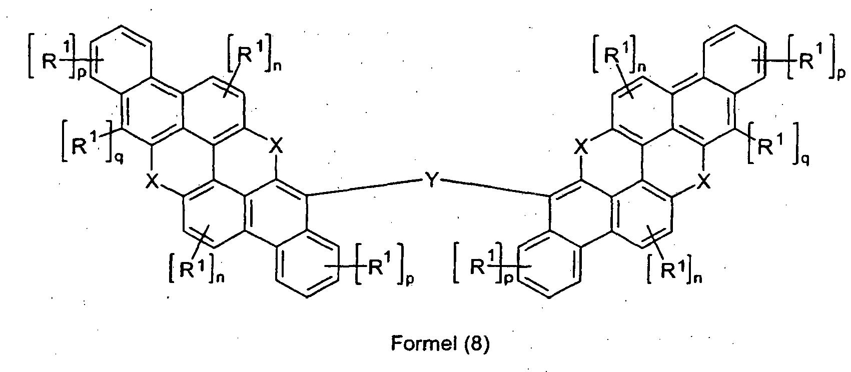 wobei für die Symbole und Indizes gilt:
X ist bei jedem Auftreten gleich oder verschieden O, S, NR¹, C(R¹)₂, BR¹, PR¹, POR¹, SO oder SO₂;
Y ist eine Einfachbindung, eine Gruppe C(=O), P(=O)Ar, N(Ar), S(=O), S(=O)₂, O, S, eine Alkylen- oder Alkylidengruppe mit 1 bis 20 C-Atomen oder ein bivalentes aromatisches Ringsystem mit 5 bis 40 aromatischen Ringatomen, welches mit einem oder mehreren nicht-aromatischen Resten R¹ substituiert sein kann;
R ist bei jedem Auftreten gleich oder verschieden eine Gruppe N(Ar)₂, C(=O)Ar, P(=O)Ar₂, S(=O)Ar, S(=O)₂Ar, CR²=CR²Ar, OAr, SAr, Si(R¹)₃, eine geradkettige Alkylgruppe mit 1 bis 40 C-Atomen, eine verzweigte oder cyclische Alkylgruppe mit 3 bis 40 C-Atomen, die jeweils durch einen oder mehrere Reste R² substituiert sein kann und wobei ein oder mehrere H-Atome durch F, Cl, Br, I, CN oder NO₂ ersetzt sein können, eine geradkettige Alkoxygruppe mit 2 bis 40 C-Atomen oder eine verzweigte oder cyclische Alkoxygruppe mit 3 bis 40 C-Atomen, wobei die Alkoxygruppe jeweils durch einen oder mehrere Reste R² substituiert sein kann, wobei eine oder mehrere nicht benachbarte CH₂-Gruppen der Alkoxygruppen durch R²C=CR², C=C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere nicht-aromatische Reste R¹ substituiert sein kann; dabei kann R auch mit benachbarten Substituenten R¹ ein mono- oder polycyclisches Ringsystem bilden;
R¹ ist bei jedem Auftreten gleich oder verschieden H, F, Cl, Br, I, CHO, N(Ar)₂, C(=O)Ar, P(=O)Ar₂, S(=O)Ar, S(=O)₂Ar, CR²=CR²Ar, OAr, SAr CN, NO₂, Si(R²)₃, B(OR²)₂, OSO₂R², eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C , Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere nicht-aromatische Reste R¹ substituiert sein kann, oder eine Kombination dieser Systeme; dabei können zwei oder mehrere benachbarte Substituenten R¹ auch miteinander oder R¹ mit R ein mono- oder polycyclisches Ringsystem bilden;
Ar ist bei jedem Auftreten gleich oder verschieden ein aromatisches oder heteroaromatisches Ringsystem mit 5 bis 40 aromatischen Ringatomen, das mit einem oder mehreren nicht-aromatischen Resten R¹ substituiert sein kann; dabei können auch zwei Reste Ar, welche an dasselbe Stickstoff- oder Phosphoratom binden, durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verknüpft sein;
R² ist bei jedem Auftreten gleich oder verschieden H oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten R² auch miteinander ein mono- oder polycyclisches aliphatisches oder aromatisches Ringsystem bilden;
n list bei jedem Auftreten gleich oder verschieden 0, 1 oder 2;
m ist bei jedem Auftreten gleich oder verschieden 0, 1, 2 oder 3;
p ist bei jedem Auftreten gleich oder verschieden 0, 1, 2, 3 oder 4;
q ist bei jedem Auftreten gleich oder verschieden 0 oder 1;
dabei sind die folgenden Verbindungen von der Erfindung ausgenommen:

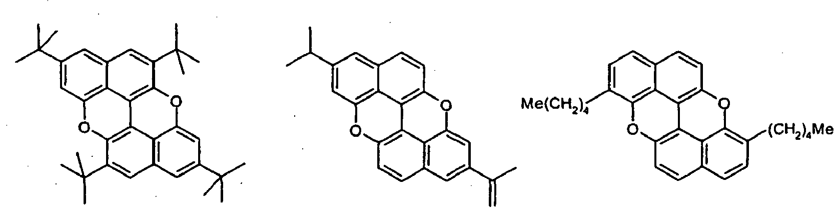

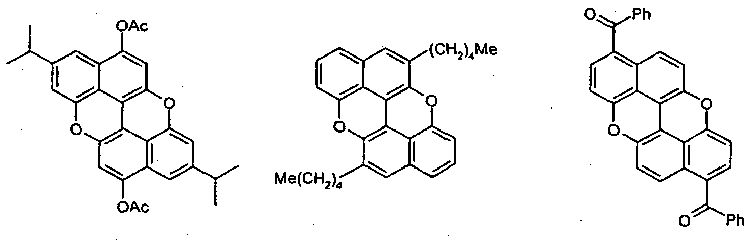

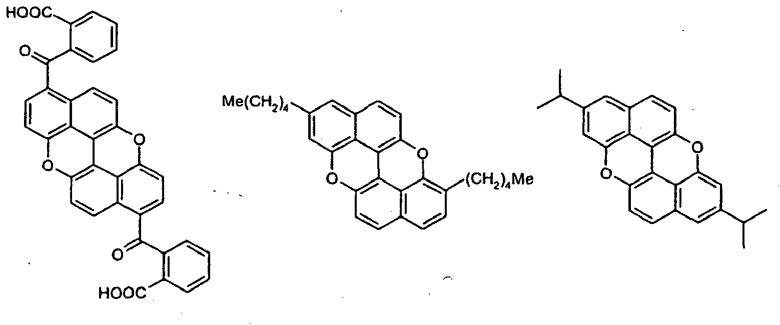

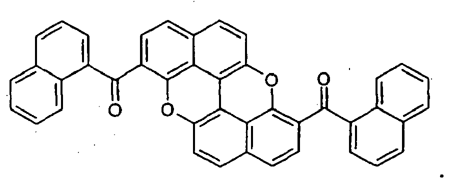

2. Verbindungen nach Anspruch 1, gewählt aus den Strukturen der Formeln (9) bis (24), 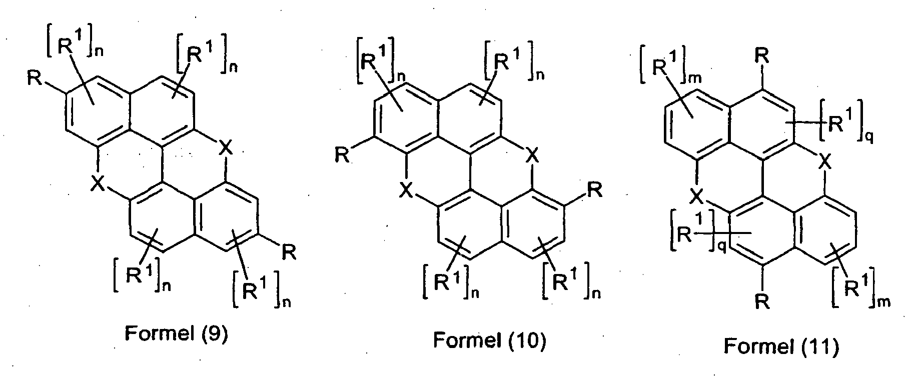 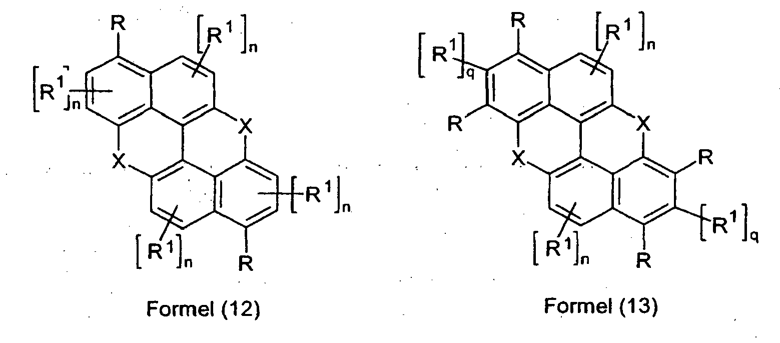 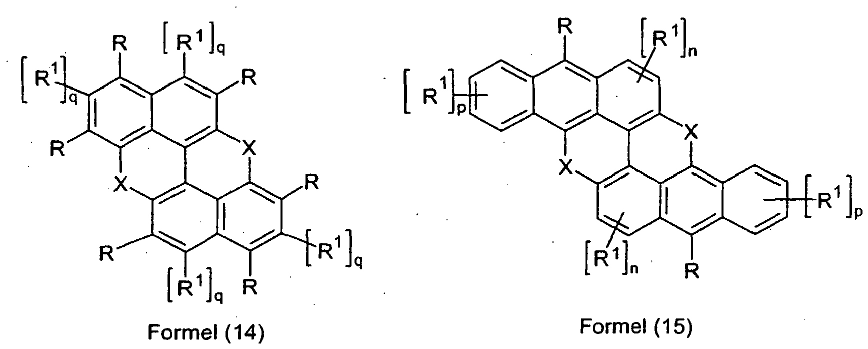 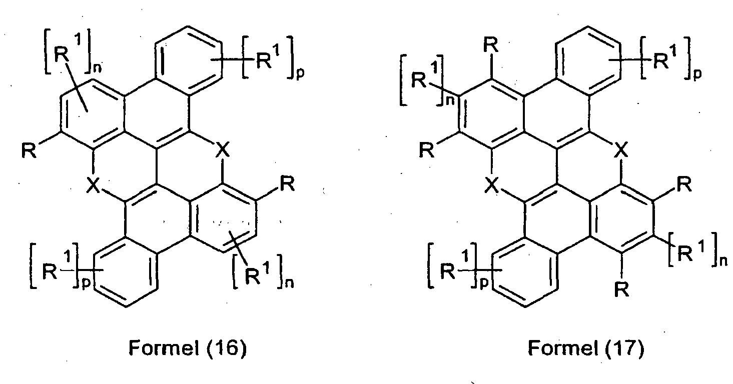 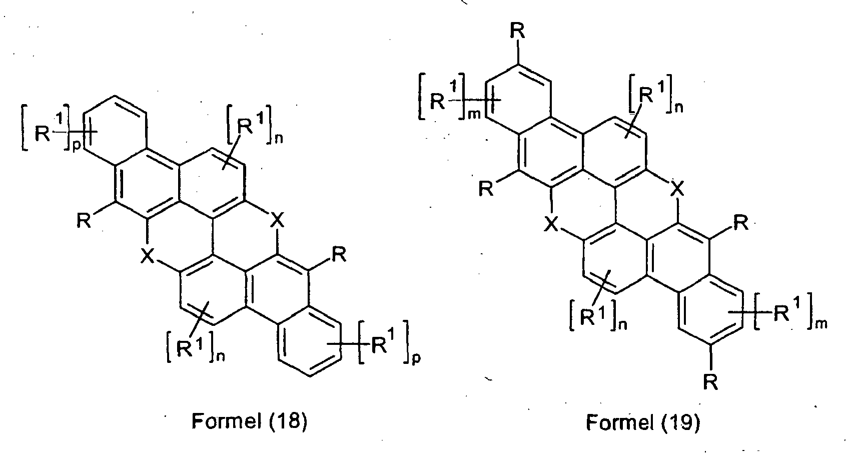 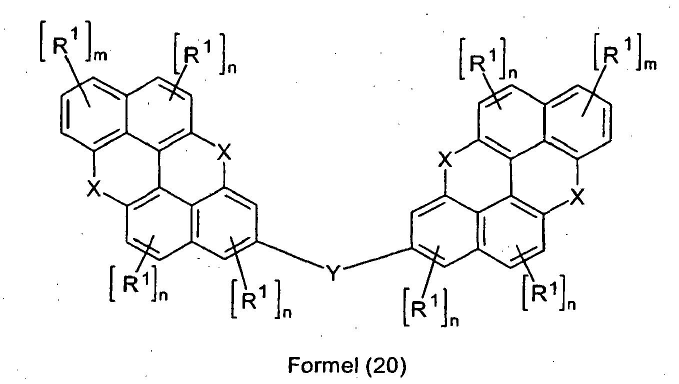 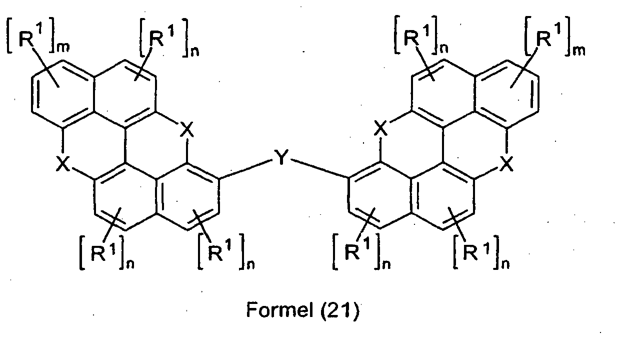 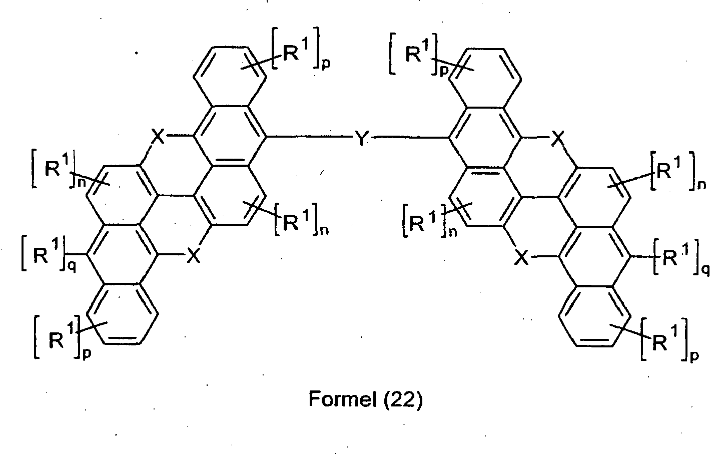 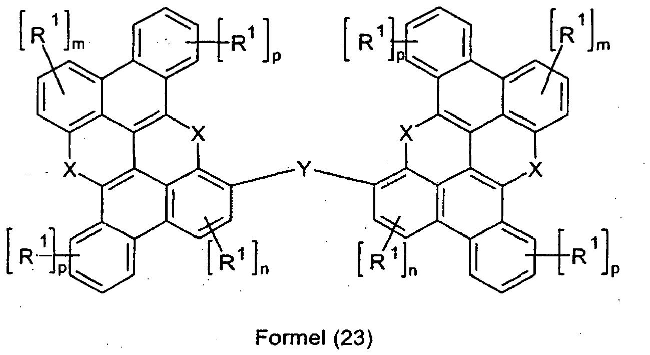 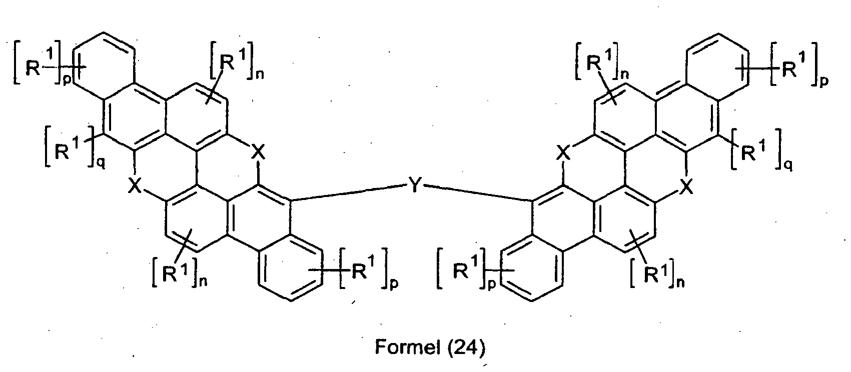 wobei R, R¹, R², Ar, X, Y, m, n, p und q dieselbe Bedeutung haben, wie in Anspruch 1 beschrieben.

3. Verbindungen nach Anspruch 1 oder 2, gewählt aus den Strukturen der Formeln (9a) bis (24a), 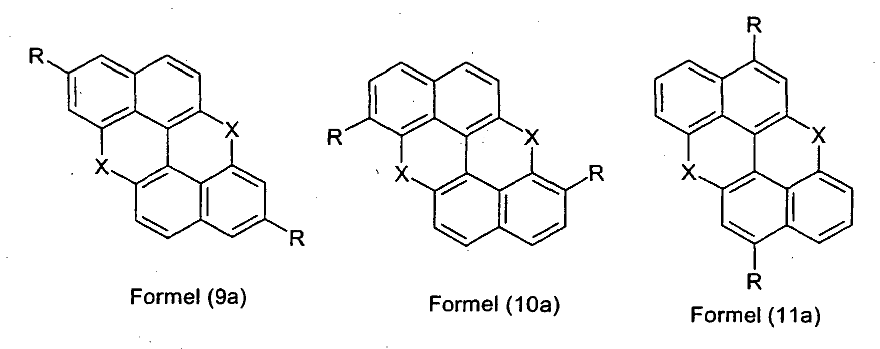 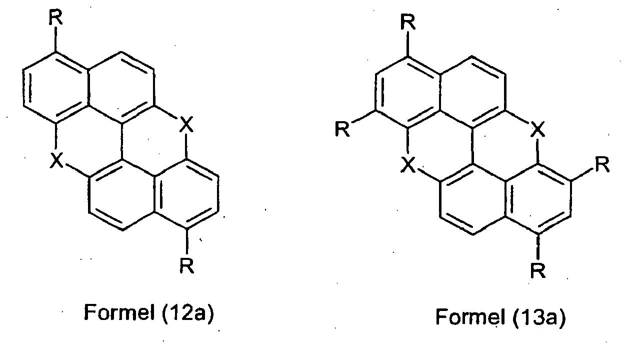 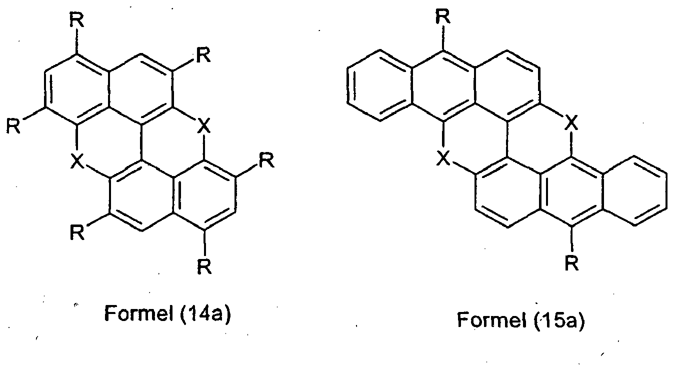 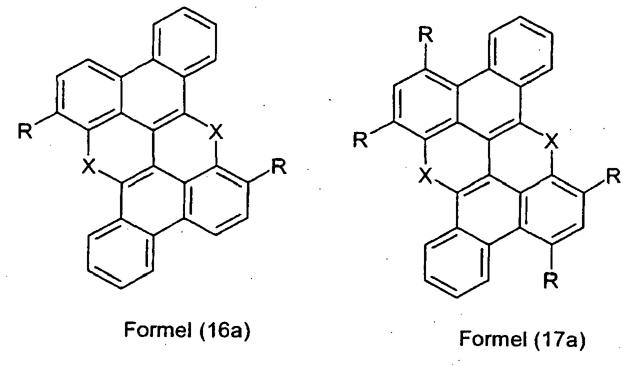 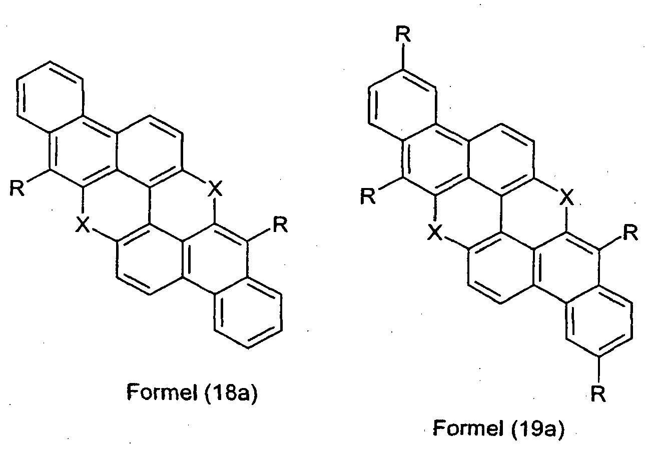 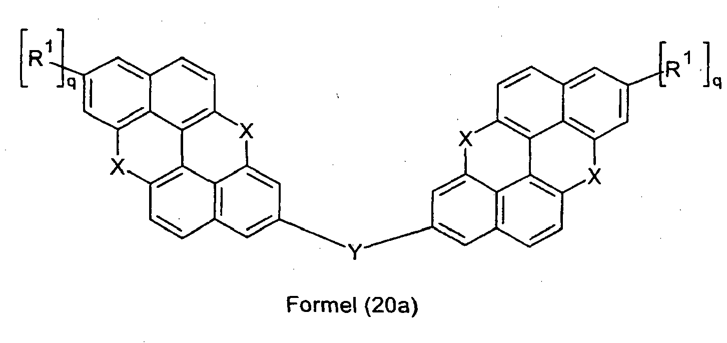 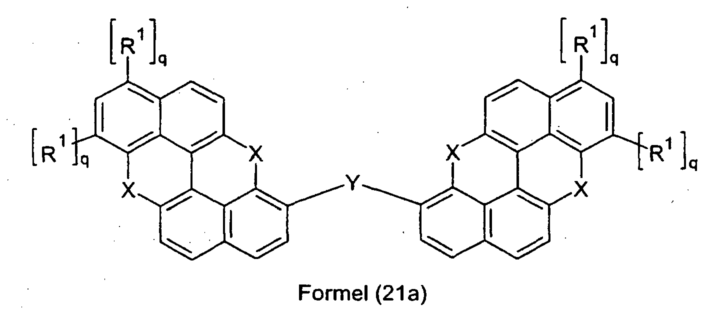 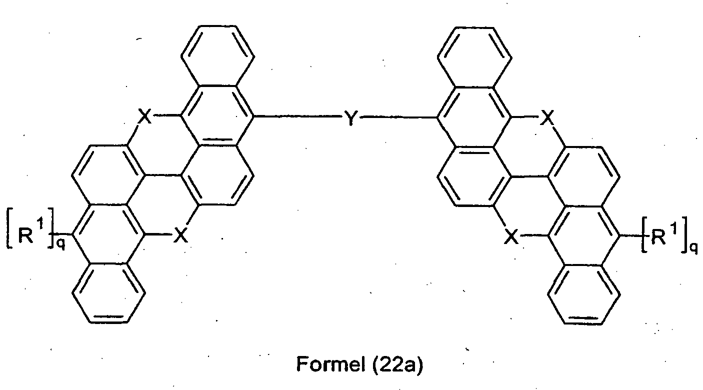 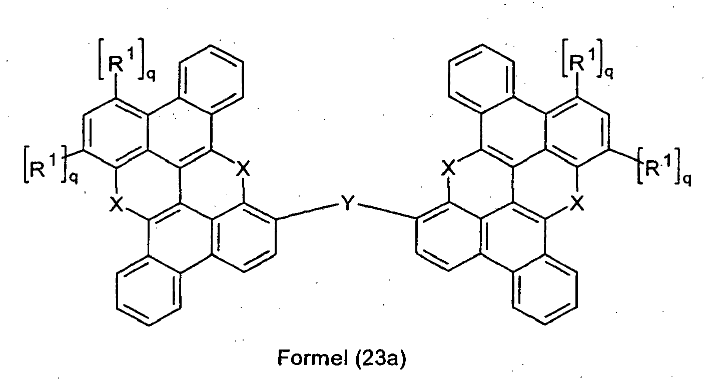 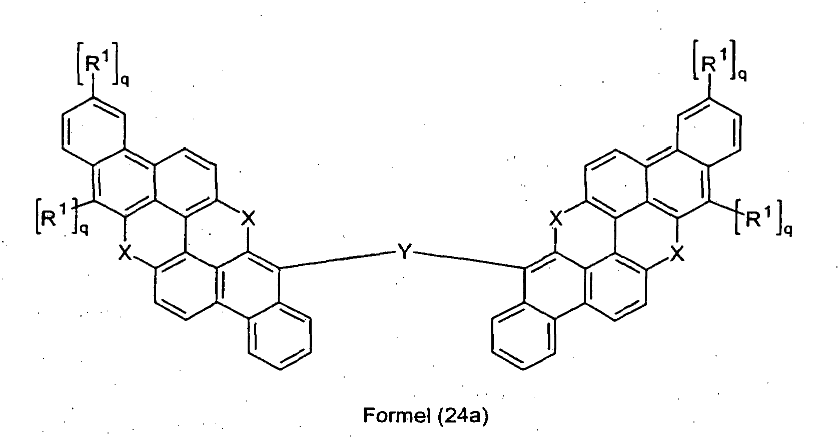 wobei R, R¹, R², Ar, X, Y und q dieselbe Bedeutung haben, wie in Anspruch 1 beschrieben.

4. Verbindungen nach einem oder mehreren der Ansprüche 1 bis 3, **dadurch gekennzeichnet, dass** das Symbol R, gleich oder verschieden bei jedem Auftreten, für eine Gruppe N(Ar)₂, C(=O)Ar, P(=O)Ar₂, eine geradkettige Alkylgruppe mit 1 bis 4 C-Atomen oder eine verzweigte Alkylgruppe mit 3 bis 5 C-Atomen oder ein aromatisches oder heteroaromatisches Ringsystem mit 5 bis 25 aromatischen Ringatomen, welches durch einen oder mehrere nicht-aromatische Reste R¹ substutiert sein kann, steht und dass das Symbol Y für eine Gruppe C(=O), N(Ar) oder für eine bivalente Arylgruppe mit 6 bis 16 C-Atomen, welche mit einem oder mehreren nicht-aromatischen Resten R¹ substituiert sein kann, steht.

5. Verbindungen nach einem oder mehreren der Ansprüche 1 bis 4, **dadurch gekennzeichnet, dass** die Verbindungen symmetrisch bezüglich den vorhandenen Substituenten aufgebaut sind.

6. Polymere, Oligomere oder Dendrimere enthaltend eine oder mehrere Verbindungen nach einem oder mehreren der Ansprüche 1 bis 5, wobei ein oder mehrere Reste R, R¹ und/oder R² Bindungen zum Polymer, Oligomer oder Dendrimer darstellen.

7. Verfahren zur Herstellung von Verbindungen nach einem oder mehreren der Ansprüche 1 bis 5, in denen X = O gilt, **gekennzeichnet durch** oxidative Cyclisierung des zuvor entsprechend funktionalisierten Binaphthol- bzw. Bianthrol- bzw. Biphenanthrolderivats.

8. Verfahren zur Herstellung von Verbindungen nach einem oder mehreren der Ansprüche 1 bis 5, in denen X = C(R¹)₂ gilt, **gekennzeichnet durch** säurekatalysierte dehydratisierende Cyclisierung des gegebenenfalls zuvor entsprechend funktionalisierten Binaphthyl- bzw. Bianthryl- bzw. Biphenanthrylderivats, welches jeweils mit zwei Gruppen der Formel -C(R¹)₂(OH) in den ortho-Positionen zur Verknüpfung des Binapthyls bzw. Bianthryls bzw. Biphenanthryls substituiert ist

9. Verwendung von Verbindungen nach einem oder mehreren der Ansprüche 1 bis 6 in elektronischen Vorrichtungen, insbesondere in organischen Elektrolumineszenzvorrichtungen.

10. Elektronische Vorrichtungen, enthaltend mindestens eine Verbindung nach einem oder mehreren der Ansprüche 1 bis 6.

11. Organische Elektrolumineszenzvorrichtung, enthaltend Anode, Kathode, mindestens eine emittierende Schicht und gegebenenfalls weitere Schichten, gewählt aus Lochinjektionsschicht, Lochtransportschicht, Elektronentransportschicht, Elektroneninjektionsschicht und/oder Charge-Generation Layer, **dadurch gekennzeichnet, dass** mindestens eine organische Schicht mindestens eine Verbindung nach einem oder mehreren der Ansprüche 1 bis 6 enthält.

12. Organische Elektrolumineszenzvorrichtung nach Anspruch 11, **dadurch gekennzeichnet, dass** das Symbol X in Verbindungen nach einem oder mehreren der Ansprüche 1 bis 7 für O, S oder N(R¹) steht und dass die Verbindung als emittierende Verbindung, gegebenenfalls in Mischung mit einem Hostmaterial, eingesetzt wird.

13. Organische Elektrolumineszenzvorrichtung nach Anspruch 11 oder 12, **dadurch gekennzeichnet, dass** das Symbol X in Verbindungen nach einem oder mehreren der Ansprüche 1 bis 6 für C(R¹)₂ steht und dass die Verbindung als Hostmaterial für fluoreszierende oder phosphoreszierende Dotanden eingesetzt wird.

14. Organische Elektrolumineszenzvorrichtung nach einem oder mehreren der Ansprüche 11 bis 13, **dadurch gekennzeichnet, dass** das Symbol X in Verbindungen nach einem oder mehreren der Ansprüche 1 bis 7 für O, S oder N(R¹) steht und/oder dass mindestens eine Gruppe R und/oder R¹ für eine Gruppe N(Ar)₂ steht und/oder dass die Gruppe Y für eine Gruppe N(Ar) steht, dass die Verbindung gegebenenfalls dotiert sein kann und dass die Verbindung als Lochtransportmaterial bzw. als Lochinjektionsmaterial eingesetzt wird, insbesondere in einer Lochtransport- bzw. in einer Lochinjektionsschicht.

15. Organische Elektrolumineszenzvorrichtung nach einem oder mehreren der Ansprüche 11 bis 14, **dadurch gekennzeichnet, dass** das Symbol X für C(R¹)₂, BR¹, POR¹, SO oder SO₂ steht und dass gegebenenfalls einer oder mehrere der Substituenten R und/oder R¹ für eine Gruppe C(=O)Ar oder P(=O)Ar₂ oder für einen elektronenarmen Heterocyclus stehen und/oder die Gruppe Y für eine Gruppe C(=O) oder P(=O)Ar steht, dass die Verbindung gegebenenfalls dotiert sein kann und dass die Verbindung in einer Elektronentransportschicht und/oder einer Lochblockierschicht eingesetzt wird.

## Claims

1. Compounds of the formulae (1) to (8), 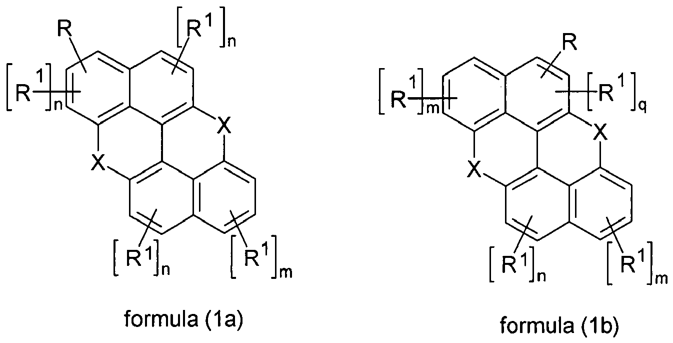 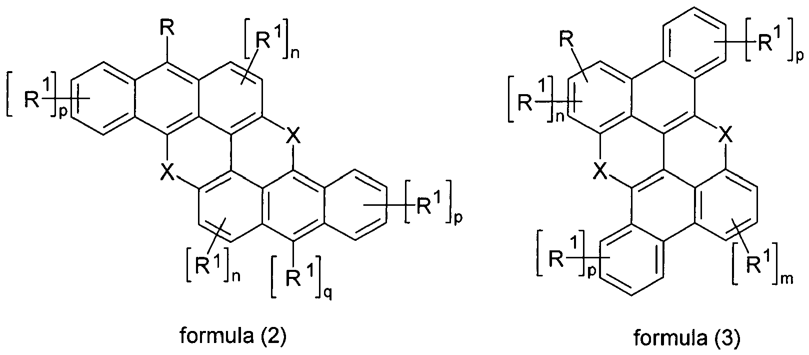 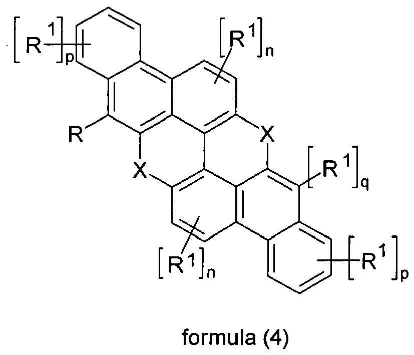 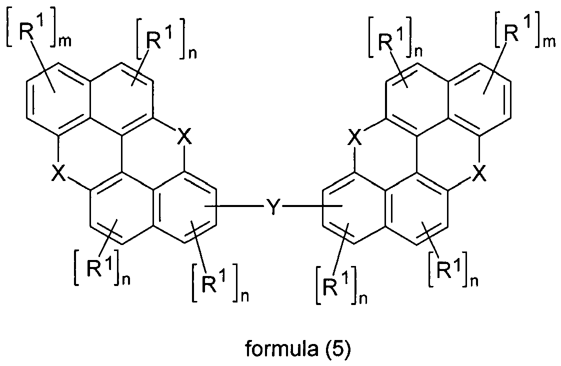 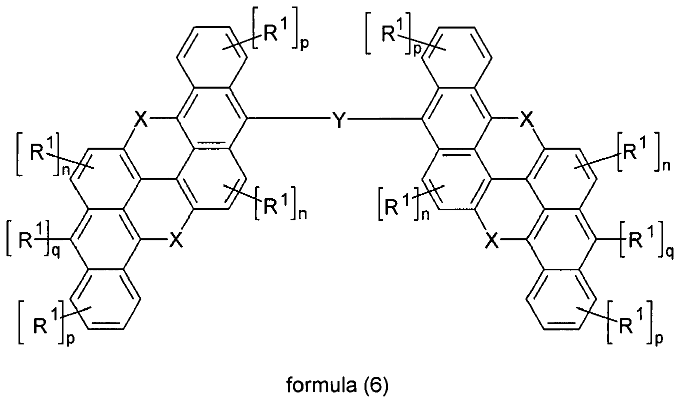 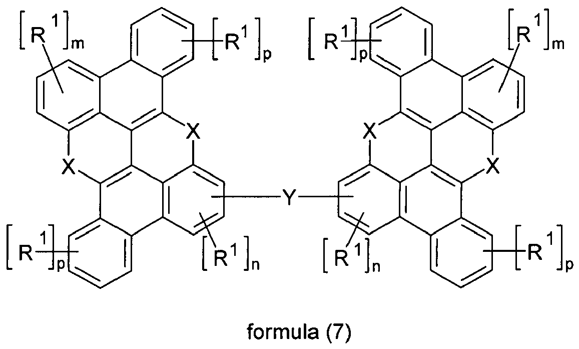 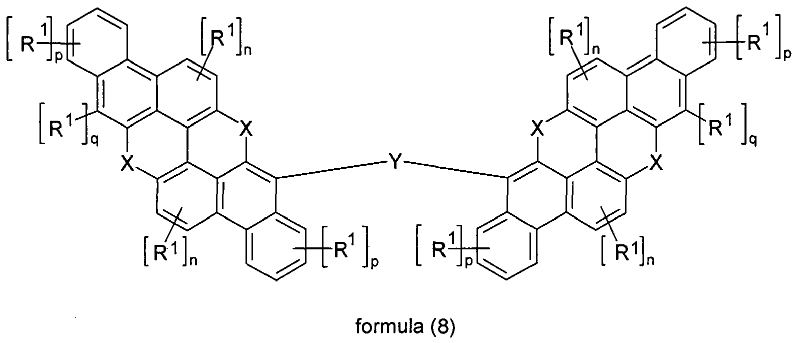 where the following applies to the symbols and indices:
X is on each occurrence, identically or differently, O, S, NR¹, C(R¹)₂, BR¹, PR¹, POR¹, SO or SO₂;
Y is a single bond, a C(=O), P(=O)Ar, N(Ar), S(=O), S(=O)₂, O, S group, an alkylene or alkylidene group having 1 to 20 C atoms or a divalent aromatic ring system having 5 to 40 aromatic ring atoms, which may be substituted by one or more non-aromatic radicals R¹;
R is on each occurrence, identically or differently, an N(Ar)₂, C(=O)Ar, P(=O)Ar₂, S(=O)Ar, S(=O)₂Ar, CR²=CR²Ar, OAr, SAr, Si(R¹)₃ group, a straight-chain alkyl group having 1 to 40 C atoms, a branched or cyclic alkyl group having 3 to 40 C atoms, each of which may be substituted by one or more radicals R² and in which one or more H atoms may be replaced by F, Cl, Br, I, CN or NO₂, a straight-chain alkoxy group having 2 to 40 C atoms or a branched or cyclic alkoxy group having 3 to 40 C atoms, where the alkoxy group may in each case be substituted by one or more radicals R², where one or more non-adjacent CH₂ groups in the alkoxy groups may be replaced by R²C=CR², C=C, Si(R²)₂, Ge(R²)₂, Sn(R²)₂, C=O, C=S, C=Se, C=NR², P(=O)(R²), SO, SO₂, NR², O, S or CONR² and where one or more H atoms may be replaced by F, Cl, Br, I, CN or NO₂, or an aromatic or heteroaromatic ring system having 5 to 60 aromatic ring atoms, each of which may be substituted by one or more non-aromatic radicals R¹; R here may also form a mono- or polycyclic ring system with adjacent substituents R¹;
R¹ is on each occurrence, identically or differently, H, F, Cl, Br, I, CHO, N(Ar)₂, C(=O)Ar, P(=O)Ar₂, S(=O)Ar, S(=O)₂Ar, CR²=CR²Ar, OAr, SAr, CN, NO₂, Si(R²)₃, B(OR²)₂, OSO₂R², a straight-chain alkyl, alkoxy or thioalkoxy group having 1 to 40 C atoms or a branched or cyclic alkyl, alkoxy or thioalkoxy group having 3 to 40 C atoms, each of which may be substituted by one or more radicals R², where one or more non-adjacent CH₂ groups may be replaced by R²C=CR², C=C, Si(R²)₂, Ge(R²)₂, Sn(R²)₂, C=O, C=S, C=Se, C=NR², P(=O)(R²), SO, SO₂, NR², O, S or CONR² and where one or more H atoms may be replaced by F, Cl, Br, I, CN or NO₂, or an aromatic or heteroaromatic ring system having 5 to 60 aromatic ring atoms, each of which may be substituted by one or more non-aromatic radicals R¹, or a combination of these systems; two or more adjacent substituents R¹ here may also form a mono- or polycyclic ring system with one another or R¹ with R;
Ar is on each occurrence, identically or differently, an aromatic or heteroaromatic ring system having 5 to 40 aromatic ring atoms, which may be substituted by one or more non-aromatic radicals R¹; two radicals Ar which are bonded to the same nitrogen or phosphorus atom may also be linked to one another here by a single bond or a bridge selected from B(R²), C(R²)₂, Si(R²)₂, C=O, C=NR², C=C(R²)₂, O, S, S=O, SO₂, N(R²), P(R²) and P(=O)R²_{;}
R² is on each occurrence, identically or differently, H or an aliphatic, aromatic and/or heteroaromatic hydrocarbon radical having 1 to 20 C atoms, in which, in addition, H atoms may be replaced by F; two or more adjacent substituents R² here may also form a mono- or polycyclic aliphatic or aromatic ring system with one another;
n is on each occurrence, identically or differently, 0, 1 or 2;
m is on each occurrence, identically or differently, 0, 1, 2 or 3;
p is on each occurrence, identically or differently, 0, 1, 2, 3 or 4;
q is on each occurrence, identically or differently, 0 or 1;
the following compounds are excluded from the invention:

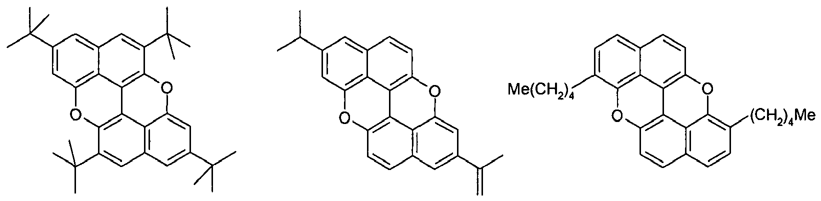

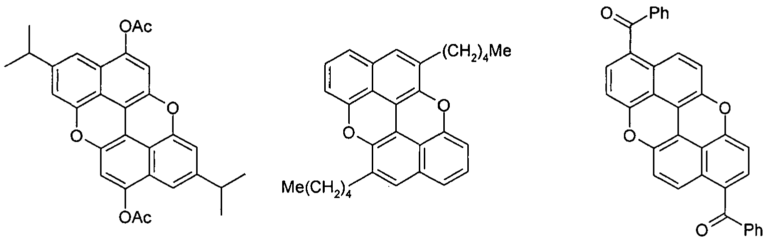

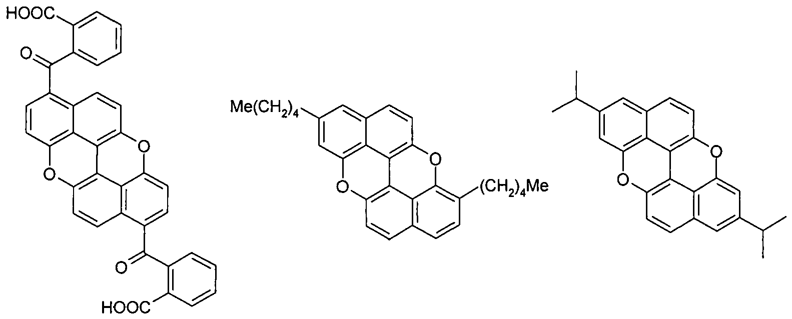

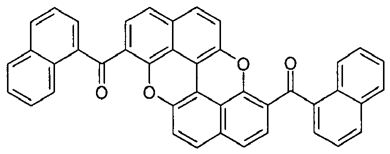

2. Compounds according to Claim 1, selected from the structures of the formulae (9) to (24), 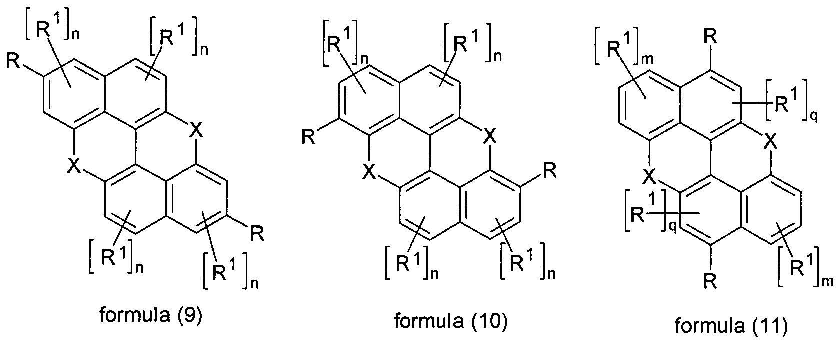 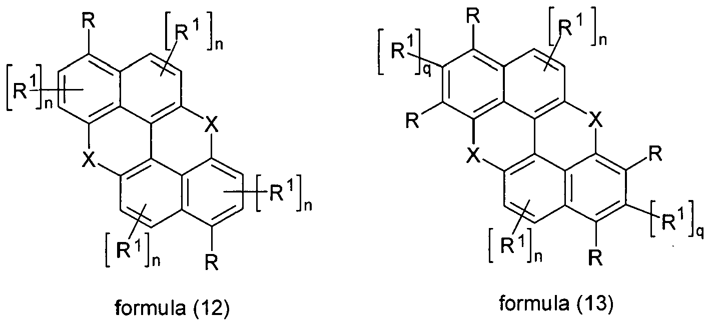 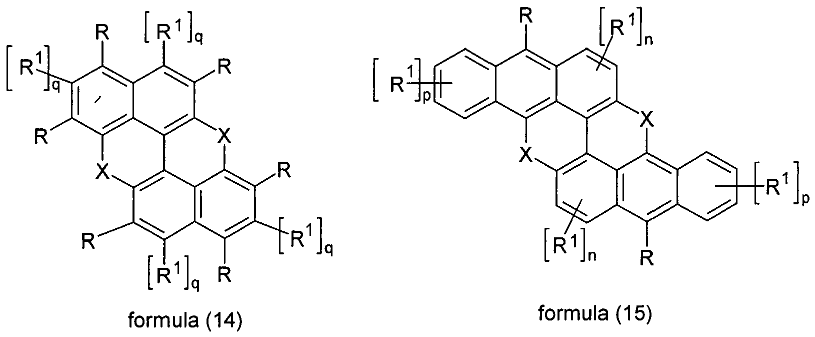 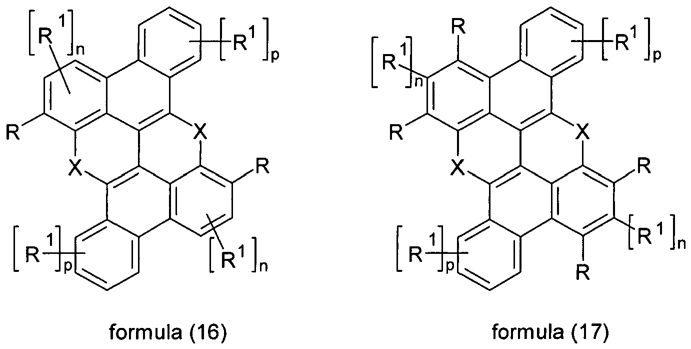 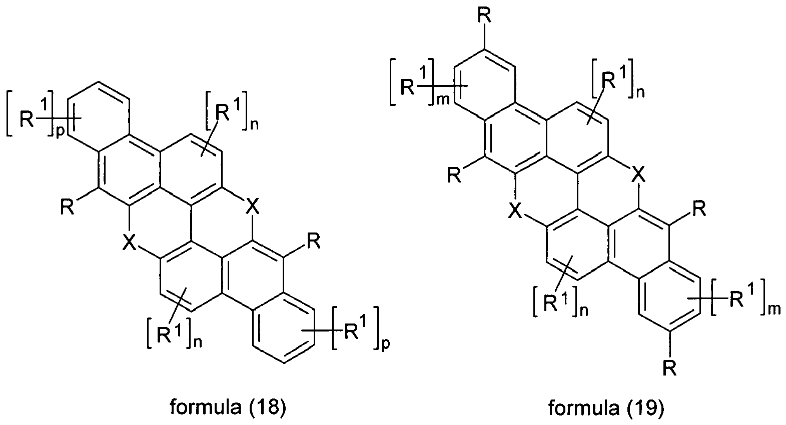 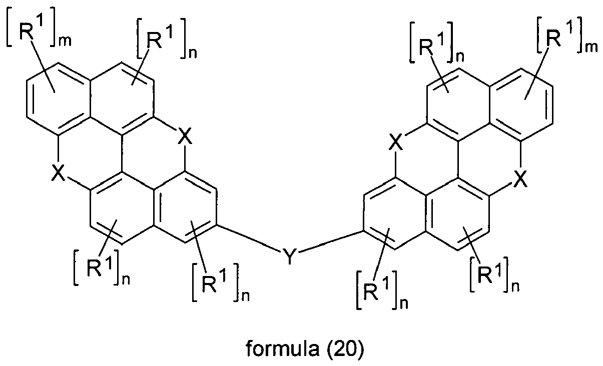 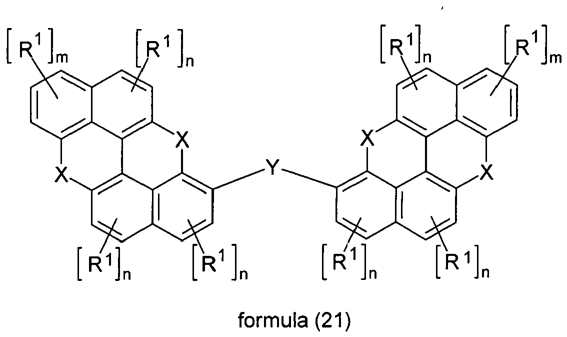 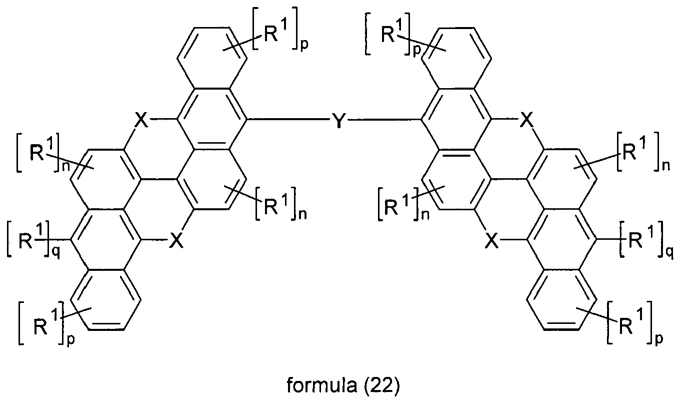 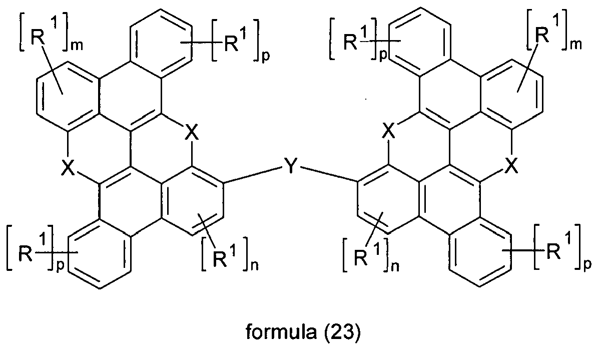 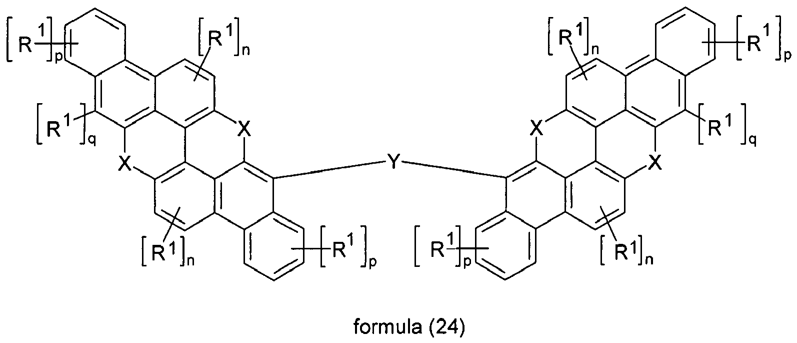 where R, R¹, R², Ar, X, Y, m, n, p and q have the same meaning as described in Claim 1.

3. Compounds according to Claim 1 or 2, selected from the structures of the formulae (9a) to (24a), 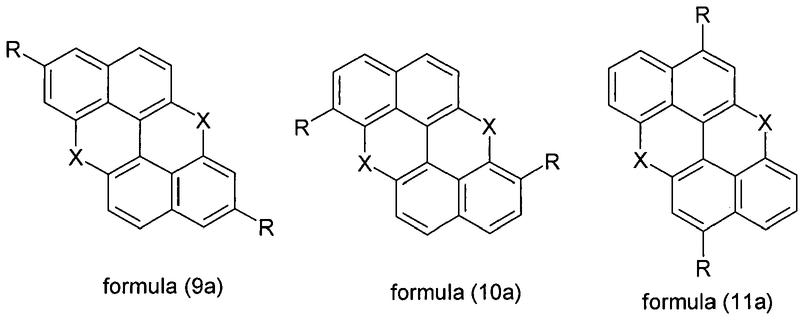 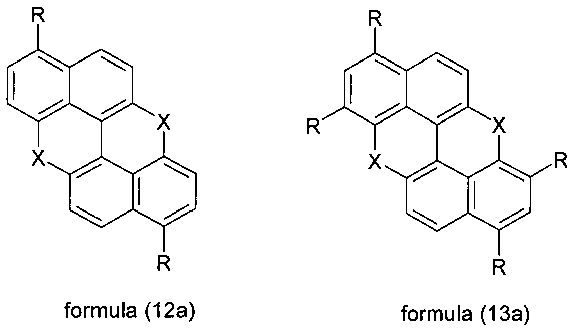 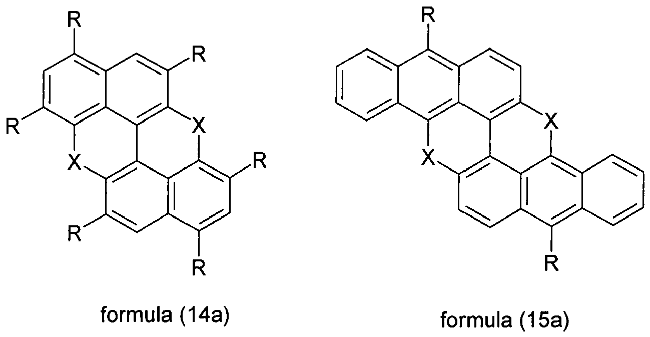 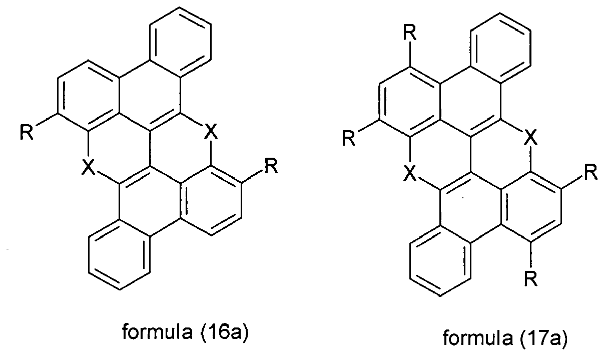 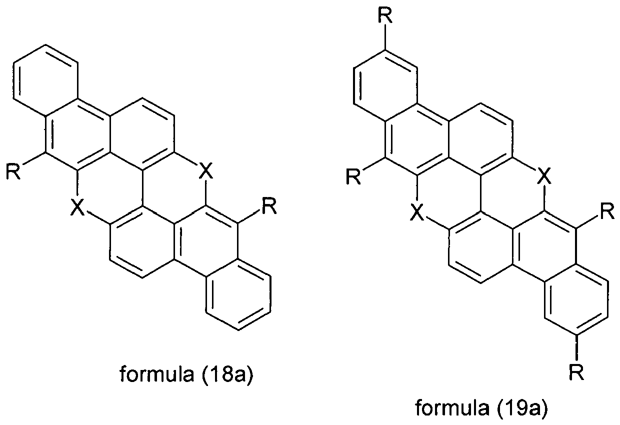 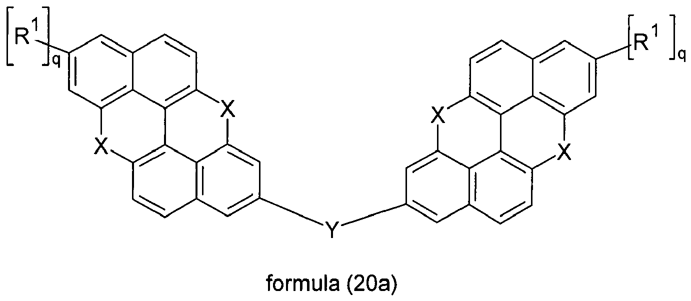 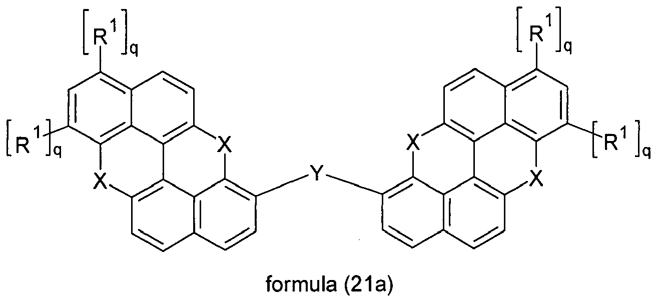 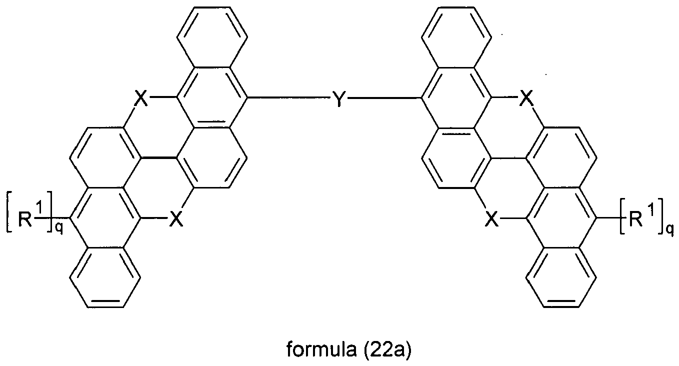 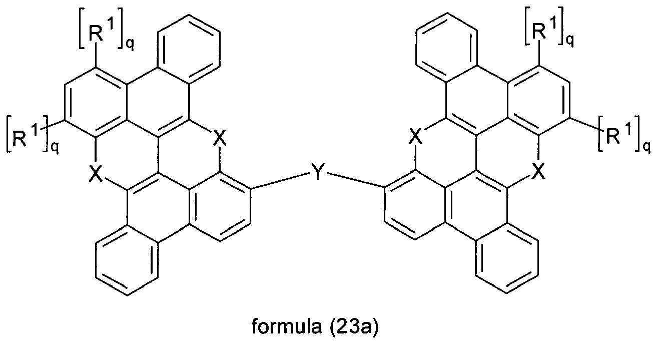 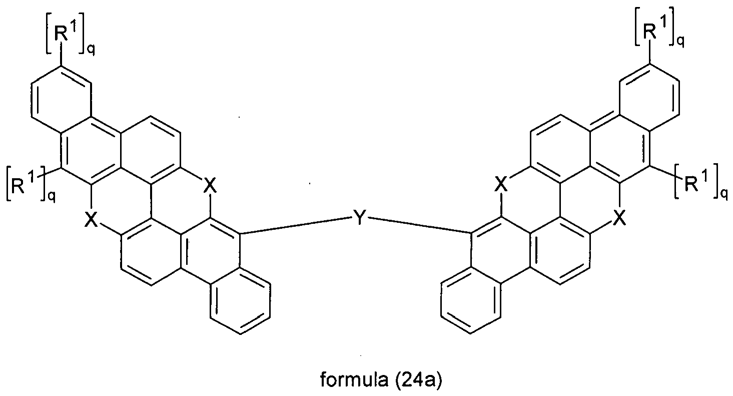 where R, R¹, R², Ar, X, Y and q have the same meaning as described in Claim 1.

4. Compounds according to one or more of Claims 1 to 3, **characterised in that** the symbol R, identically or differently on each occurrence, stands for an N(Ar)₂, C(=O)Ar, P(=O)Ar₂ group, a straight-chain alkyl group having 1 to 4 C atoms or a branched alkyl group having 3 to 5 C atoms or an aromatic or heteroaromatic ring system having 5 to 25 aromatic ring atoms, which may be substituted by one or more non-aromatic radicals R¹, and **in that** the symbol Y stands for a C(=O) or N(Ar) group or for a divalent aryl group having 6 to 16 C atoms, which may be substituted by one or more non-aromatic radicals R¹.

5. Compounds according to one or more of Claims 1 to 4, **characterised in that** the compounds have a symmetrical structure with respect to the substituents present.

6. Polymers, oligomers or dendrimers containing one or more compounds according to one or more of Claims 1 to 5, where one or more radicals R, R¹ and/or R² represent bonds to the polymer, oligomer or dendrimer.

7. Process for the preparation of compounds according to one or more of Claims 1 to 5 in which X = O, **characterised by** oxidative cyclisation of the binaphthol or bianthrol or biphenanthrol derivative which has already been correspondingly functionalised.

8. Process for the preparation of compounds according to one or more of Claims 1 to 5 in which X = C(R¹)₂, **characterised by** acid-catalysed dehydrating cyclisation of the binaphthyl or bianthryl or biphenanthryl derivative which has optionally already been correspondingly functionalised and which is in each case substituted by two groups of the formula -C(R¹)₂(OH) in the ortho-positions to the link of the binaphthyl or bianthryl or biphenanthryl.

9. Use of compounds according to one or more of Claims 1 to 6 in electronic devices, in particular in organic electroluminescent devices.

10. Electronic devices comprising at least one compound according to one or more of Claims 1 to 6.

11. Organic electroluminescent device comprising anode, cathode, at least one emitting layer and optionally further layers selected from hole-injection layer, hole-transport layer, electron-transport layer, electron-injection layer and/or charge-generation layer, **characterised in that** at least one organic layer comprises at least one compound according to one or more of Claims 1 to 6.

12. Organic electroluminescent device according to Claim 11, **characterised in that** the symbol X in compounds according to one or more of Claims 1 to 7 stands for O, S or N(R¹) and **in that** the compound is employed as emitting compound, optionally mixed with a host material.

13. Organic electroluminescent device according to Claim 11 or 12, **characterised in that** the symbol X in compounds according to one or more of Claims 1 to 6 stands for C(R¹)₂ and **in that** the compound is employed as host material for fluorescent or phosphorescent dopants.

14. Organic electroluminescent device according to one or more of Claims 11 to 13, **characterised in that** the symbol X in compounds according to one or more of Claims 1 to 7 stands for O, S or N(R¹) and/or **in that** at least one group R and/or R¹ stands for an N(Ar)₂ group and/or **in that** the group Y stands for an N(Ar) group, **in that** the compound may optionally be doped and **in that** the compound is employed as hole-transport material or as hole-injection material, in particular in a hole-transport or hole-injection layer respectively.

15. Organic electroluminescent device according to one or more of Claims 11 to 14, **characterised in that** the symbol X stands for C(R¹)₂, BR¹, POR¹, SO or SO₂ and **in that** one or more of the substituents R and/or R¹ optionally stand for a C(=O)Ar or P(=O)Ar₂ group or for an electron-deficient heterocycle and/or the group Y stands for a C(=O) or P(=O)Ar group, **in that** the compound may optionally be doped and **in that** the compound is employed in an electron-transport layer and/or a hole-blocking layer.

## Revendications

1. Composés des formules (1) à (8) : 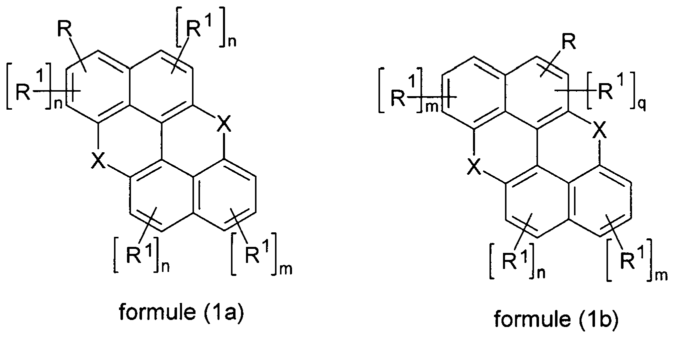 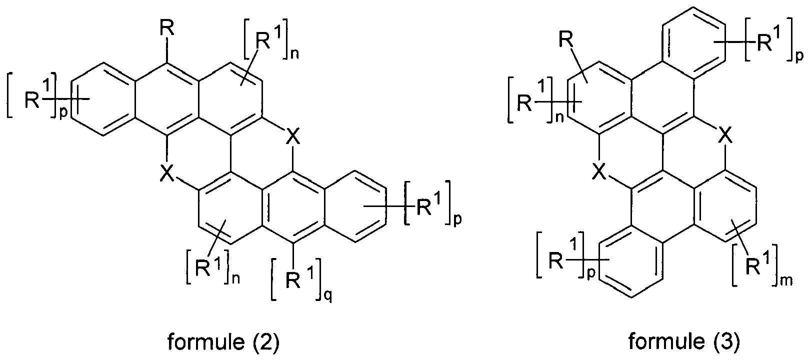 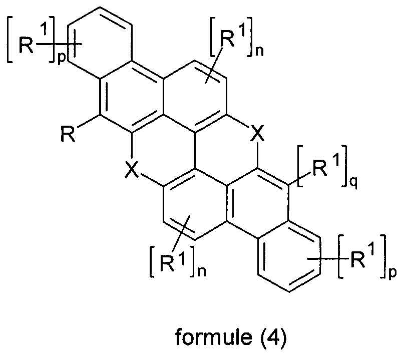 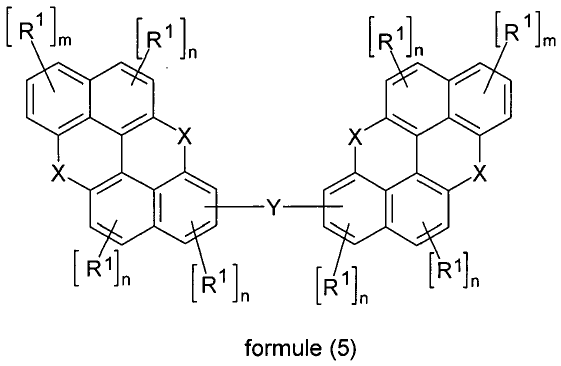 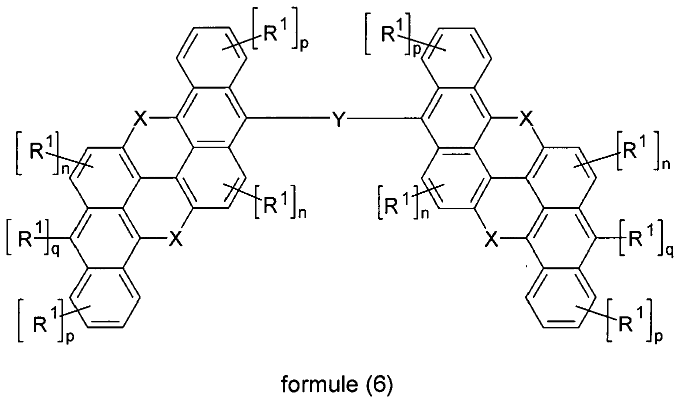 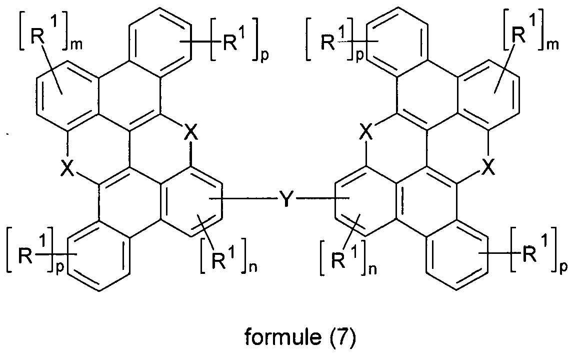 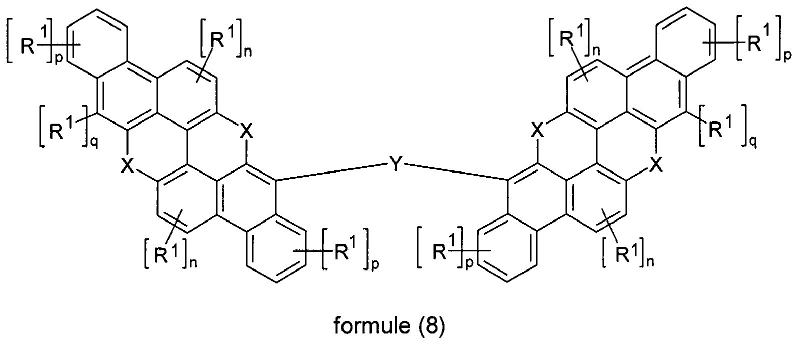 dans lesquelles ce qui suit s'applique aux symboles et indices :
X est, pour chaque occurrence, de manière identique ou différente, O, S, NR¹, C(R¹)₂, BR¹, PR¹, POR¹, SO ou SO₂;
Y est une liaison simple, un groupe C(=O), P(=O)Ar, N(Ar), S(=O), S(=O)₂, O, S, un groupe alkylène ou alkylidène comportant 1 à 20 atome(s) de C ou un système de cycle aromatique divalent comportant 5 à 40 atomes de cycle aromatique, lequel peut être substitué par un radical ou plusieurs radicaux non aromatique(s) R^{1;}
R est, pour chaque occurrence, de manière identique ou différente, un groupe N(Ar)₂, C(=O)Ar, P(=O)Ar₂, S(=O)Ar, S(=O)₂Ar, CR²=CR²Ar, OAr, SAr, Si(R¹)₃, un groupe alkyle en chaîne droite comportant 1 à 40 atome(s) de C, un groupe alkyle ramifié ou cyclique comportant 3 à 40 atomes de C, dont chacun peut être substitué par un radical ou plusieurs radicaux R² et où un ou plusieurs atome(s) de H peut/peuvent être remplacé(s) par F, CI, Br, I, CN ou NO₂, un groupe alcoxy en chaîne droite comportant 2 à 40 atomes de C ou un groupe alcoxy ramifié ou cyclique comportant 3 à 40 atomes de C, où le groupe alcoxy peut dans chaque cas être substitué par un radical ou plusieurs radicaux R², où un ou plusieurs groupes CH₂ non adjacents dans les groupes alcoxy peut/peuvent être remplacé(s) par R²C=CR², C≡C, Si(R²)₂, Ge(R²)₂, Sn(R²)₂, C=O, C=S, C=Se, C=NR², P(=O)(R²), SO, SO₂, NR², O, S ou CONR² et où un ou plusieurs atome(s) de H peut/peuvent être remplacé(s) par F, CI, Br, I, CN ou NO₂, ou un système de cycle aromatique ou hétéroaromatique comportant 5 à 60 atomes de cycle aromatique, dont chacun peut être substitué par un radical ou plusieurs radicaux non aromatique(s) R^{1 ;} R peut également ici former un système de cycle monocyclique ou polycyclique avec des substituants adjacents R^{1 ;}
R¹ est, pour chaque occurrence, de manière identique ou différente, H, F, CI, Br, I, CHO, N(Ar)₂, C(=O)Ar, P(=O)Ar₂, S(=O)Ar, S(=O)₂Ar, CR²=CR²Ar, OAr, SAr, CN, NO₂, Si(R²)₃, B(OR²)₂, OSO₂R², un groupe alkyle, alcoxy ou thioalcoxy en chaîne droite comportant 1 à 40 atome(s) de C ou un groupe alkyle, alcoxy ou thioalcoxy ramifié ou cyclique comportant 3 à 40 atomes de C, dont chacun peut être substitué par un radical ou plusieurs radicaux R², où un ou plusieurs groupe(s) CH₂ non adjacents peut/peuvent être remplacé(s) par R²C=CR², C=C, Si(R²)₂, Ge(R²)₂, Sn(R²)₂, C=O, C=S, C=Se, C=NR², P(=O)(R²), SO, SO₂, NR², O, S ou CONR² et où un ou plusieurs atome(s) de H peut/peuvent être remplacé(s) par F, CI, Br, I, CN ou NO₂, ou un système de cycle aromatique ou hétéroaromatique comportant 5 à 60 atomes de cycle aromatique, dont chacun peut être substitué par un radical ou plusieurs radicaux non aromatique(s) R¹, ou une combinaison de ces systèmes ; deux substituants adjacents R¹ ou plus peuvent ici également former un système de cycle monocyclique ou polycyclique l'un avec l'autre ou les uns avec les autres ou R¹ avec R ;
Ar est, pour chaque occurrence, de manière identique ou différente, un système de cycle aromatique ou hétéroaromatique comportant 5 à 40 atomes de cycle aromatique, lequel peut être substitué par un radical ou plusieurs radicaux non aromatique(s) R^{1 ;} deux radicaux Ar qui sont liés au même atome d'azote ou de phosphore peuvent également être liés l'un à l'autre ici par une liaison simple ou un pont choisi parmi B(R²), C(R²)₂, Si(R²)₂, C=O, C=NR², C=C(R²)₂, O, S, S=O, SO₂, N(R²), P(R²) et P(=O)R²;
R² est, pour chaque occurrence, de manière identique ou différente, H ou un radical hydrocarbone aliphatique, aromatique et/ou hétéroaromatique comportant 1 à 20 atome(s) de C, où, en outre, des atomes de H peuvent être remplacés par F; deux substituants R² adjacents ou plus peuvent ici également former un système de cycle aliphatique ou aromatique monocyclique ou polycyclique l'un avec l'autre ou les uns avec les autres ;
n est, pour chaque occurrence, de manière identique ou différente, 0, 1 ou 2 ;
m est, pour chaque occurrence, de manière identique ou différente, 0, 1, 2 ou 3 ;
p est, pour chaque occurrence, de manière identique ou différente, 0, 1, 2, 3 ou 4 ;
q est, pour chaque occurrence, de manière identique ou différente, 0 ou 1 ;
les composés qui suivent sont exclus de l'invention :

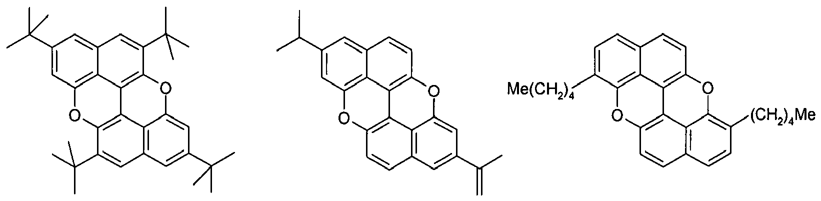

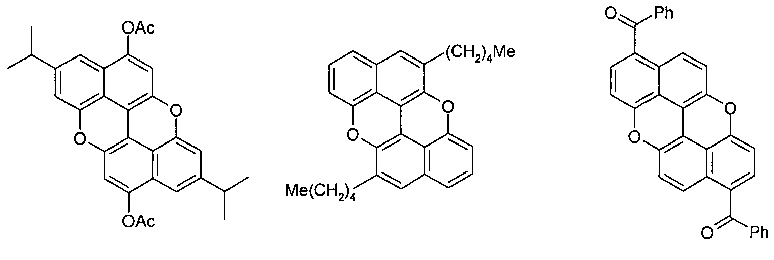

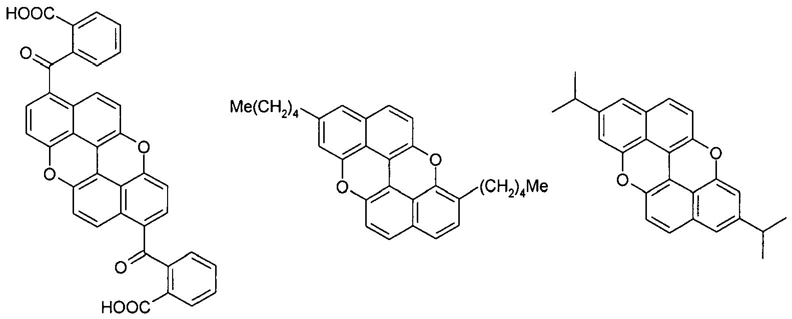

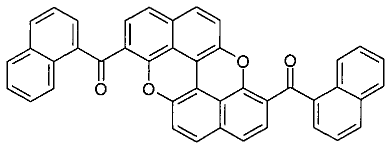

2. Composés selon la revendication 1, choisis parmi les structures des formules (9) à (24) : 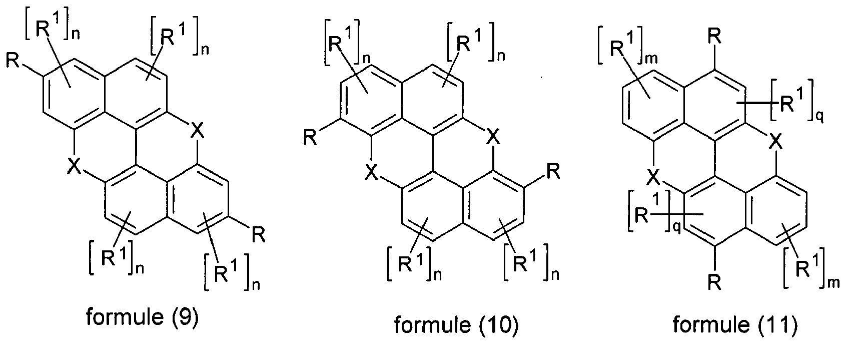 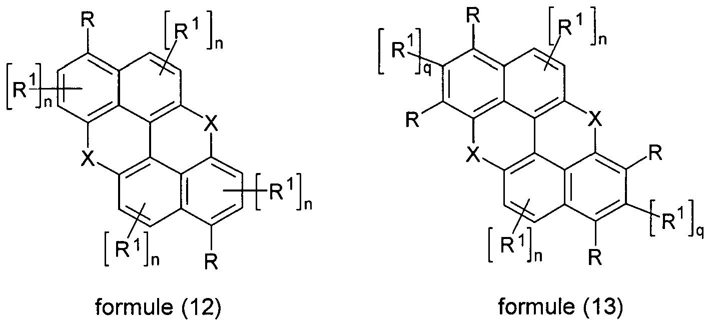 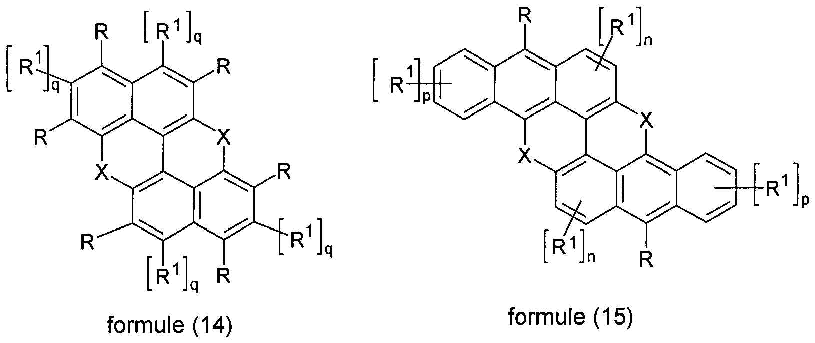 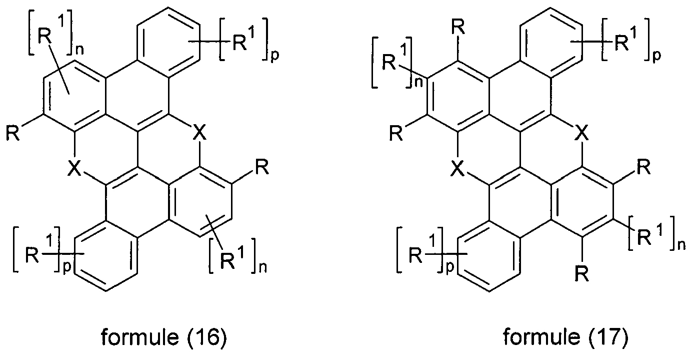 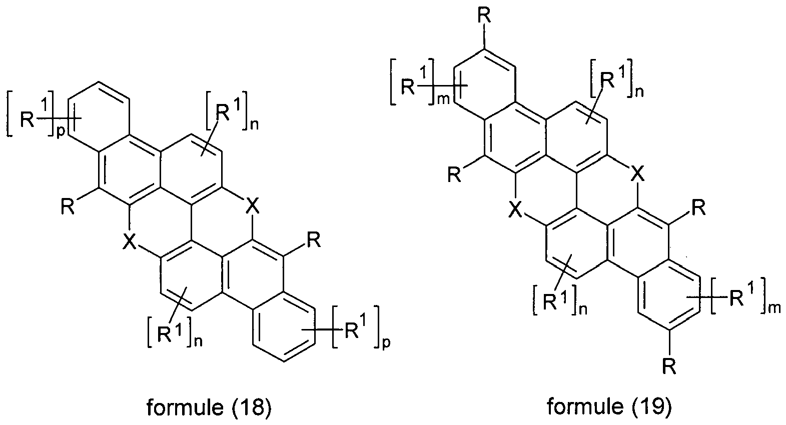 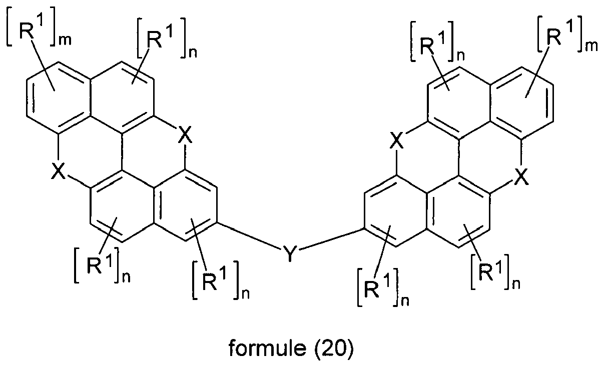 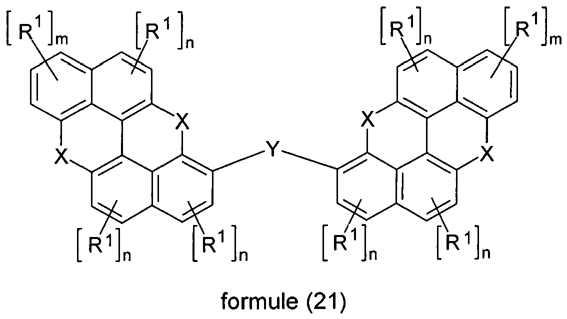 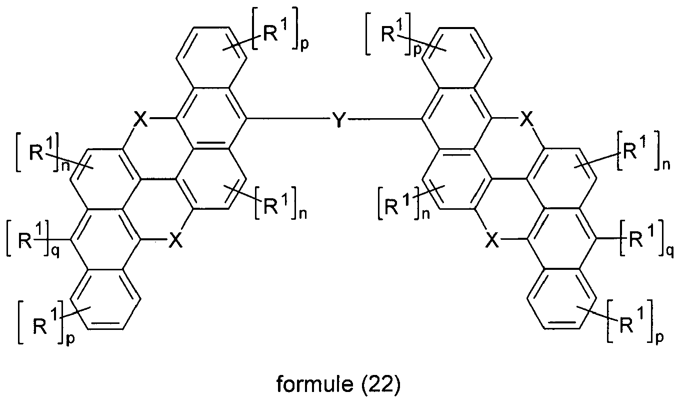 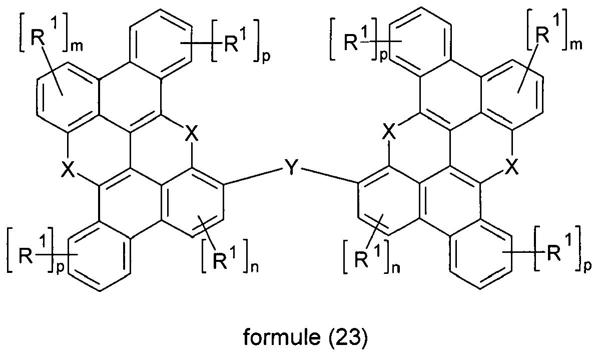 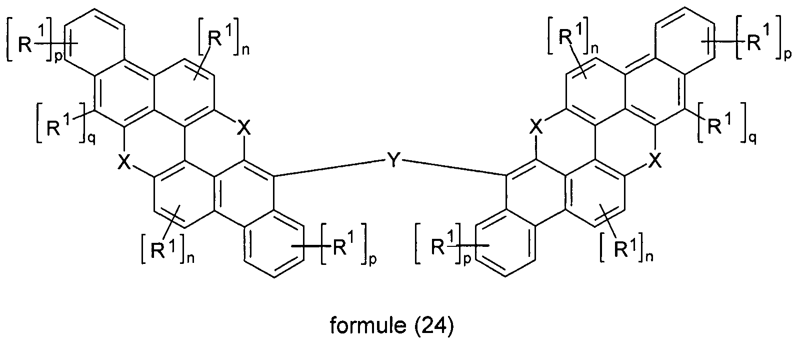 dans lesquelles R, R¹, R², Ar, X, Y, m, n, p et q présentent la même signification que décrit selon la revendication 1.

3. Composés selon la revendication 1 ou 2, choisis parmi les structures des formules (9a) à (24a) : 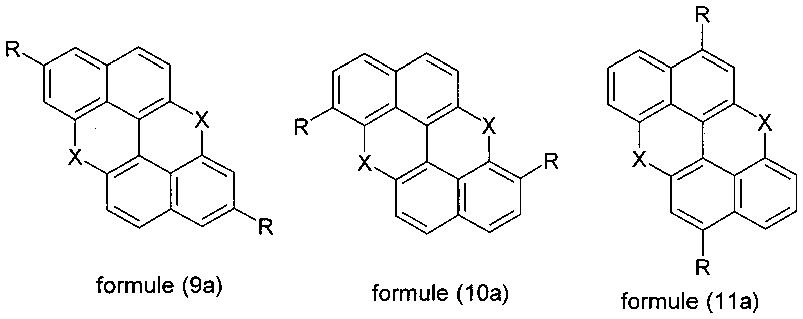 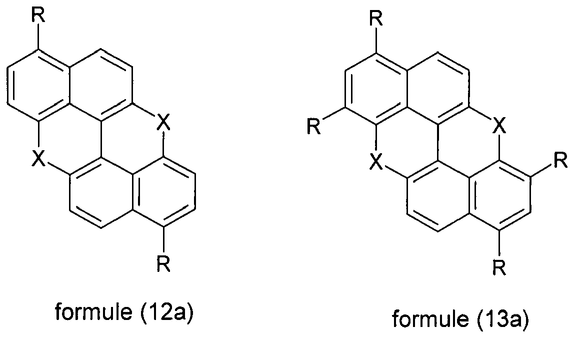 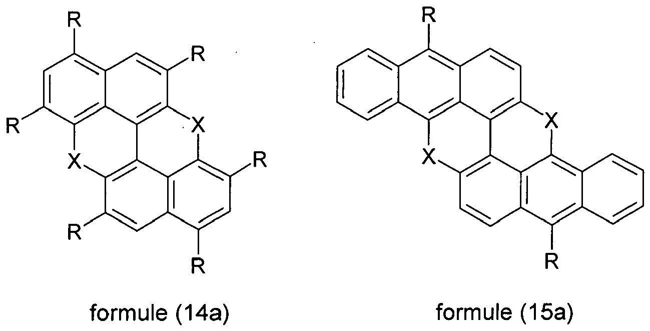 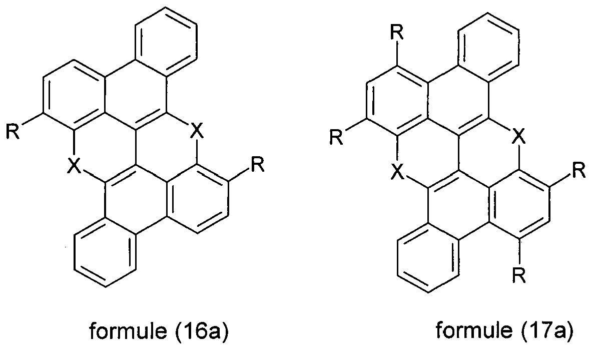 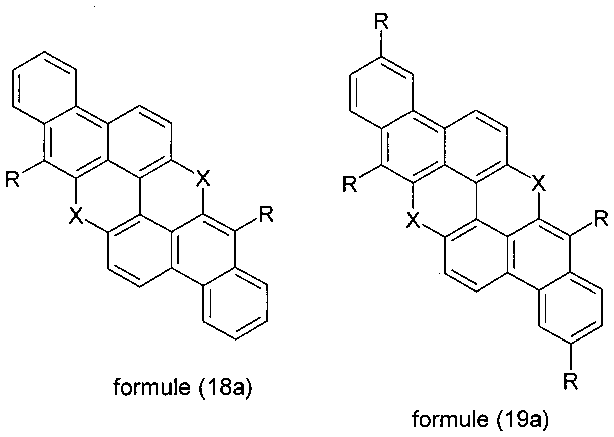 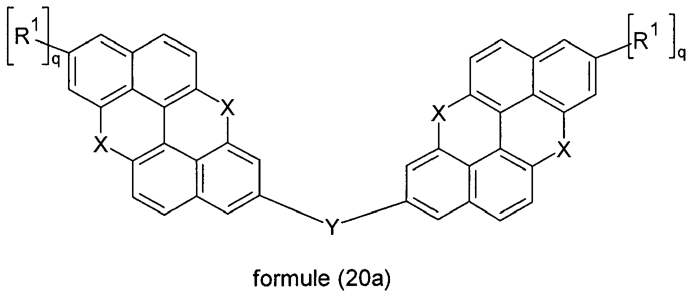 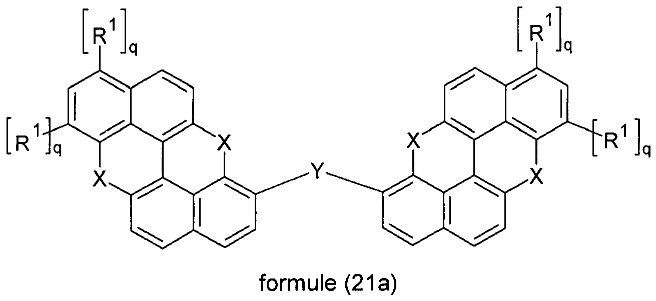 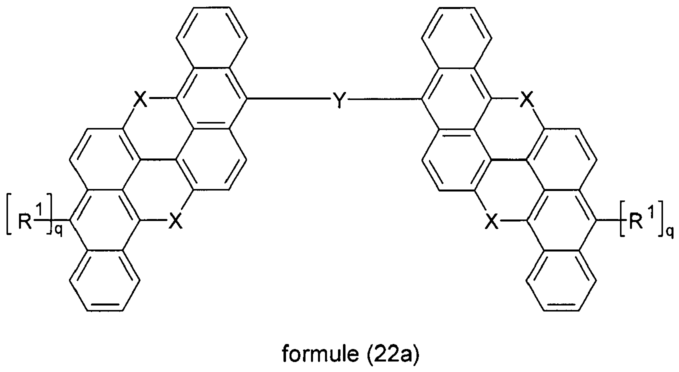 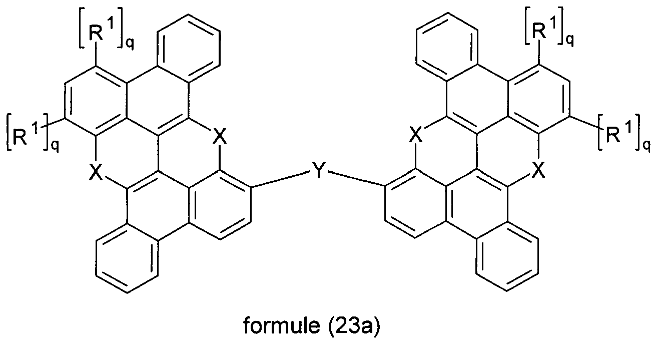 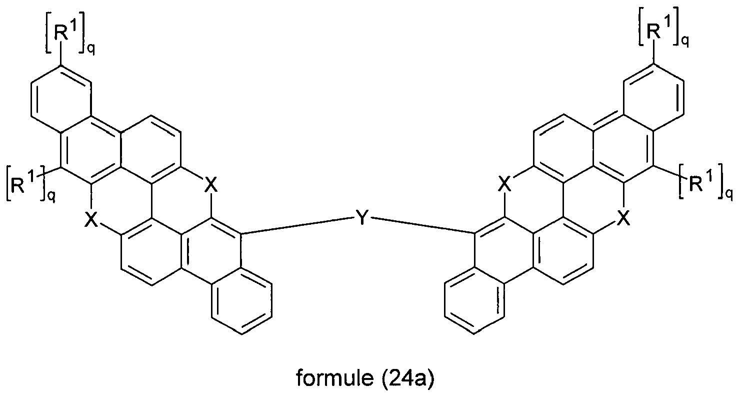 dans lesquelles R, R¹, R², Ar, X, Y et q présentent la même signification que décrit selon la revendication 1.

4. Composés selon une ou plusieurs des revendications 1 à 3, **caractérisés en ce que** le symbole R représente, de manière identique ou différente pour chaque occurrence, un groupe N(Ar)₂, C(=O)Ar, P(=O)Ar₂, un groupe alkyle en chaîne droite comportant 1 à 4 atome(s) de C ou un groupe alkyle ramifié comportant 3 à 5 atomes de C ou un système de cycle aromatique ou hétéroaromatique comportant 5 à 25 atomes de cycle aromatique, lequel peut être substitué par un radical ou plusieurs radicaux non aromatique(s) R¹, et **en ce que** le symbole Y représente un groupe C(=O) ou N(Ar) ou un groupe aryle divalent comportant 6 à 16 atomes de C, lequel peut être substitué par un radical ou plusieurs radicaux non aromatique(s) R¹.

5. Composés selon une ou plusieurs des revendications 1 à 4, **caractérisés en ce que** les composés présentent une structure symétrique par rapport aux substituants présents.

6. Polymères, oligomères ou dendrimères contenant un ou plusieurs composé(s) selon une ou plusieurs des revendications 1 à 5, où un radical ou plusieurs radicaux R, R¹ et/ou R² représente(nt) des liaisons sur le polymère, l'oligomère ou le dendrimère.

7. Procédé pour la préparation de composés selon une ou plusieurs des revendications 1 à 5 où X = O, **caractérisé par** une cyclisation oxydative du dérivé binaphthol ou bianthrol ou biphénanthrol qui a déjà été fonctionnalisé en correspondance.

8. Procédé pour la préparation de composés selon une ou plusieurs des revendications 1 à 5 où X = C(R¹)₂, **caractérisé par** une cyclisation de déshydratation catalysée par acide du dérivé binaphtyle ou bianthryle ou biphénanthryle qui a en option déjà été fonctionnalisé en correspondance et qui est dans chaque cas substitué par deux groupes de la formule -C(R¹)₂(OH) au niveau des positions ortho sur la liaison du binaphtyle ou du bianthryle ou du biphénanthryle.

9. Utilisation de composés selon une ou plusieurs des revendications 1 à 6 dans des dispositifs électroniques, en particulier dans des dispositifs électroluminescents organiques.

10. Dispositifs électroniques comprenant au moins un composé selon une ou plusieurs des revendications 1 à 6.

11. Dispositif électroluminescent organique comprenant une anode, une cathode, au moins une couche d'émission et en option, d'autres couches choisies parmi une couche d'injection de trous, une couche de transport de trous, une couche de transport d'électrons, une couche d'injection d'électrons et/ou une couche de génération de charges, **caractérisé en ce qu'**au moins une couche organique comprend au moins un composé selon une ou plusieurs des revendications 1 à 6.

12. Dispositif électroluminescent organique selon la revendication 11, **caractérisé en ce que** le symbole X dans des composés selon une ou plusieurs des revendications 1 à 7 représente O, S ou N(R¹) et **en ce que** le composé est utilisé en tant que composé d'émission, en option mélangé avec un matériau hôte.

13. Dispositif électroluminescent organique selon la revendication 11 ou 12, **caractérisé en ce que** le symbole X dans des composés selon une ou plusieurs des revendications 1 à 6 représente C(R¹)₂ et **en ce que** le composé est utilisé en tant que matériau hôte pour des dopants fluorescents ou phosphorescents.

14. Dispositif électroluminescent organique selon une ou plusieurs des revendications 11 à 13, **caractérisé en ce que** le symbole X dans des composés selon une ou plusieurs des revendications 1 à 7 représente O, S ou N(R¹) et/ou **en ce qu'**au moins un groupe R et/ou R¹ représente un groupe N(Ar)₂ et/ou **en ce que** le groupe Y représente un groupe N(Ar), **en ce que** le composé peut en option être dopé et **en ce que** le composé est utilisé en tant que matériau de transport de trous ou en tant que matériau d'injection de trous, en particulier respectivement dans une couche de transport de trous ou dans une couche d'injection de trous.

15. Dispositif électroluminescent organique selon une ou plusieurs des revendications 11 à 14, **caractérisé en ce que** le symbole X représente C(R¹)₂, BR¹, POR¹, SO ou SO₂ et **en ce qu'**un ou plusieurs des substituants R et/ou R¹ représente(nt) en option un groupe C(=O)Ar ou P(=O)Ar₂ ou un hétérocycle déficient en électrons et/ou le groupe Y représente un groupe C(=O) ou P(=O)Ar, **en ce que** le composé peut en option être dopé et **en ce que** le composé est utilisé dans une couche de transport d'électrons et/ou dans une couche de blocage de trous.
